# EUROPEAN PATENT APPLICATION

(11) **EP 1 256 341 A1**
(43) Date of publication of application: **13.11.2002**
(21) Application number: 01906131.6
(22) Date of filing: 15.02.2001
(51) Int. Cl.: A61K 31/196, A61K 31/215, A61P 35/00

(54) **CANCER REMEDY COMPRISING ANTHRANILIC ACID DERIVATIVE AS ACTIVE INGREDIENT**

(30) Priority: 15.02.2000 JP 2000036386
(71) Applicant: TEIJIN LIMITED, Osaka-shi Osaka 541-0054 (JP)
(72) Inventor: TSUCHIYA, Naoki, c/o Teijin Limited, Hino-shi, Tokyo 191-0065 (JP); TAKEYASU, Takumi, c/o Teijin Limited, Iwakuni-shi, Yamaguchi 740-0014 (JP); KAWAMURA, Takashi, c/o Teijin Limited, Tokyo 100-0011 (JP); YAMORI, Takao, 102, Minami-cho Queen Hills, Tokyo 162-0836 (JP); TSURUO, Takashi, Setagaya-ku, Tokyo 156-0051 (JP)
(74) Representative: Hallybone, Huw George
(86) International application number: JP0101090
(87) International publication number: WO01060354

(57) **Abstract**

A cancer remedy comprising a compound represented by the following formula as an active ingredient: 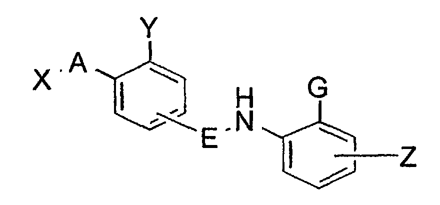 wherein, X represents a group represented by either of the following formulae: 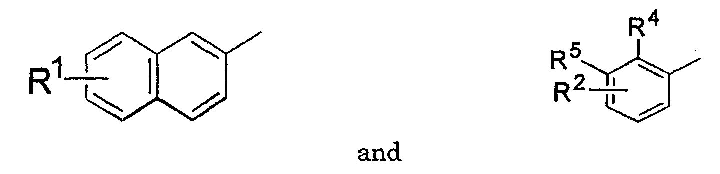 wherein, R¹ and R² represent each a hydrogen atom, a hydroxy group, a trihalomethyl group, a C₁-C₁₂ alkoxy group or alkylthio group, a (substituted) C₇-C₁₁ aralkyloxy group or a (substituted) C₃-C₁₀ alkenyloxy group; R⁴ and R⁵ represent each a hydrogen atom, a halogen atom, a C₁-C₄ alkyl group or a C₁-C₄ alkoxy group; A represents -O-, -S-, -S(=O)-, -S(=O)₂-, -CH₂-, -OCH₂-, -SCH₂, -C(=O)- or -CH(OR⁶)-; Y represents a hydrogen atom, a halogen atom, a nitro group, a nitrile group, an amino group, -COOR⁷, -NHCOR⁸ or -NHSO₂R⁹; E represents -C(=O)-, -CR¹⁰R¹¹C(=O)-, -CH₂CH₂C(=O)- or -CH=CHC(=O)-; G represents a hydrogen atom, a hydroxy group, -SO₂NH₂, -COOR³, -CN or a tetrazol-5-yl group; and Z represents a hydrogen atom, a halogen atom, a nitro group or a methyl group.

## Description

### Technical Field

The present invention relates to a cancer remedy comprising an anthranilic acid derivative or a pharmaceutically acceptable salt as an active ingredient. More particularly, it relates to a cancer remedy comprising an anthranilic acid derivative, having an anthranilic acid skeleton and a benzene skeleton and further having the benzene skeleton or a naphthalene skeleton, or a pharmaceutically acceptable salt thereof, as an active ingredient.

### Background Art

There have been strong demands from the society for the development of excellent carcinostatic agents, and it is extremely important to create a new compound having strong cytotoxicity in development of excellent carcinostatic agents. In general, the carcinostatic activity of. compounds and the carcinostatic spectra depend largely on the chemical structure thereof. Accordingly, there is a very great possibility of developing better carcinostatic agents than those put into practical use at present from cytotoxic compounds having a novel structure.

The carcinostatic activity based on the cytotoxic activity of compounds having an aryl skeleton are known as, for example substituted phenylsulfonyl derivatives [JP-A No. 5-9170 (hereinafter, JP-A refers to Japanese Unexamined Patent Publication)] 2-arylquinolinol derivatives (JP-A No. 7-33743) and benzoylacetylene derivatives (JP-A No. 7-97350).

On the other hand, WO95/32943 and Journal of Medicinal Chemistry (J. Med. Chem.), vol. 40, No.4, pp. 395-407 (1997) describe compounds having a naphthalene skeleton and an anthranilic acid skeleton and an antiallergic activity and an inhibitory activity against the production of IgE antibodies.

WO97/19910 describes compounds having a benzene skeleton and an anthranilic acid skeleton and further an antiallergic activity and an inhibitory activity against the production of IgE antibodies.

However, it is unknown that a group of compounds having the aryl skeleton and the anthranilic acid skeleton at the same time have a cytotoxic activity or a carcinostatic activity.

### Disclosure of the Invention

An object of the present invention is to provide a cancer remedy having a novel structure.

About the problems described above, the inventors of the present application have newly found that the anthranilic acid derivatives have a cytotoxic activity against cell strains having a high growth property, and that the anthranilic acid derivatives have a strong growth inhibitory activity or cytotoxic activity against human cancer cells. Therefore, a pharmaceutical composition comprising the anthranilic acid derivative, its pharmaceutically acceptable salt or a pharmaceutically acceptable solvate thereof as an active ingredient is useful as a cancer remedy.

Namely, the present invention provides a cancer remedy comprising an anthranilic acid derivative represented by the following formula (1) or a pharmaceutically acceptable salt thereof as an active ingredient: 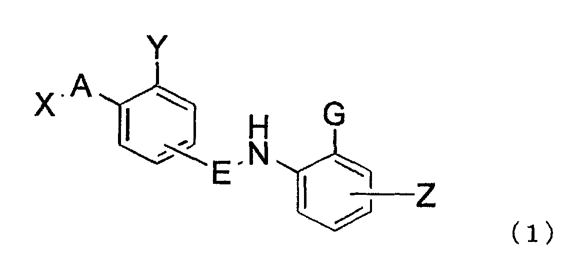 wherein, X represents a group selected from the following formula (2)-1 and formula (2)-2 in the formula (1): 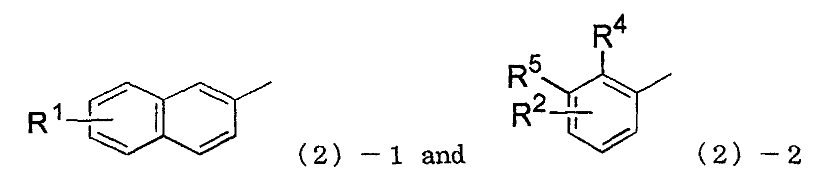 wherein, R¹ and R² represent each independently a hydrogen atom, a hydroxy group, a trihalomethyl group, an alkoxy group or an alkylthio group comprising a C₁-C₁₂ chain or cyclic hydrocarbon group and an oxy group or a thio group, a C₇-C₁₁ aralkyloxy group wherein an aryl group moiety may be substituted with one or more halogen atoms, methyl groups or methyloxy groups or a C₃-C₁₀ alkenyloxy group which may be substituted with one or more phenyl groups; R⁴ and R⁵ represent each independently a hydrogen atom, a halogen atom, a C₁-C₄ alkyl group or a C₁-C₄ alkoxy group, in the formula (2)-1 or the formula (2)-2;
A represents a bond; -O-, -S-, -S(=O)-, -S(=O)₂-, -CH₂-, -OCH₂-, -SCH₂-, -C(=O)- or -CH(OR⁶)-,
wherein, R⁶ represents a hydrogen atom or a C₁-C₄ alkyl group;
Y represents a hydrogen atom, a halogen atom, a nitro group, a nitrile group, an amino group, -COOR⁷, -NHCOR⁸ or -NHSO₂R⁹,
wherein, R⁷ represents a hydrogen atom or a C₁-C₄ alkyl group; R⁸ and R⁹ represent each independently a C₁-C₄ alkyl group;
E represents a bond; -C(=O)-, -CR¹⁰R¹¹C(=O)- (wherein, R¹⁰ and R¹¹ represent each independently a hydrogen atom or a fluorine atom),
-CH₂CH₂C(=O)- or -CH=CHC(=O)-;
G represents a hydrogen atom, a hydroxy group, -SO₂NH₂, -COOR³, (wherein, R³ represents a hydrogen atom or a C₁-C₄ alkyl group), -CN or a tetrazol-5-yl group; and
Z represents a hydrogen atom, a halogen atom, a nitro group or a methyl group.

Furthermore, the present invention provides a therapy for cancer using a drug comprising the anthranilic acid derivative or a pharmaceutically acceptable salt thereof.

In addition, the present invention is the use of the anthranilic acid derivative or a pharmaceutically acceptable salt thereof in order to produce the cancer remedy.

### Best Mode for Carrying Out the Invention

In the formula (2)-1 or (2)-2 in the above formula (1) representing the anthranilic acid derivative used in the present invention, R¹ and R² represent each independently a hydrogen atom, a hydroxy group, a trihalomethyl group, an alkoxy group or an alkylthio group comprising a C₁-C₁₂ chain or cyclic hydrocarbon group and an oxy group or a thio group, a C₇-C₁₁ aralkyloxy group wherein an aryl group moiety may be substituted with one or more of halogen atoms, methyl groups or methyloxy groups or a C₃-C₁₀ alkenyloxy group which may be substituted with one or more phenyl groups.

When R¹ or R² represents a C₁-C₁₂ chain or cyclic alkyloxy group, R¹ or R² can be selected from, for example methyloxy group, ethyloxy group, propyloxy group, 2-propyloxy group, (1- or 2-)methylpropyloxy group, 2,2-dimethylpropyloxy group, (n- or tert-)butyloxy group, 2-ethylbutyloxy group, (2- or 3-)methylbutyloxy group, pentyloxy group, hexyloxy group, heptyloxy group, octyloxy group, decyloxy group, dodecyloxy group, cyclopropyloxy group, cyclopropylmethyloxy group, cyclobutyloxy group, cyclopentyloxy group, cyclohexyloxy group, cyclohexylmethyloxy group, cyclooctyloxy group, cycloheptyloxy group, cyclododecyloxy group and the like.

When R¹ or R² represents a C₁-C₁₂ chain or cyclic alkylthio group, R¹ or R² can be selected from, for example methylthio group, ethylthio group, propylthio group, 2-propylthio group, (1- or 2-)methylpropylthio group, 2,2-dimethylpropylthio group, (n- or tert-)butylthio group, 2-ethylbutyltyhio group, (2- or 3-)methylbutylthio group, pentylthio group, hexylthio group, heptylthio group, octylthio group, decylthio group, dodecylthio group, cyclopropylthio group, cyclopropylmethylthio group, cyclobutylthio group, cyclopentylthio group, cyclohexylthio group, cyclohexylmethylthio group, cyclooctylthio group, cycloheptylthio group, cyclododecylthio group and the like.

When R¹ or R² represents a C₇-C₁₂ aralkyloxy group, the aryl group moiety of the aralkyloxy group may be represented by one or more of halogen atoms, methyl groups or methyloxy groups, and examples of the substituents include fluorine atoms, chlorine atoms, bromine atoms, methyl groups, methyloxy groups and the like. Therefore, the aralkyloxy groups represented by R¹ can be selected from, for example benzyloxy group, (2-, 3-or 4-)chlorobenzyloxy group, (2-, 3- or 4-)methoxybenzyloxy group, (2-, 3- or 4-)methylbenzyloxy group, (α - or β -)phenethyloxy group, 3-phenylpropyloxy group, 2-phenyl-2-propyloxy group, 2-phenyl-1-cyclohexyloxy group, (1-phenylcyclopropyl)methyloxy group, (1-phenylcyclopentyl)methyloxy group, (1- or 2-)naphthylmethyloxy group and the like.

Furthermore, R¹ or R² may be a C₃-C₁₀ alkenyloxy group. The alkenyloxy group in this case can be selected from, for example allyloxy group, methallyloxy group, crotyloxy group, 3-butenyloxy group, 4-pentenyloxy group, 5-hexenyloxy group, 7-octenyloxy group, geranyloxy group, cinnamyloxy group, 2-cyclohexenyloxy group, (3-cyclohexenyl)methyloxy group, 1,4-pentadien-3-yloxy group and the like.

R¹ and R² may each be a hydrogen atom, a hydroxy group or a trihalomethyl group. Examples of the halogen atom representing the trihalomethyl group include fluorine atoms, chlorine atoms and the like.

Examples of preferable atoms or groups among the atoms or groups represented by R¹ or R² include a hydrogen atom, a hydroxy group, methyloxy group, ethyloxy group, propyloxy group, 2-propyloxy group, (1-or 2-)methylpropyloxy group, 2,2-dimethylpropyloxy group, (n- or tert)butyloxy group, 2-ethylbutyloxy group, (2- or 3-)methylbutyloxy group, pentyloxy group, hexyloxy group, heptyloxy group, octyloxy group, decyloxy group, dodecyloxy group, cyclopropylmethyloxy group, cyclopentyloxy group, cyclohexyloxy group, cyclohexylmethyloxy group, cyclooctyloxy group, cycloheptyloxy group, cyclododecyloxy group, methythio group, ethylthio group, isopropylthio group, t-butylthio group, 3-pentylthio group, cyclohexylthio group, cyclooctylthio group, benzyloxy group, 4-chlorobenzyloxy group, 4-methylbenzyloxy group, (α- or β - )phenethyloxy group, 3-phenylpropyloxy group, (1- or 2-)naphthylmethyloxy group, allyloxy group, 3-butenyloxy group, 4-pentenyloxy group, 5-hexenyloxy group, 7-octenylxy group, trifluoromethyl group and the like.

Among them, the atoms or groups are especially preferably an alkyloxy group wherein the R¹ or R² group comprises a hydrogen atom, a hydroxy group, a C₁-C₁₂ chain or cyclic saturated hydrocarbon or a C₇-C₁₂ aralkyloxy group, more preferably a hydrogen atom, for example a C₅-C₁₂ cyclic saturated alkoxy group such as cyclohexyloxy group, cycloheptyloxy group, cyclooctyloxy group, cyclopentyloxy group or cyclododecanyloxy group or a C₃-C₈ branched chain saturated alkoxy group, especially preferably an alkyloxy group producing a branch from the adjacent carbon of the oxygen atom, for example isopropyloxy group, 3-pentyloxy group or benzyloxy group.

In the above formula (2)-1 or (2)-2, R¹ and R² may be substituted at an optional position on the naphthalene ring or the benzene ring; however, R¹ is preferably located in the 6-position counted from the ring in which the A on the naphthalene ring is linked (A is substituted at the 2-position) or R² is preferably located in the 4-position counted from the bond of A on the benzene ring.

In the above formula (2)-2, R⁴ and R⁶ represent each independently a hydrogen atom, a halogen atom, a C₁-C₄ alkyl group or a C₁-C₄ alkoxy group. Examples of the halogen atom include a fluorine atom, a chlorine atom, a bromine atom and the like. Examples of the C₁-C₄ alkyl group include methyl group, ethyl group, isopropyl group, t-butyl group and the like. Further, examples of the C₁-C₄ alkoxy group include methyloxy group, ethyloxy group, isopropyloxy group, t-butyloxy group and the like. Among the groups, examples of R⁴ or R⁵ include preferably a hydrogen atom, a chlorine atom, methyl group or methyloxy group. Among them, hydrogen atom is preferable. In addition, R⁴ is substituted at the 2-position on the benzene ring, and R⁵ is substituted at the 3-position on the benzene ring.

In the formula (1), A represents a bond; -O-, -S-, -S(=O)-, -S(=O)₂-, -CH₂-, -OCH₂-, -SCH₂-, -C(=O)- or -CH(OR⁶)-, wherein, R⁶ represents a hydrogen atom or a C₁-C₄ alkyl group.
Examples of the C₁-C₄ alkyl group include methyl group, ethyl group, n-propyl group, tert-butyl group and the like. R⁶ is preferably a hydrogen atom or methyl group. More preferable bond is -O- or -S- as A.

Furthermore, in the formula (1), Y represents a hydrogen atom, a halogen atom, a nitro group, a nitrile group, an amino group, -COOR⁷, wherein, R⁷ represents a hydrogen atom or a C₁-C₄ alkyl group, -NHCOR⁸ or -NHSO₂R⁹, wherein, R⁸ and R⁹ represent each independently a C₁-C₄ alkyl group. When Y represents a halogen atom, examples of the halogen atom include a fluorine atom, a chlorine atom and a bromine atom. Among them, chlorine atom is preferable. When Y represents -COOR⁷, R⁷ represents a hydrogen atom or a C₁-C₄ alkyl group, and examples thereof include a hydrogen atom, methyl group, ethyl group, n-propyl group, 2-propyl group, n-butyl group and tert-butyl group. It is preferable for the -COOR⁷ to be -COOH or -COOCH₃. When Y represents -NHCOOR⁸ or -NHSO₂R⁹, R⁸ or R⁹ represents a C₁-C₄ alkyl group. Examples thereof include methyl group, ethyl group, n-propyl group, 2-propyl group, n-butyl group and tert-butyl group. When Y represents -NHCOR⁸ or -NHSO₂R⁹, it is preferable for Y to be -NHCOCH₃ or -NHSO₂CH₃.

It is especially preferable for Y to be a hydrogen atom, a chlorine atom, a nitro group or a nitrile group, among ones listed above.

Furthermore, in the above formula (1), E represents a bond; -C(=O)-, -CR¹⁰R¹¹C(=O)-, wherein R¹⁰ and R¹¹ represent each independently a hydrogen atom or a fluorine atom,
-CH₂CH₂C(=O)- or -CH=CHC(=O)-. Among them, E represents preferably a bond; -C(=O)- or -CH₂C(=O)-, more preferably a bond; -CH₂C(=O)-.

In the above formula (1), G represents a hydrogen atom, a hydroxy group, -SO₂NH₂, -COOR³, wherein, R³ represents a hydrogen atom or a C₁-C₄ alkyl group,
-CN or a tetrazol-5-yl group. When G represents -COOR³, examples of the alkyl group represented by R³ include methyl group, ethyl group, (n- or iso)propyl group, (n-, iso- or tert-)butyl group and the like. The G is preferably -COOR³, wherein R³ represents a hydrogen atom or a C₁-C₄ alkyl group, or tetrazol-5-yl group; and R³ is especially preferably a hydrogen atom, methyl group or ethyl group. It is more preferable for G to be -COOH or tetrazol-5-yl group.

In addition, in the above formula (1), Z represents a hydrogen atom, a halogen atom, a nitro group or a methyl group. Examples of the halogen atom include a fluorine atom, a chlorine atom and a bromine atom. Z is preferably a hydrogen atom, a fluorine atom, a chlorine atom and methyl group, especially preferably a hydrogen atom.

In the anthranilic acid derivative represented by the formula (1), it is especially preferable that R¹ and R² represent each a hydrogen atom, a C₁-C₁₂ alkoxy group or a C₇-C₁₂ aralkyloxy group; A represents a bond; -O-; and E represents a bond; -C(=O)- or -CH₂C(=O)-. The compound manifests an exceedingly high cytotoxic activity against cells having a strong growth activity.

In the anthranilic acid derivative represented by the formula (1), it is preferable that R¹ and R² represent each a hydrogen atom, a C₅-C₁₂ cyclic alkyl group, a C₃-C₈ branched chain alkyl group or a benzyl group; A represents a bond; -O-; E represents a bond; -CH₂C(=O)-; and G represents COOH or a tetrazol-5-yl group. The compound manifests a stronger cytotoxic activity.

In the above formula (1), when Z represents a halogen atom or a methyl group, the substituent is preferably located in the 4- or the 5-position with respect to the group G on the benzene ring to which the group Z represents bound. The Z group located in the 4- or the 5-position has advantages in preventing the compound represented by the formula (1) from being inactivated with metabolism and sustaining the pharmaceutical effects thereof.

When Y is -COOH group or G is a -COOH group or a tetrazol-5-yl group in the above formula (1) (Y and G may be present at the same time or only either one thereof may be present), the carboxylic acid group or the like, if necessary, may be converted into a pharmaceutically acceptable nontoxic salt thereof. Examples of the preferably used nontoxic salt-forming cation include alkali metal ions such as Na⁺ and K⁺; alkaline earth metal ions such as Mg²⁺ and Ca²⁺; nontoxic equivalent metal ions such as Al³⁺ and Zn²⁺; organic bases such as ammonia, triethylamine, ethylenediamine, propanediamine, pyrrolidine, piperidine, piperazine, pyridine, lysine, choline, ethanolamine, N,N-dimethylethanolamine, 4-hydroxypiperidine, glucosamine, N-methylglucamine and the like. Among the salt-forming cation, Na²⁺, Ca²⁺ and the organic bases such as lysine, choline, N,N-dimethylethanolamine and N-methylglucamine are preferably used.

The anthranilic acid derivative or a nontoxic salt thereof represented by the formula (1) may form a pharmaceutically acceptable solvate thereof. Solvents forming the solvate can be selected from water, methanol, ethanol, (n- and iso-)propyl alcohol, (n- and tert-)butanol, acetonitrile, acetone, methyl ethyl ketone, chloroform, ethyl acetate, diethyl ether, tert-butyl methyl ether, benzene, toluene, DMF, DMSO and the like. Among the solvents, water, methanol, ethanol, (n- and iso-)propyl alcohol or acetonitrile is especially preferably used.

The cancer remedy of the present invention comprises the anthranilic acid derivative, a pharmaceutically acceptable salt thereof or a solvate thereof as an active ingredient; however, a pharmaceutically acceptable carrier, if necessary, may be added.

Preferable specific examples of the anthranilic acid derivative represented by the formula (1) are listed in the following tables. When the structural formula of the compound has an asymmetric carbon (for example, compound No. 44), all the optical isomers are included. When the structural formula has a carbon-carbon double bond (for example, compound No. 120), both geometrical isomers are included. In the tables, "tet" indicates a tetrazol-5-yl group.

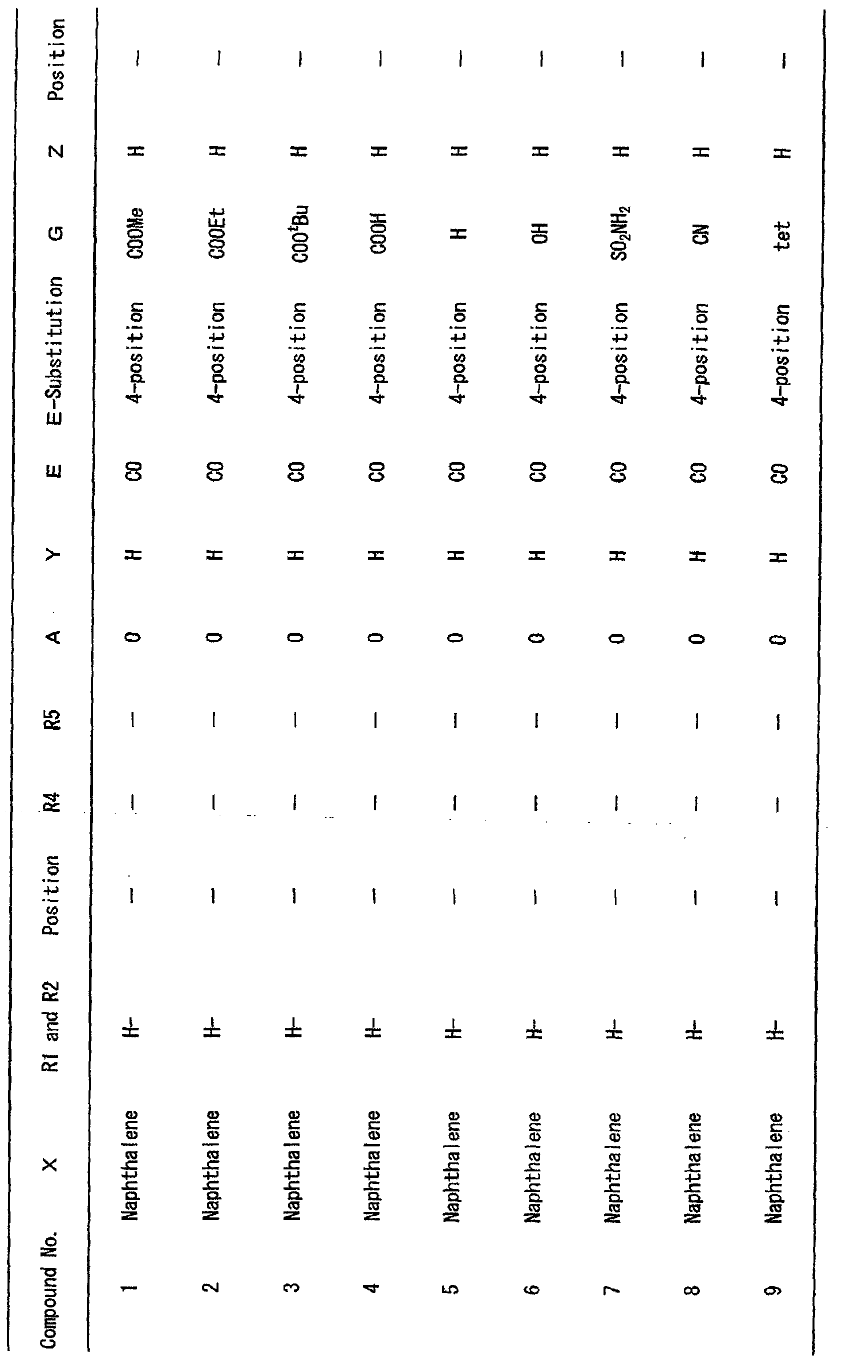

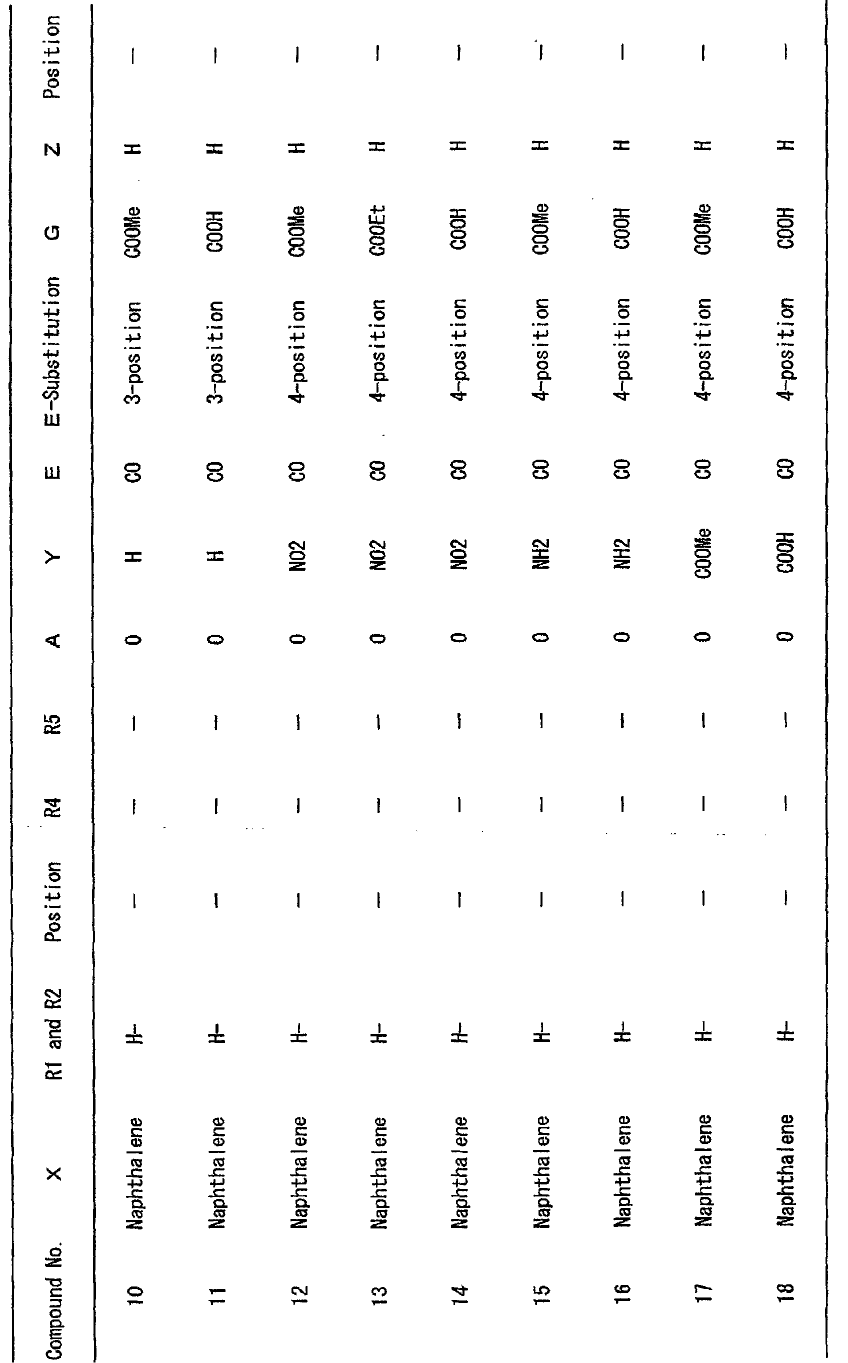

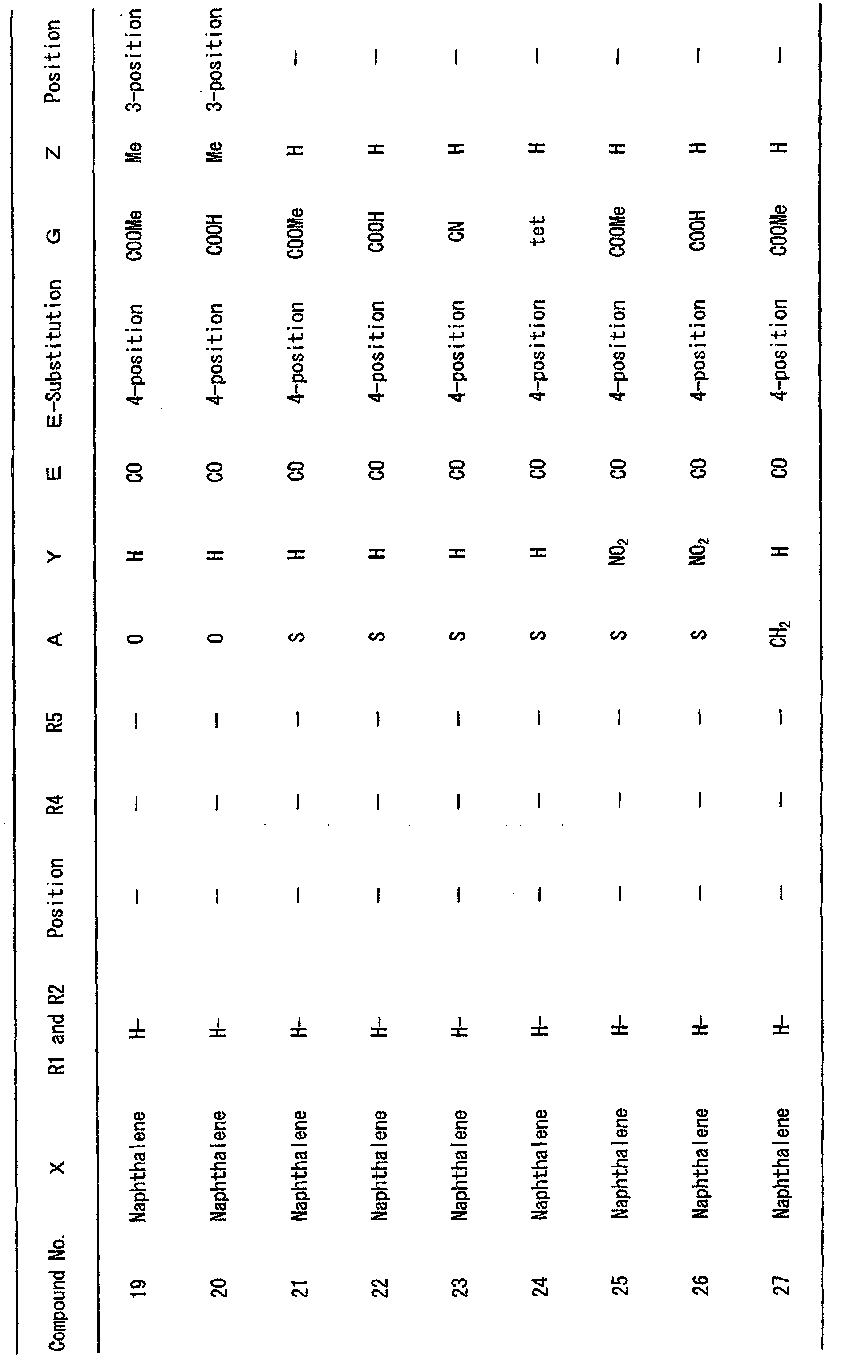

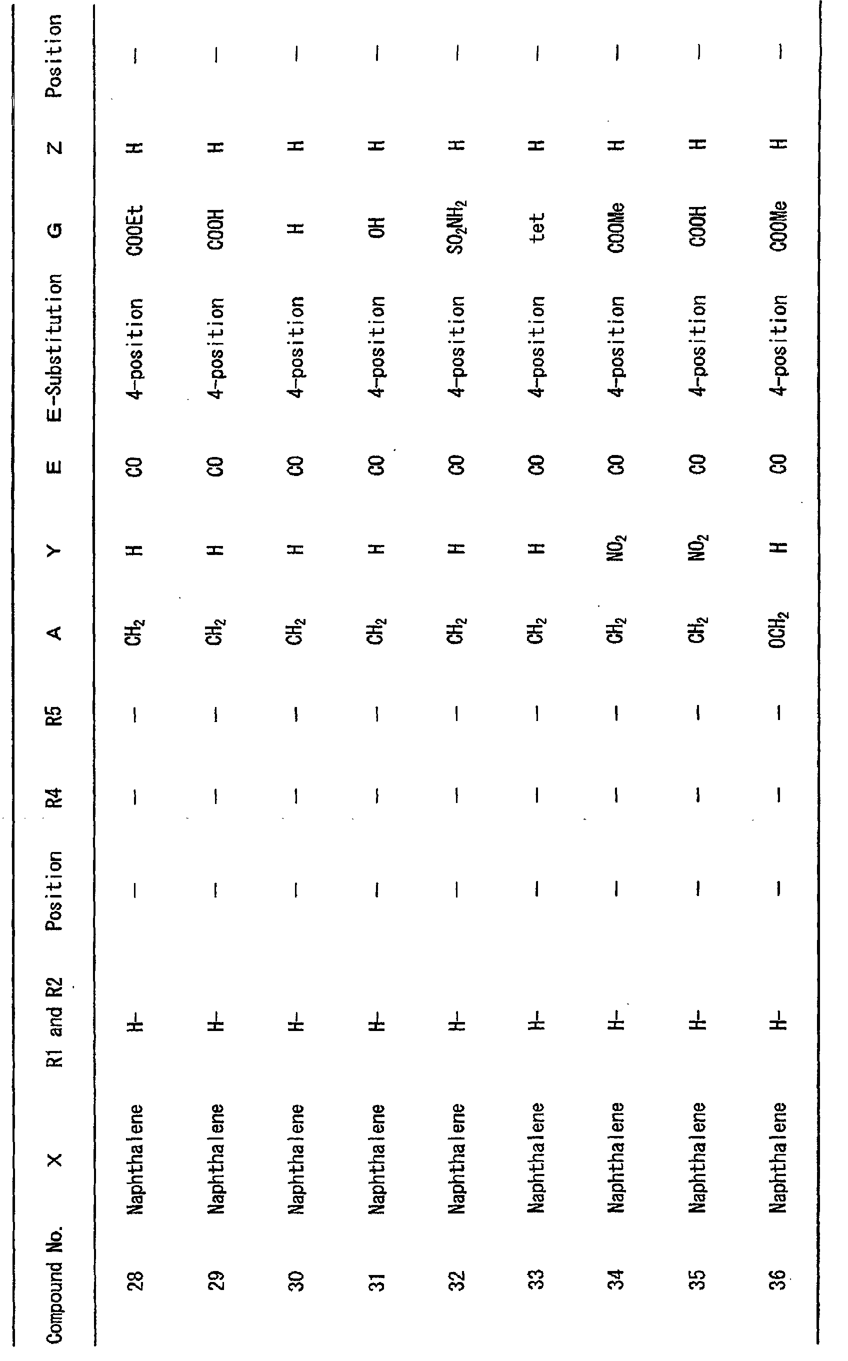

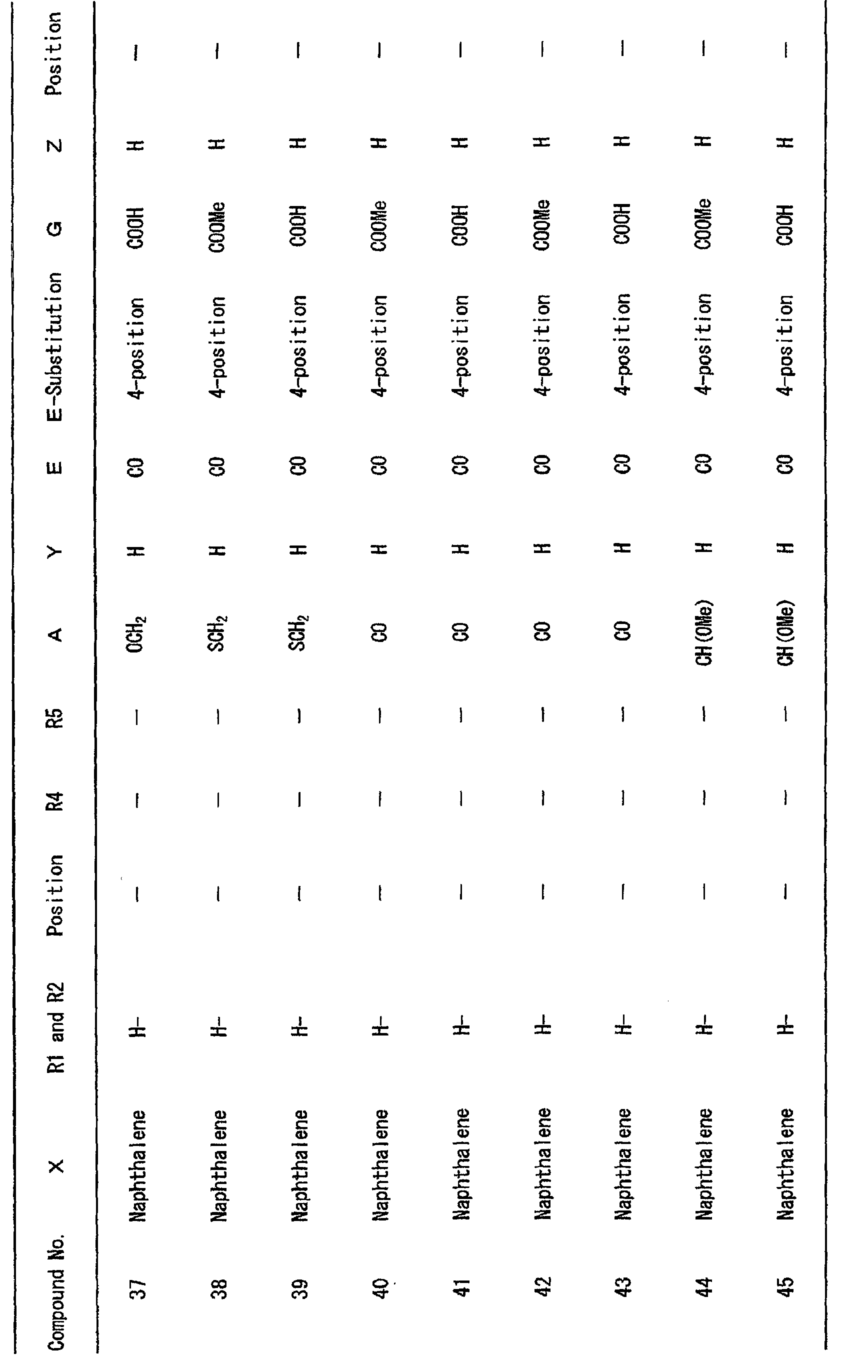

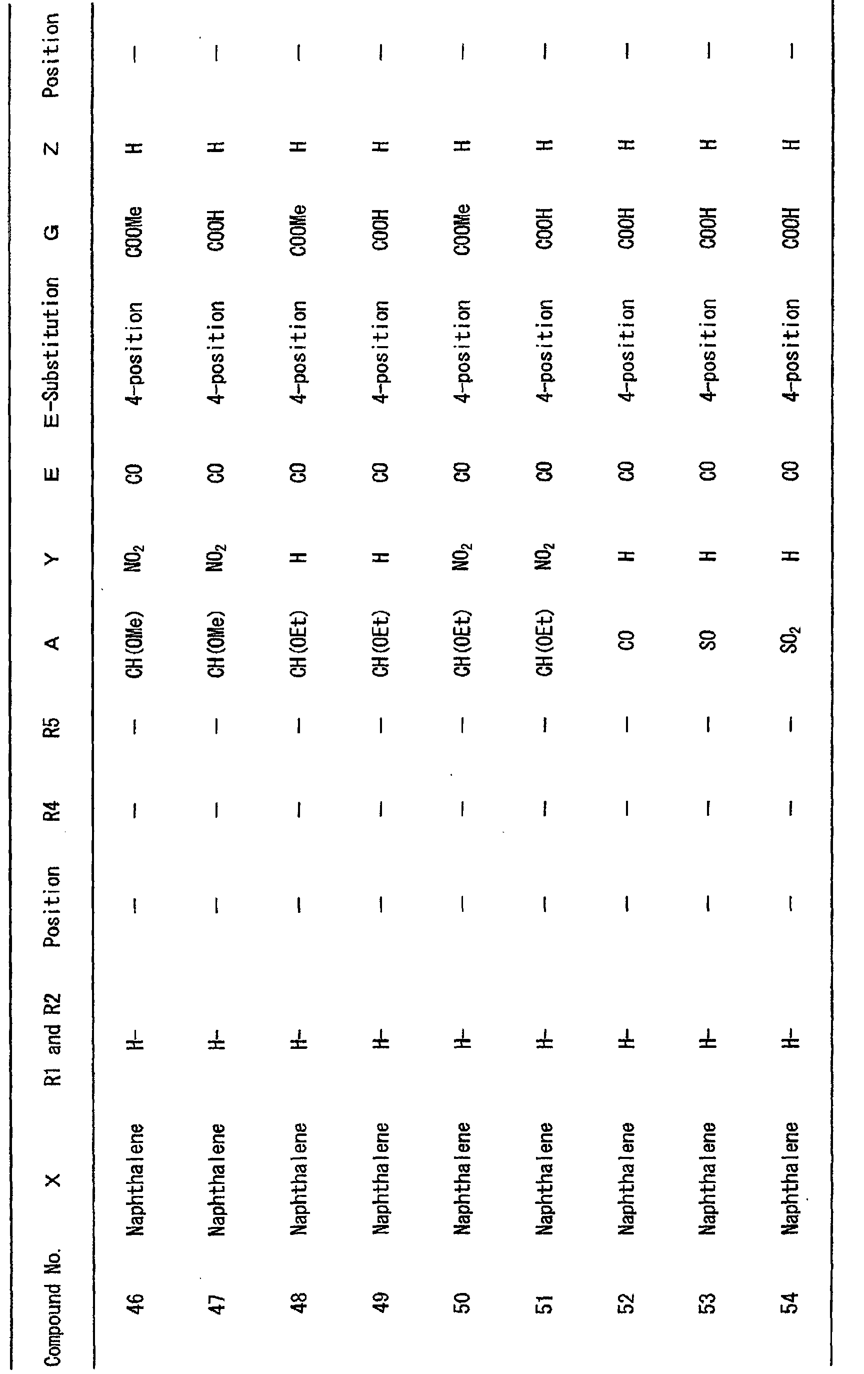

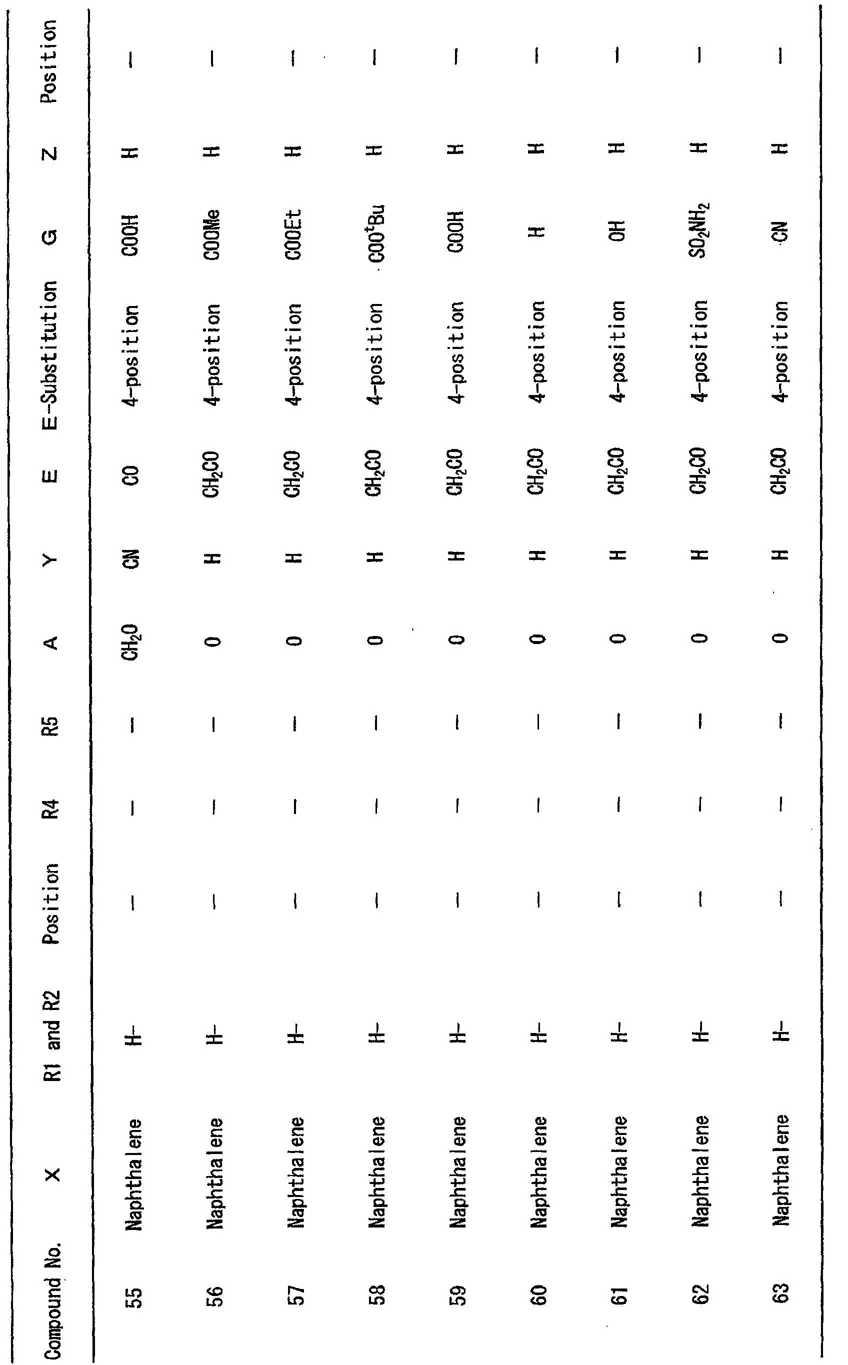

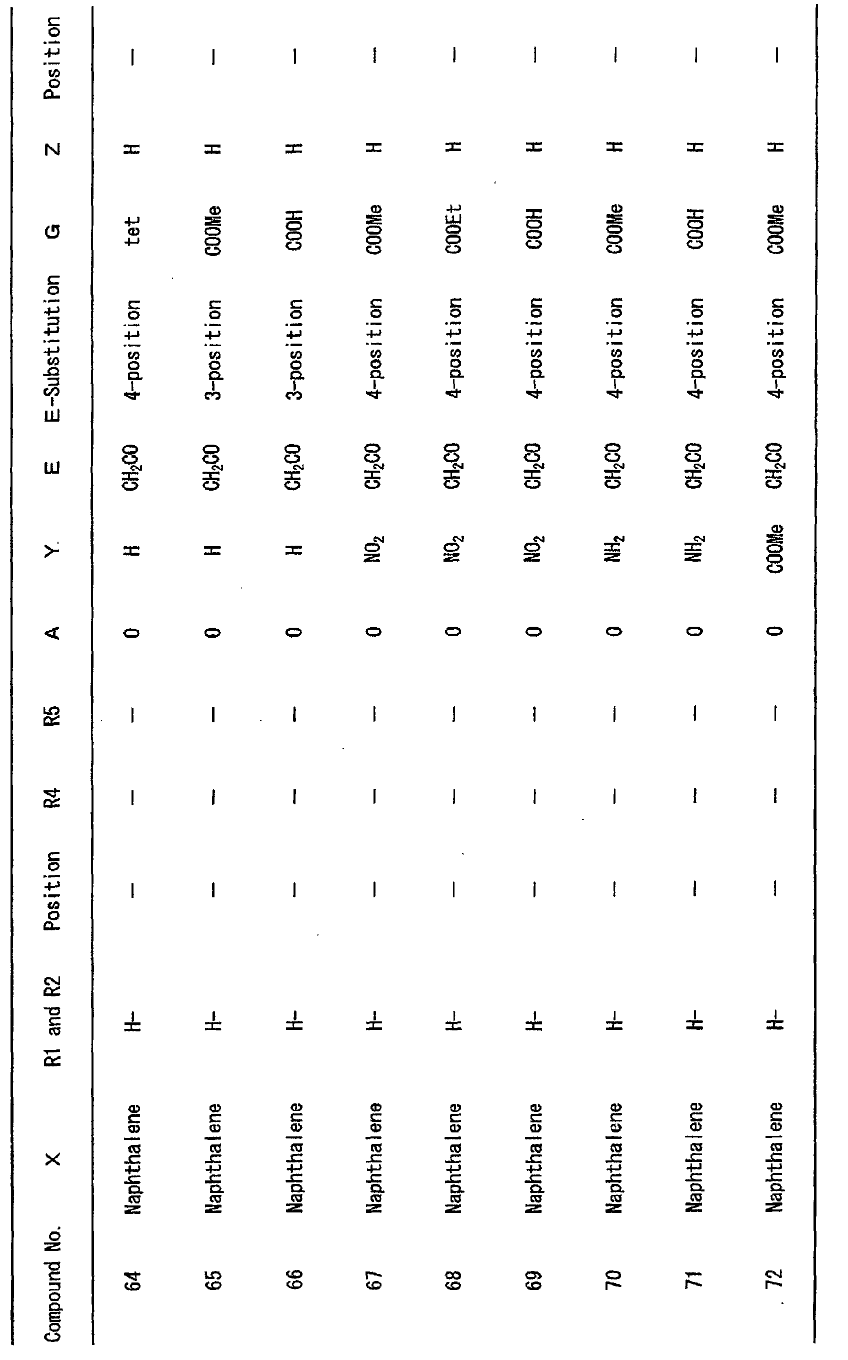

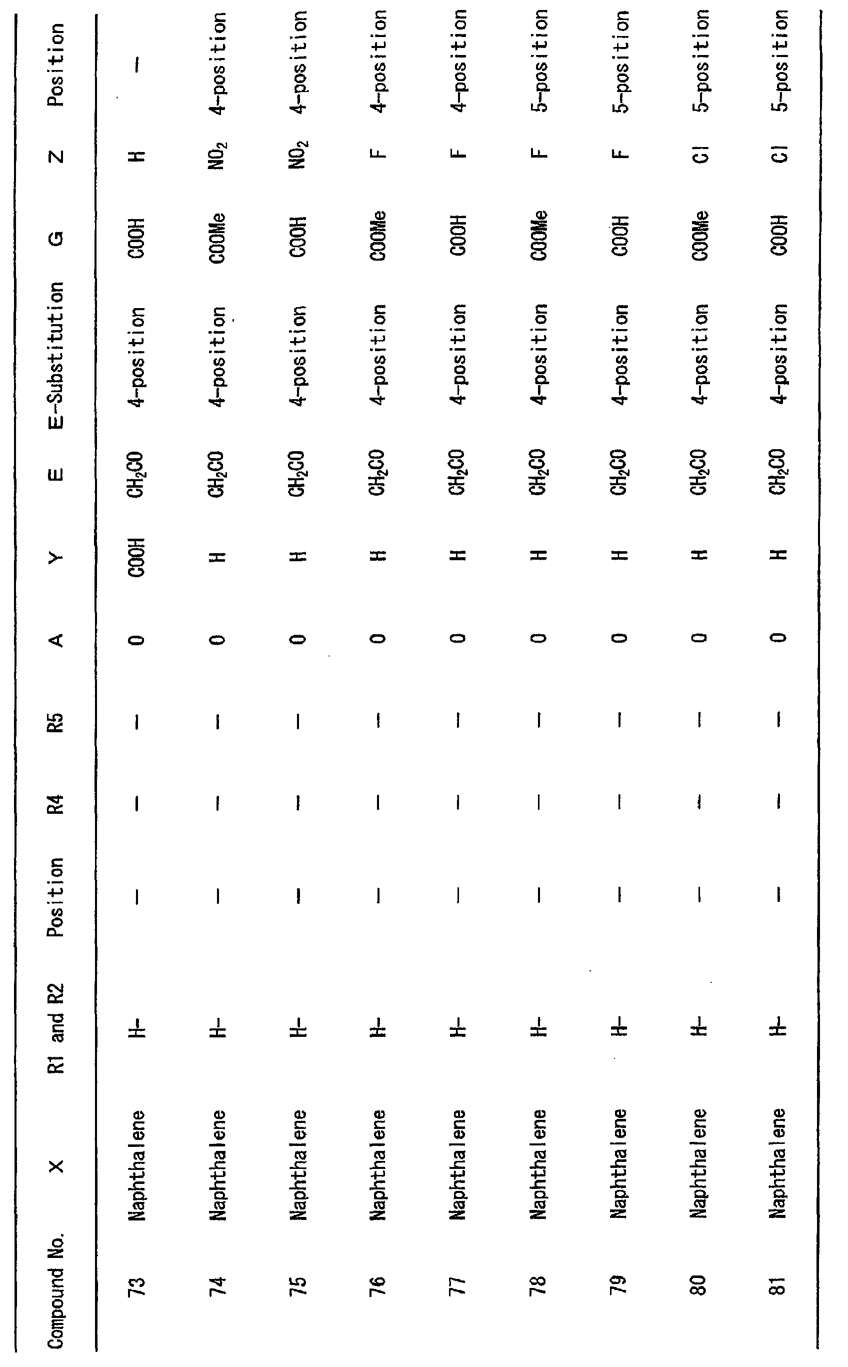

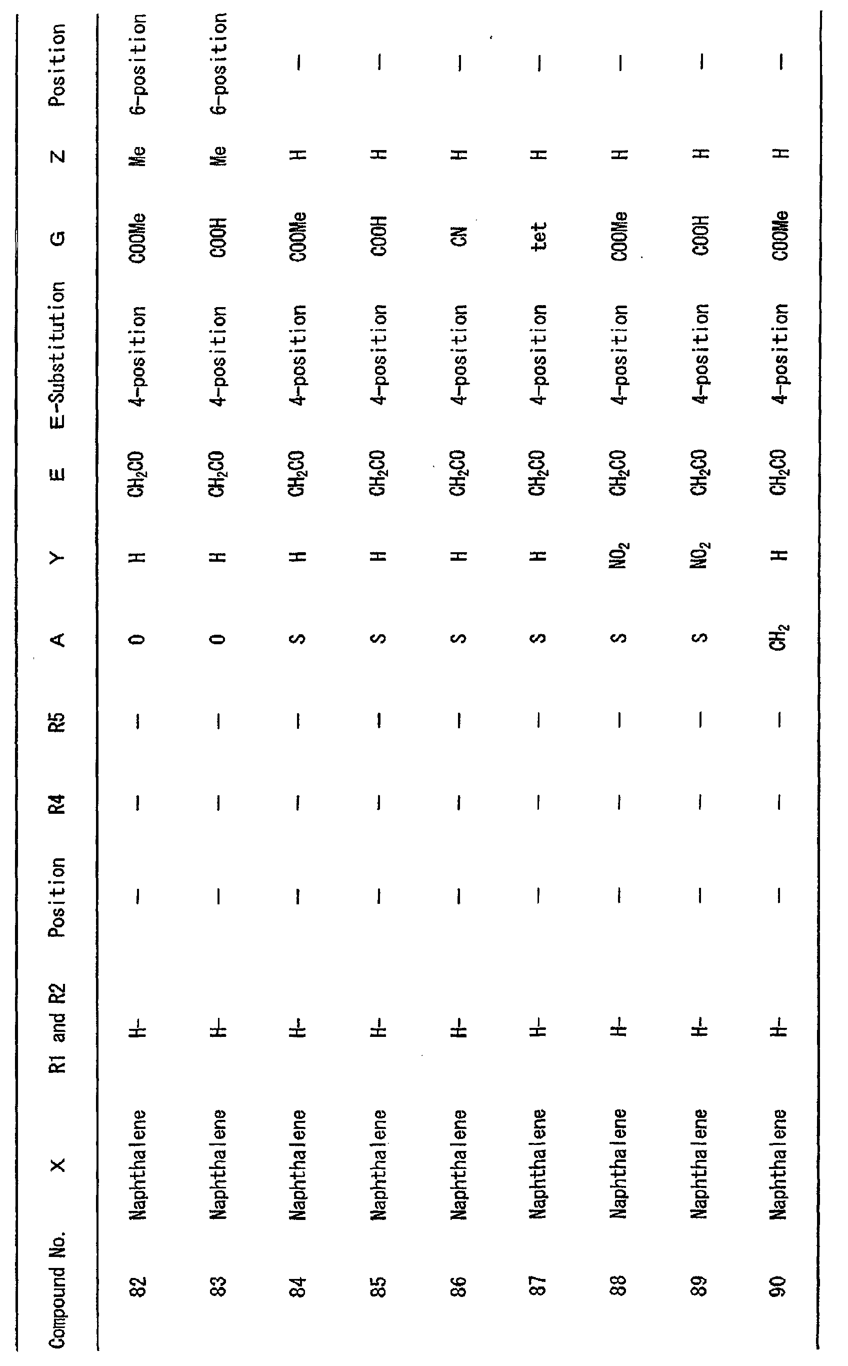

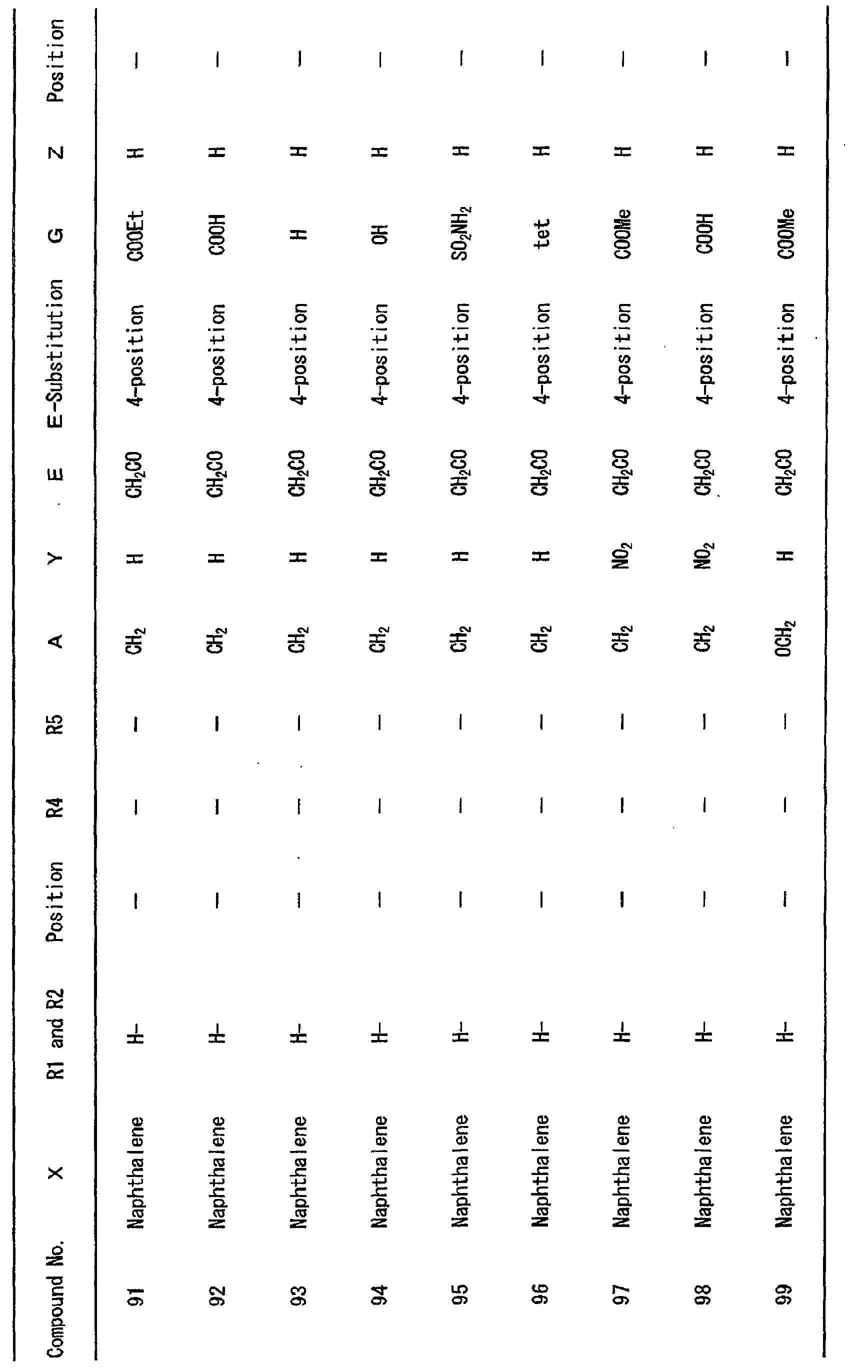

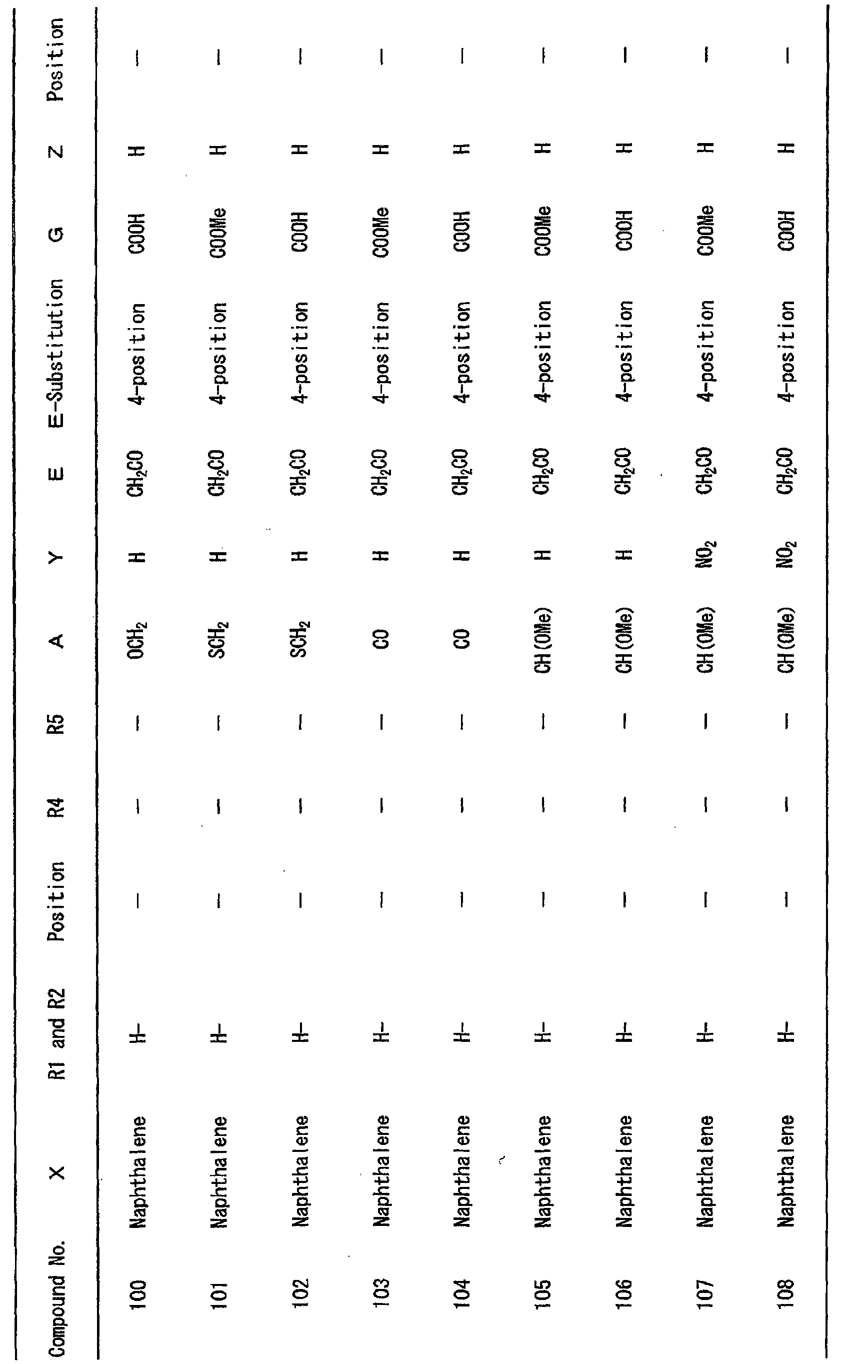

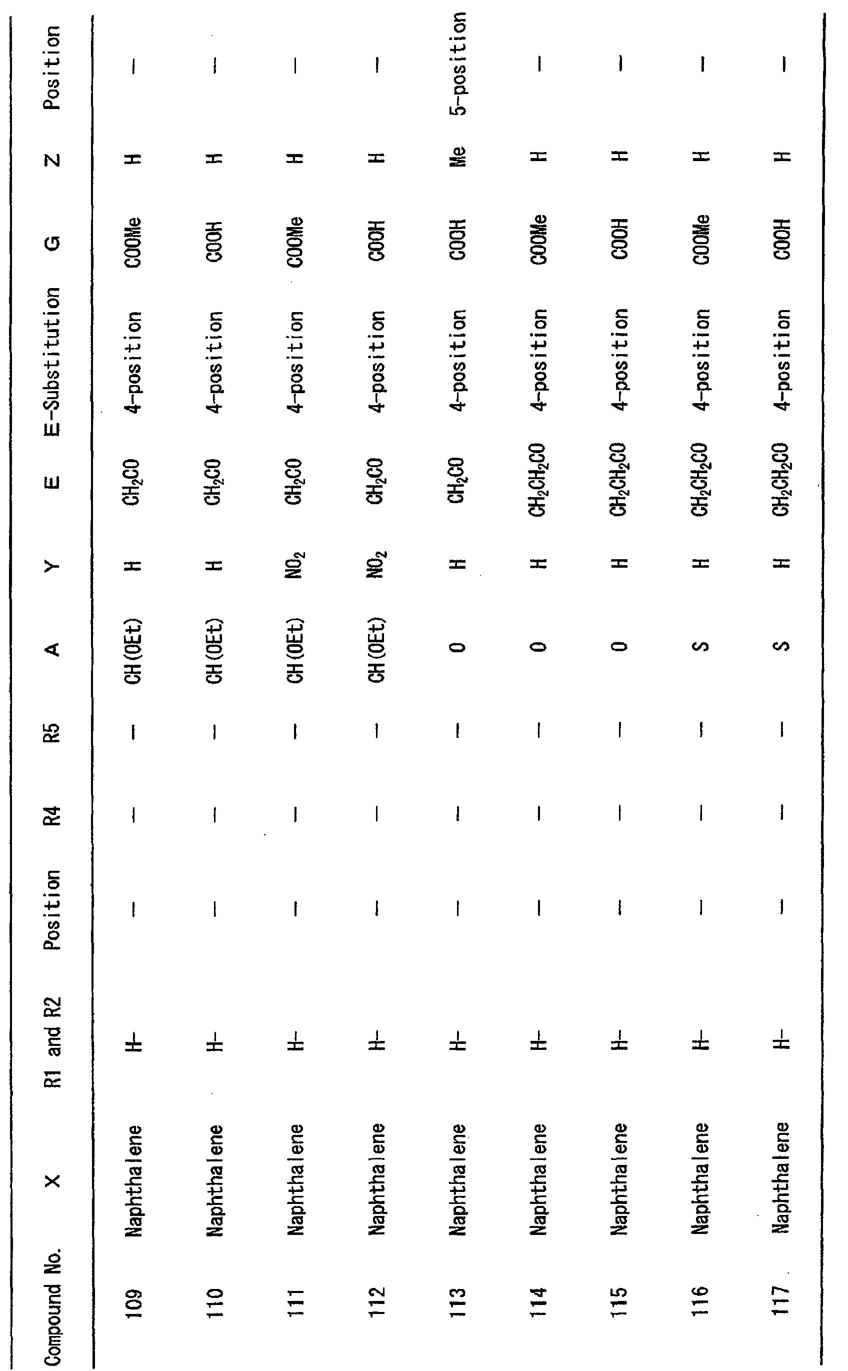

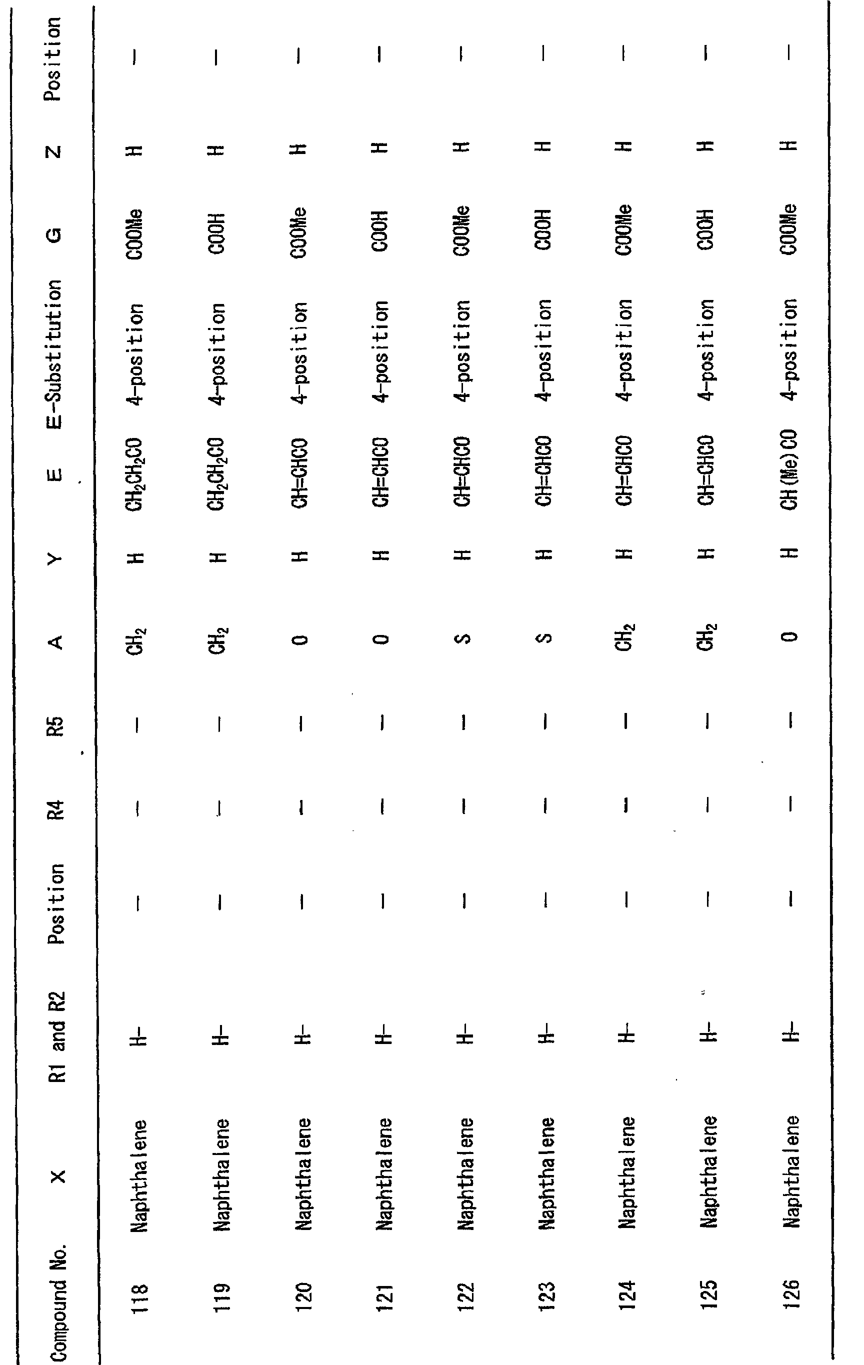

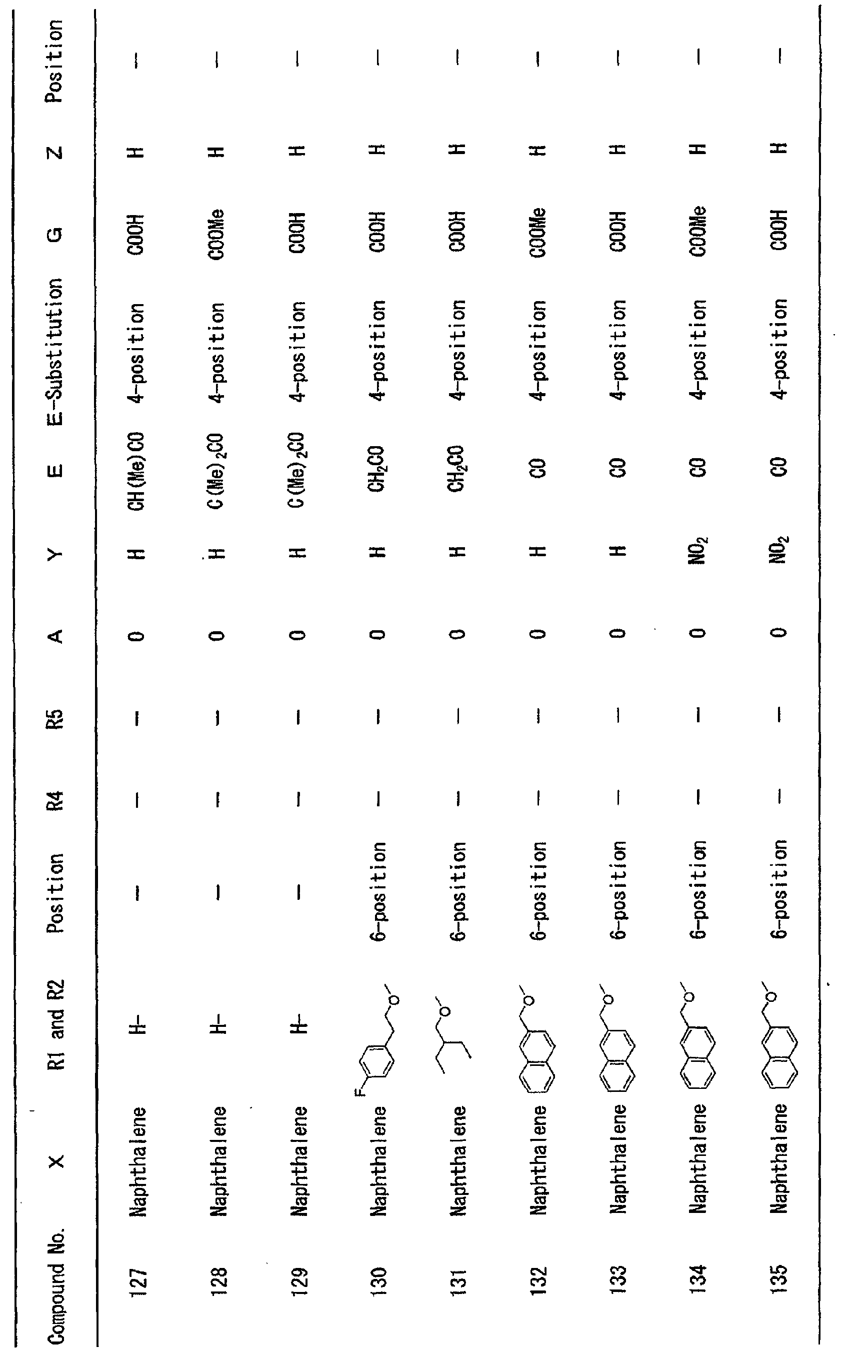

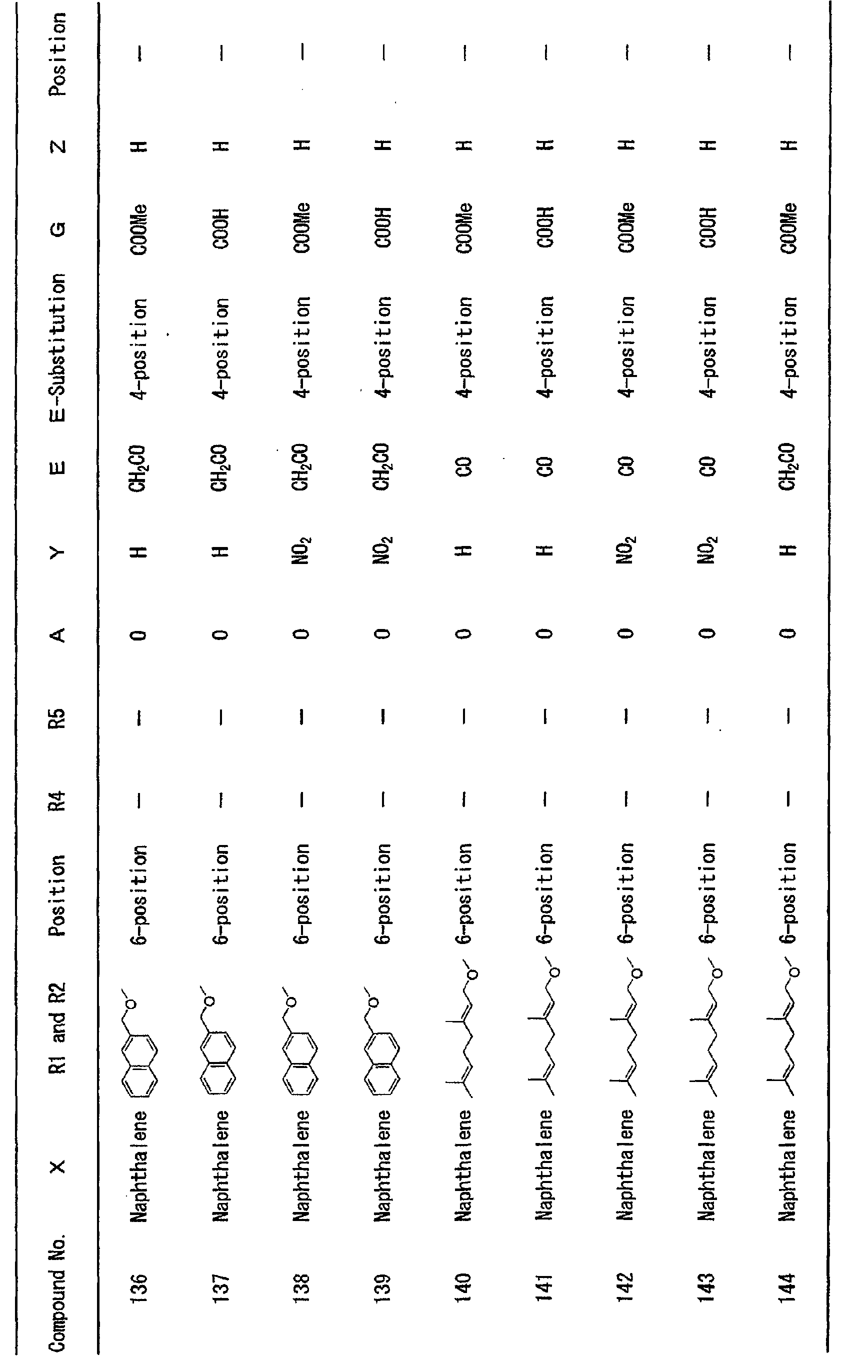

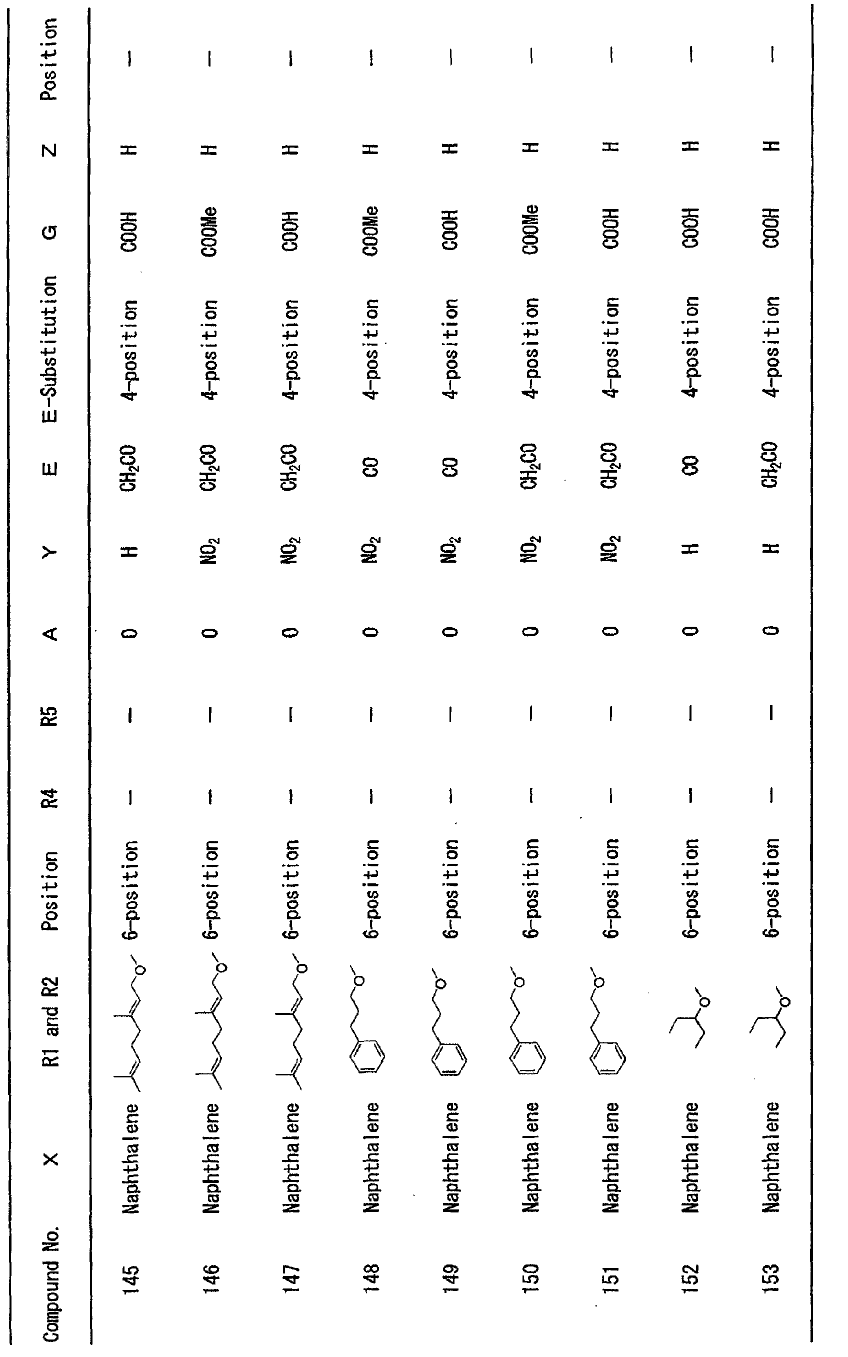

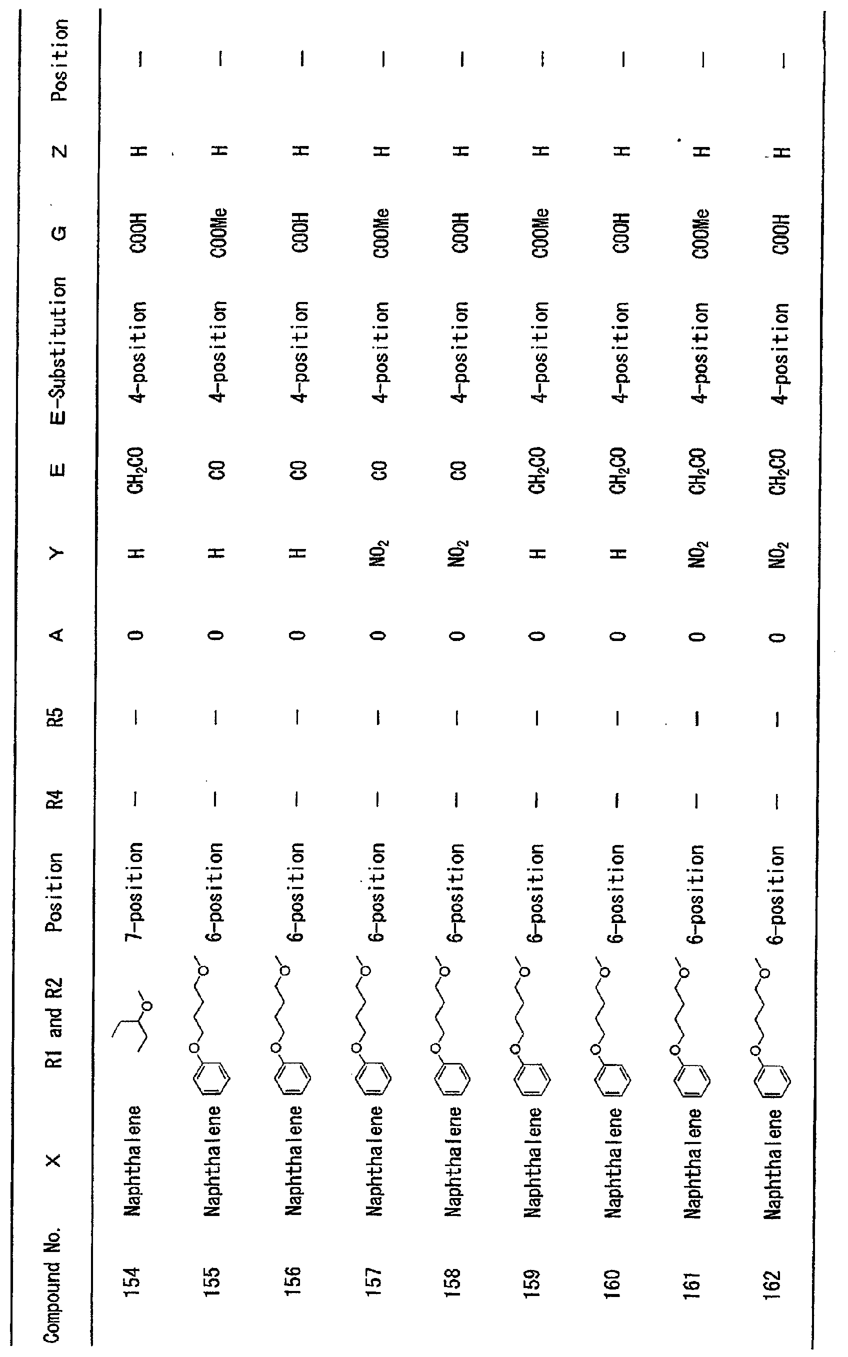

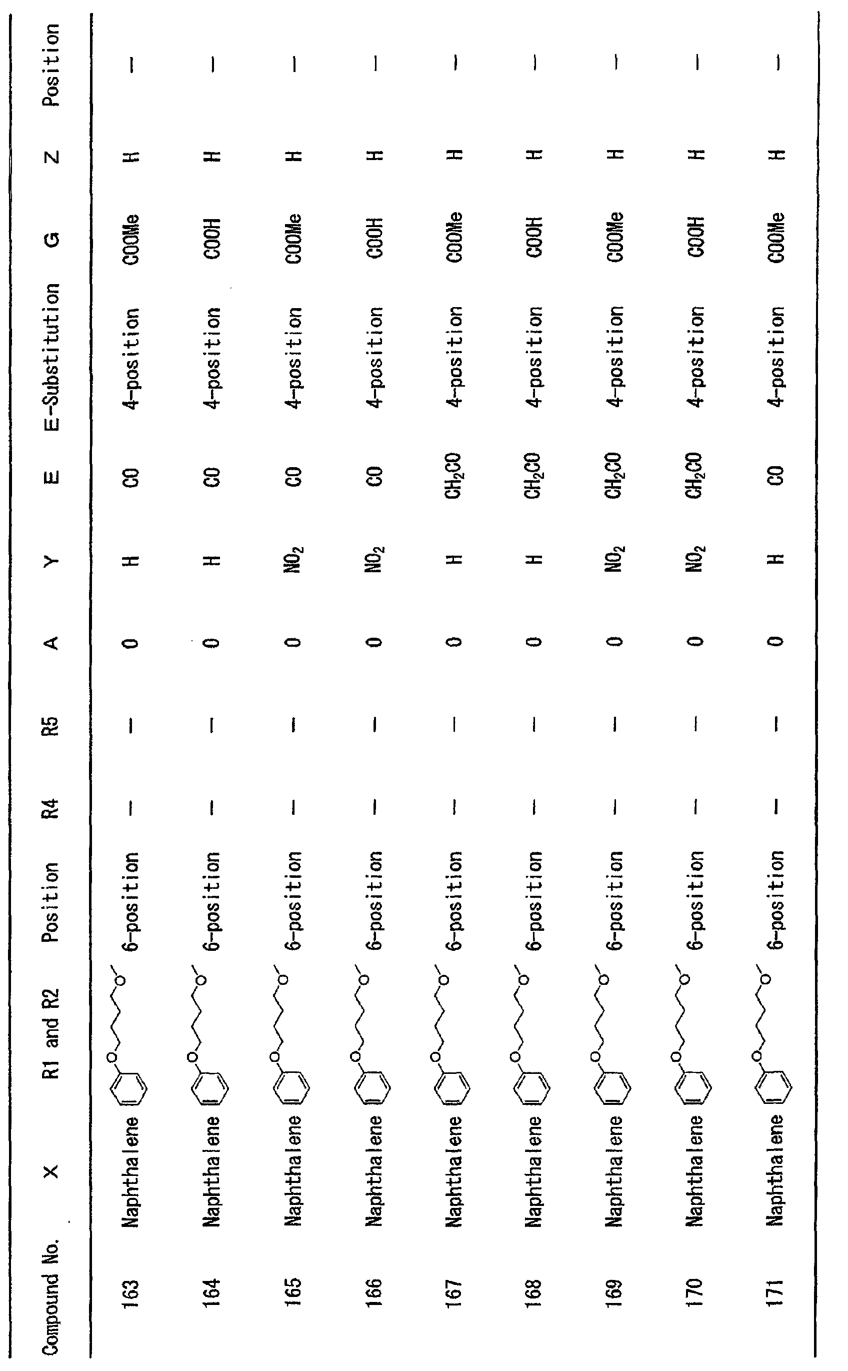

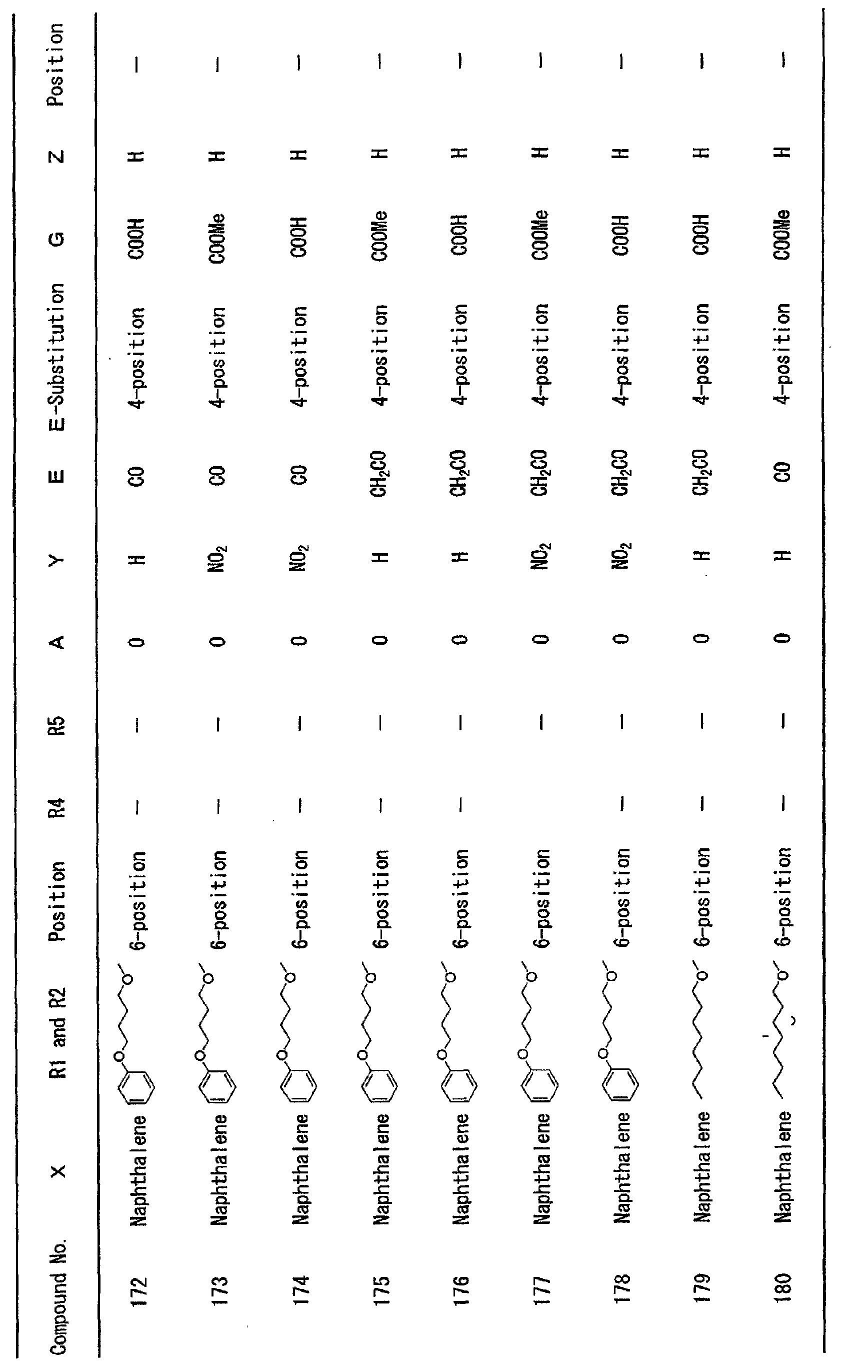

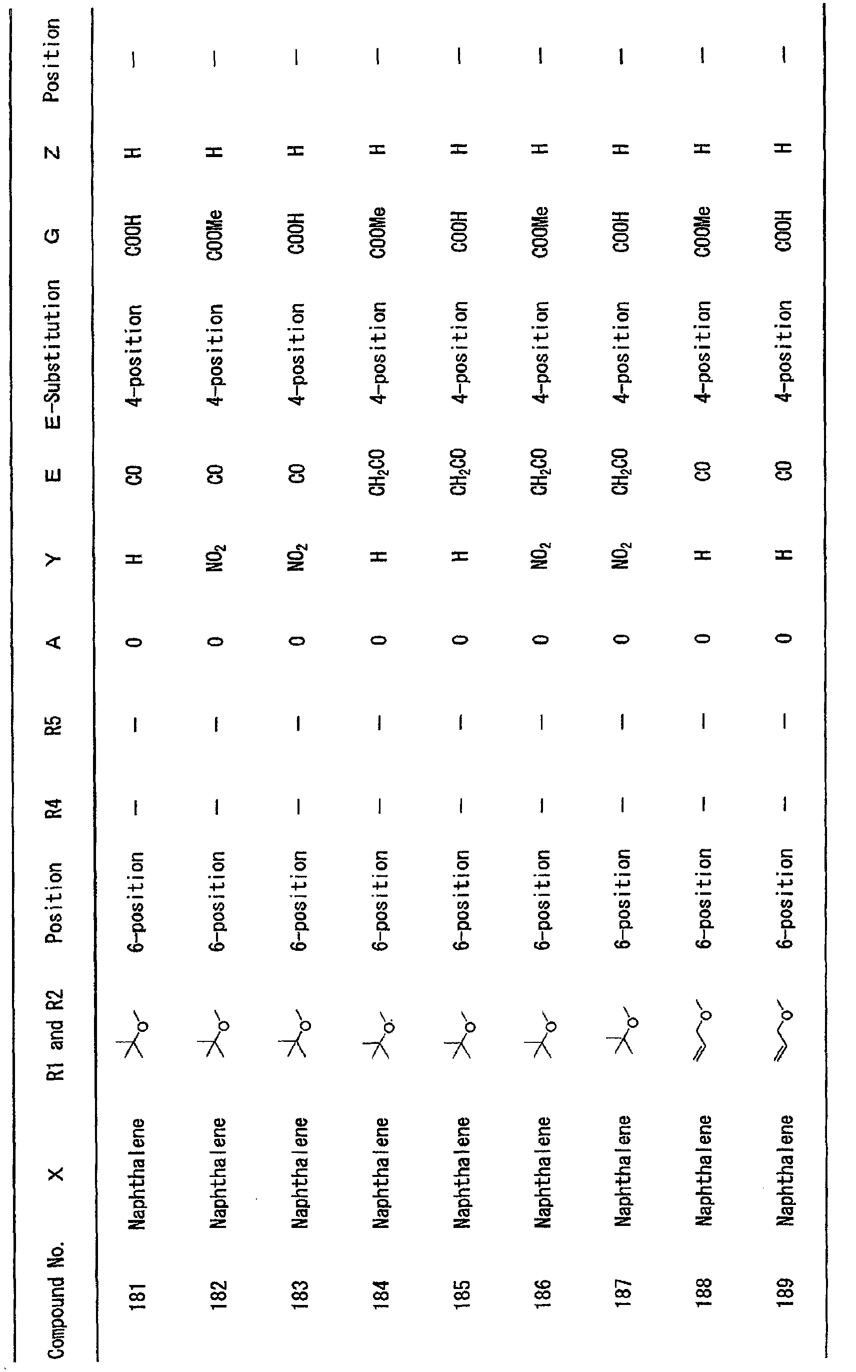

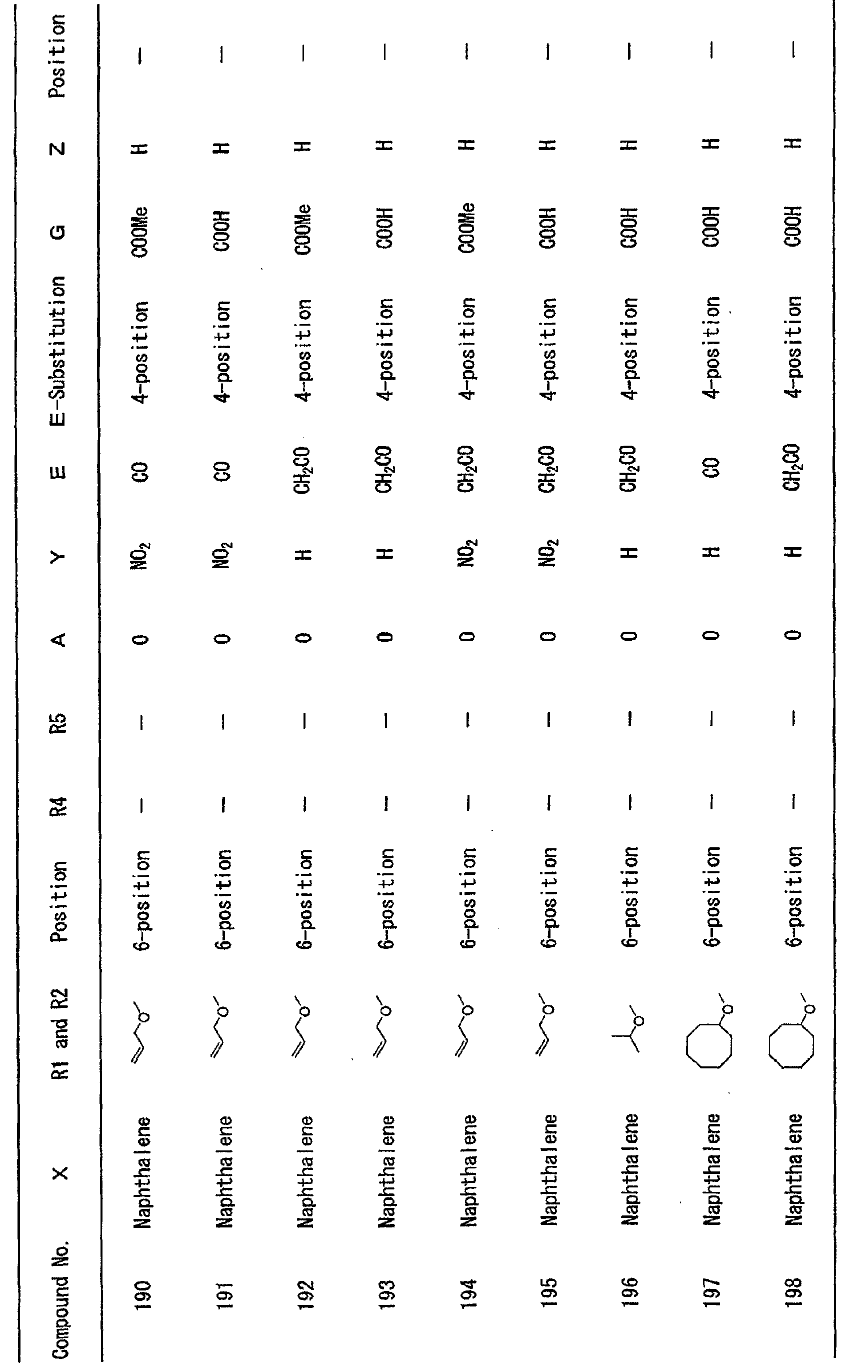

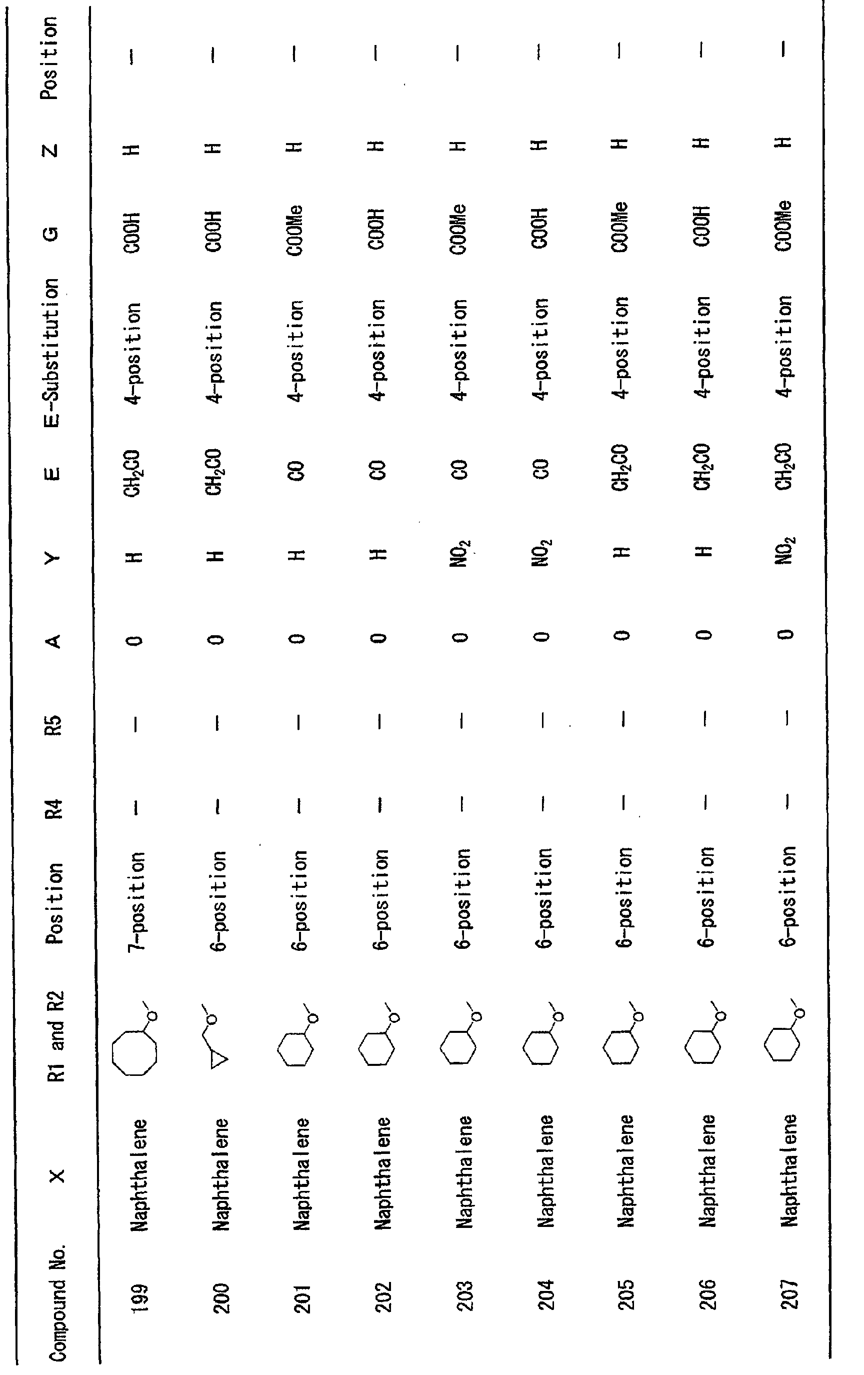

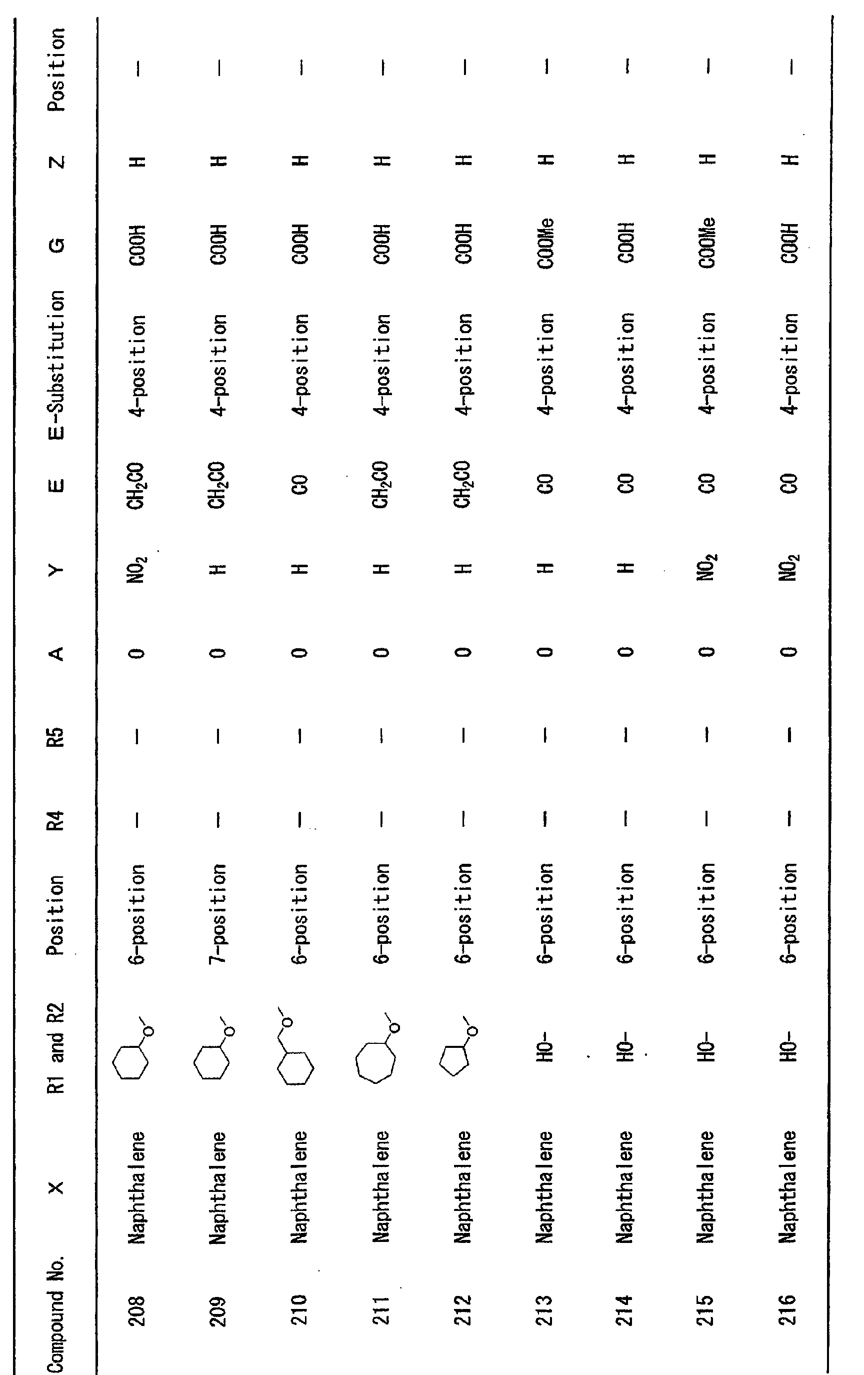

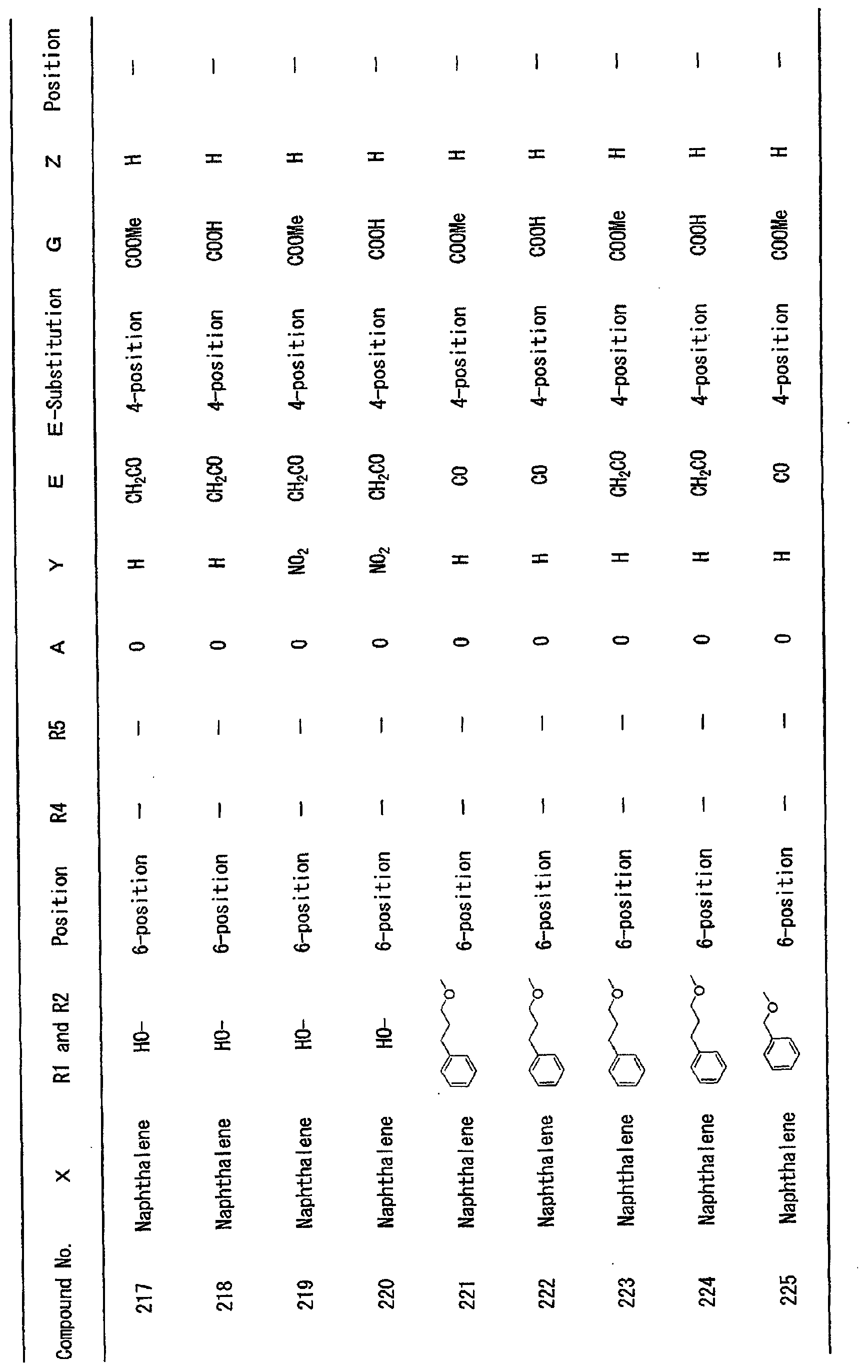

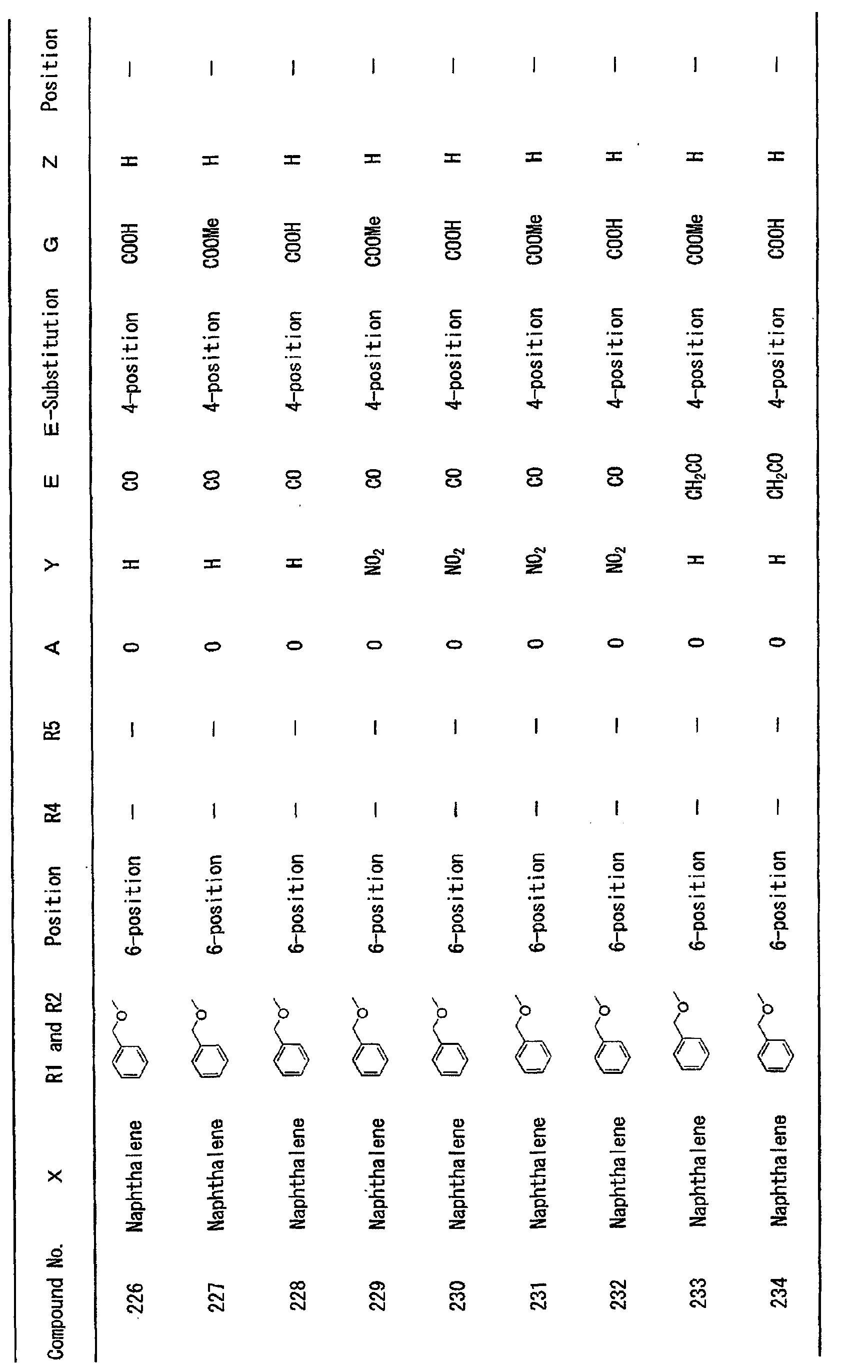

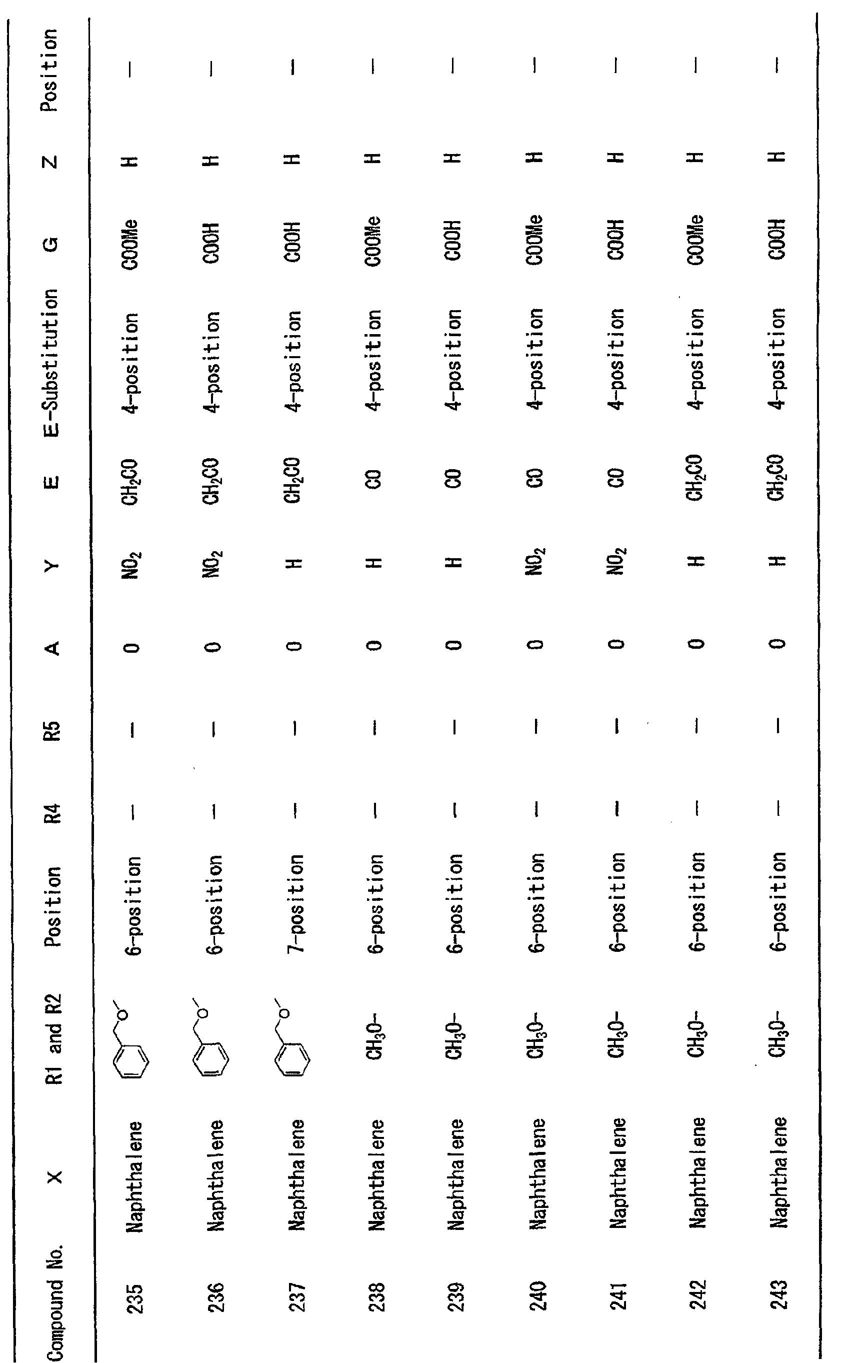

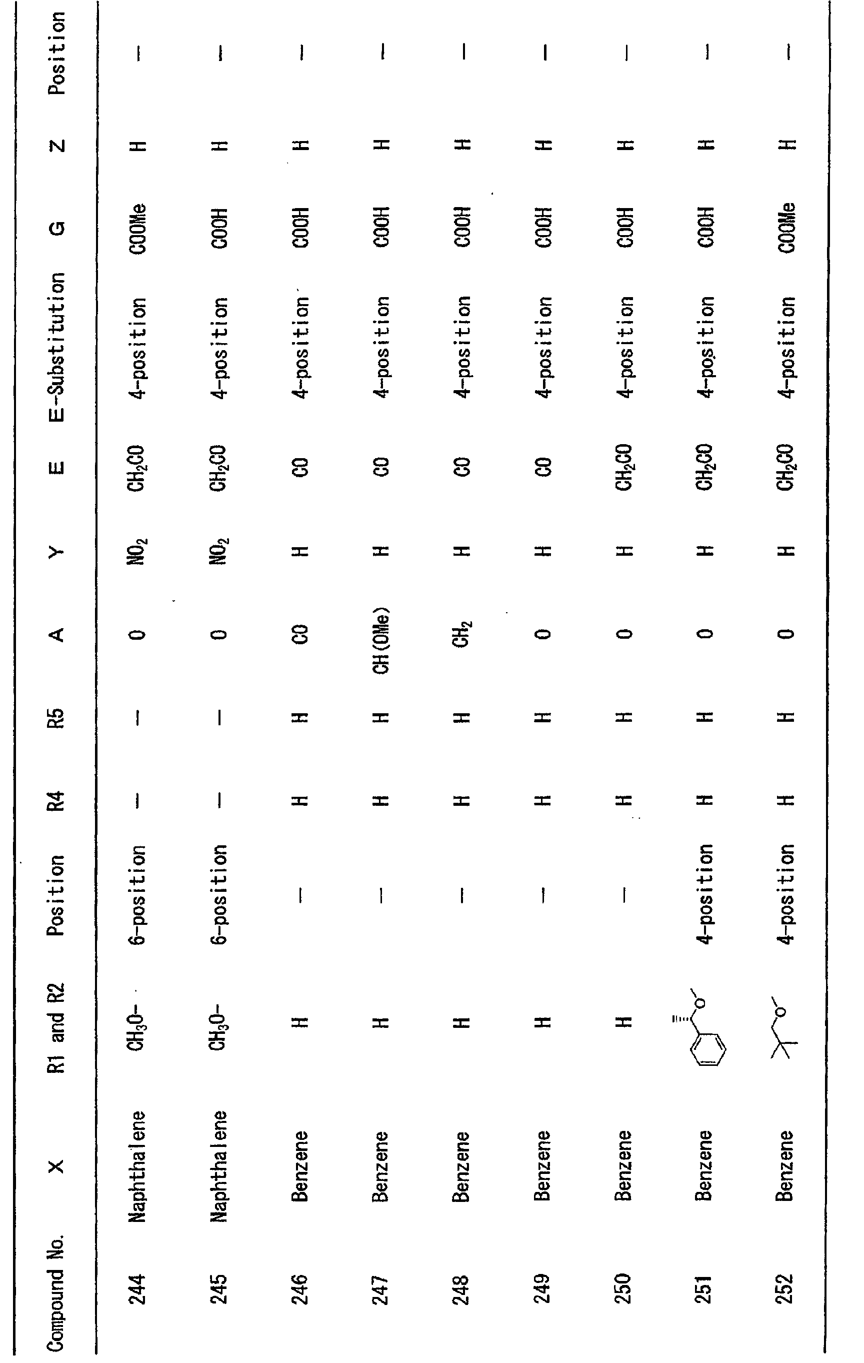

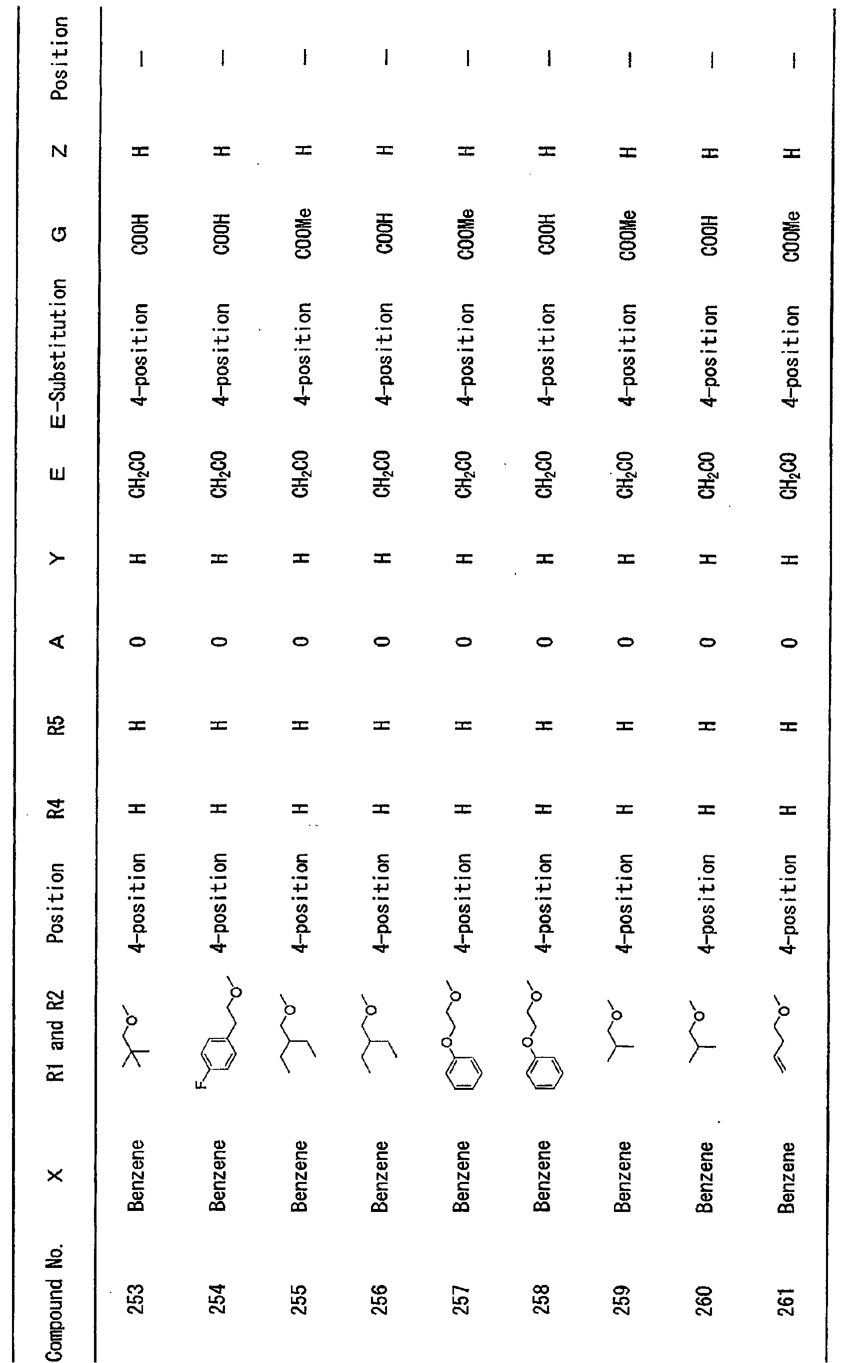

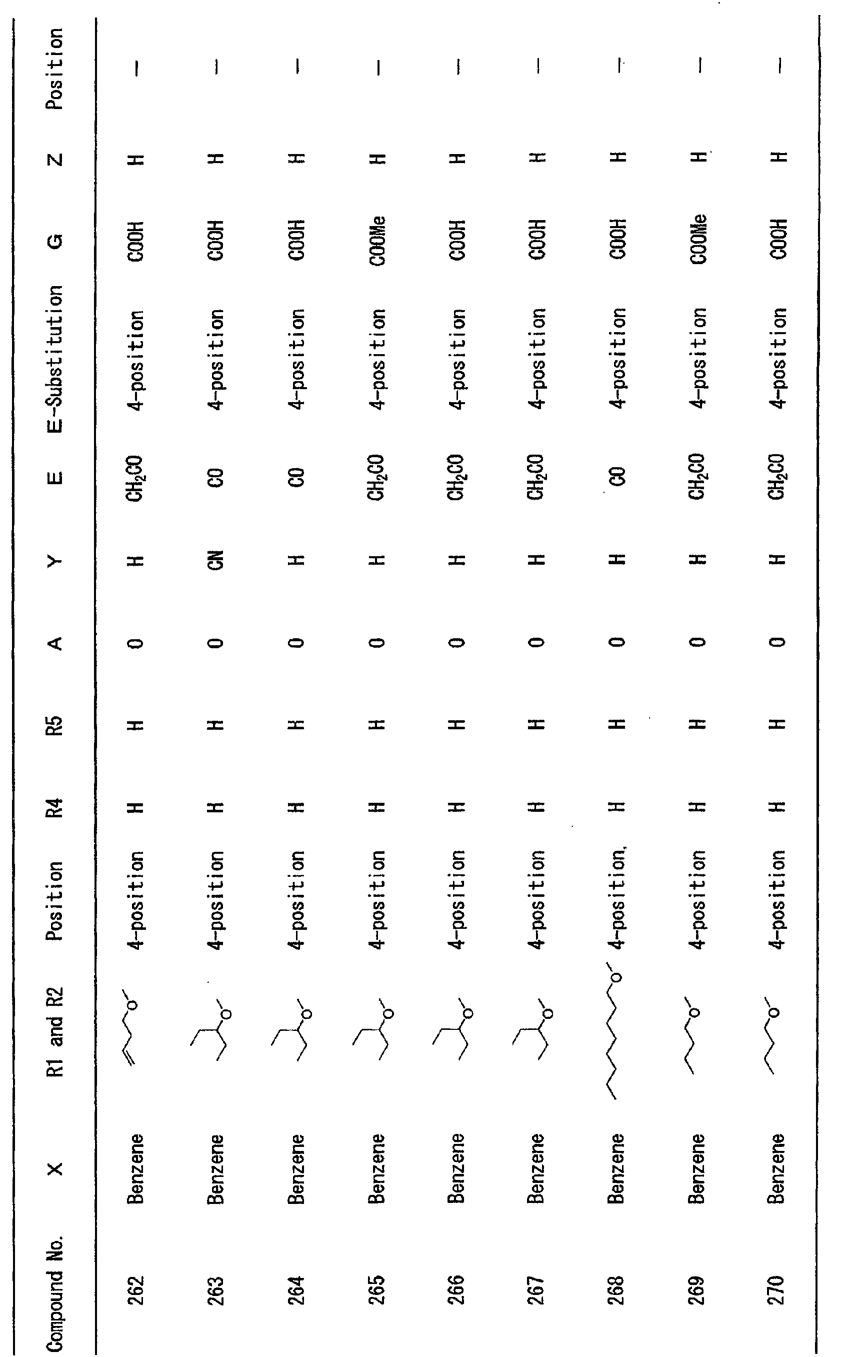

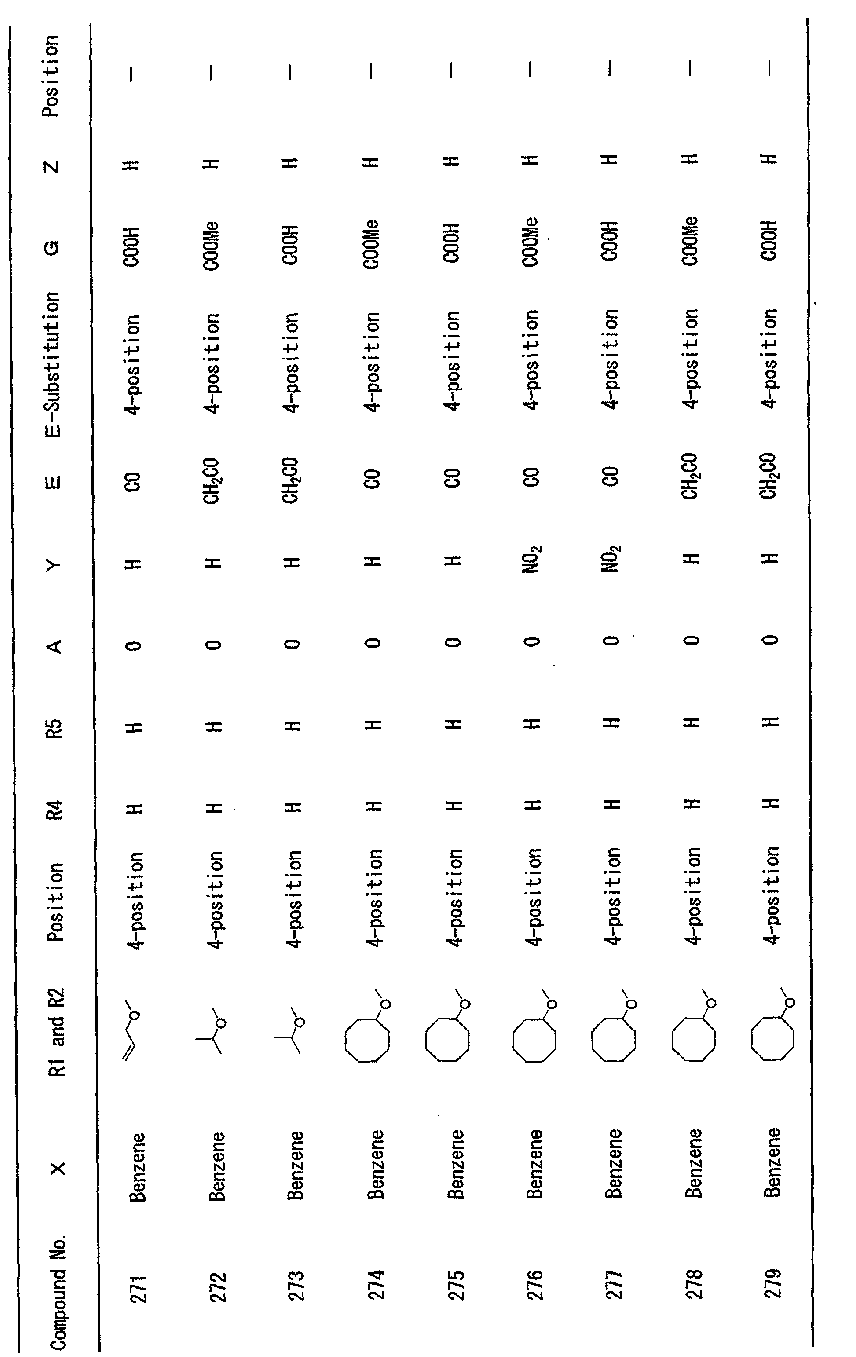

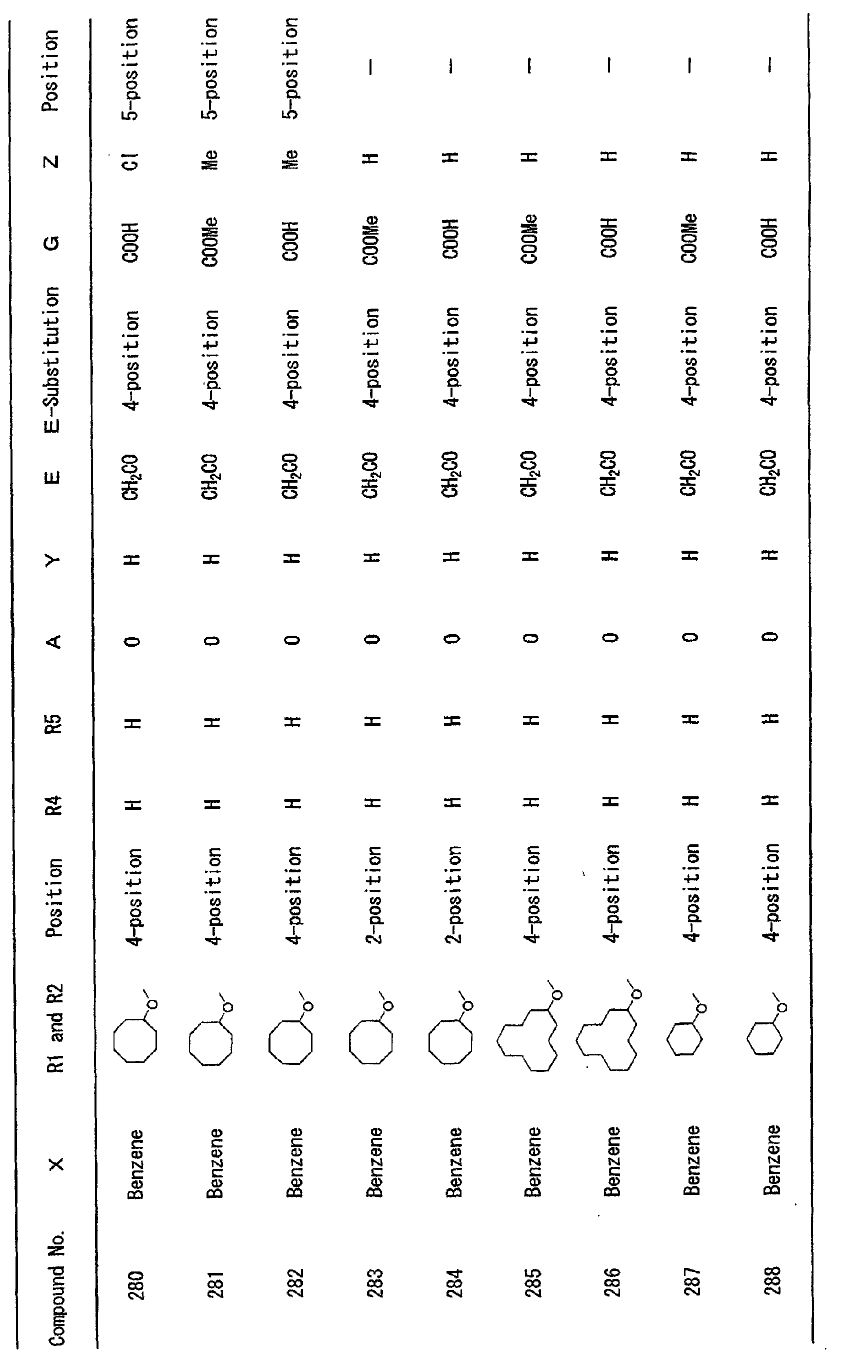

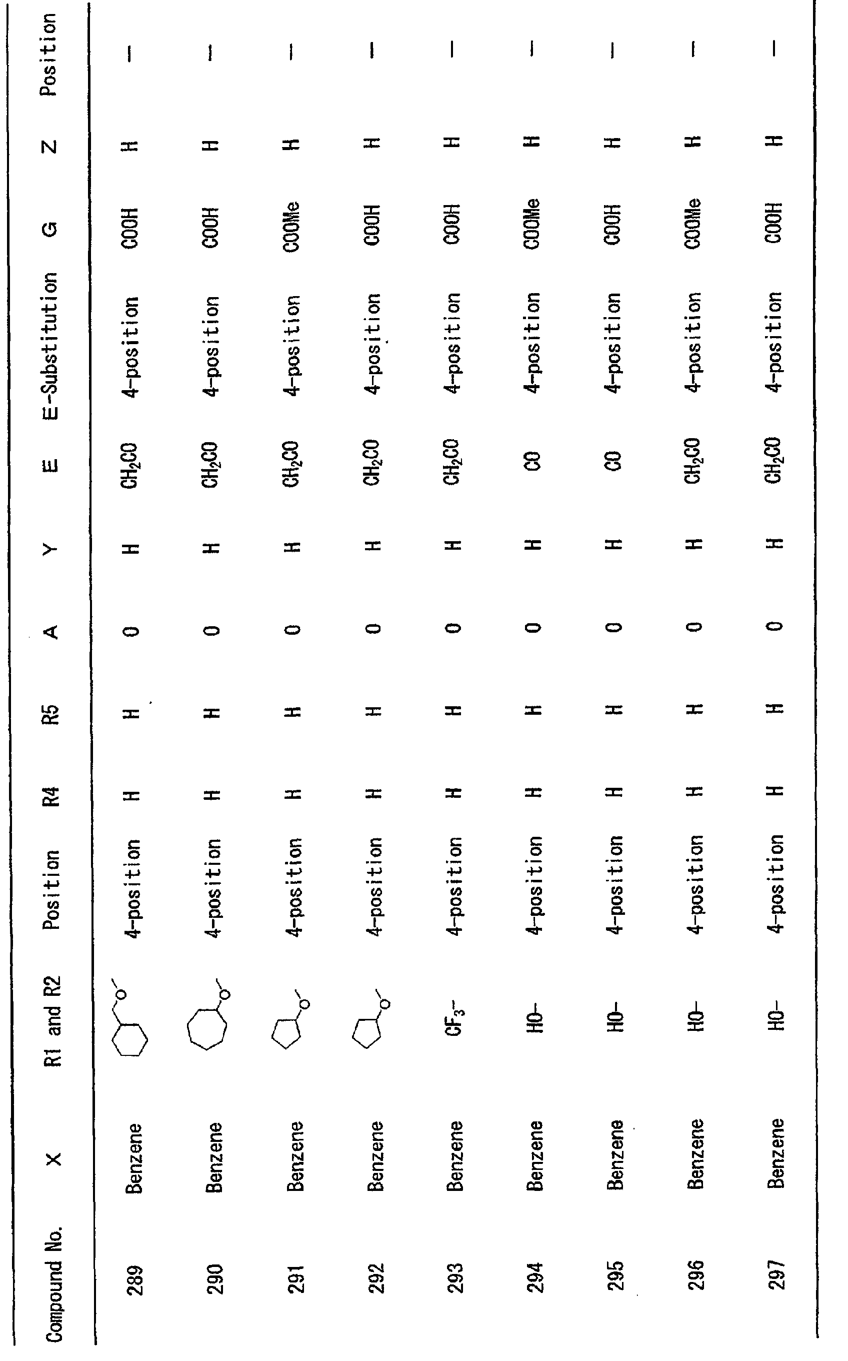

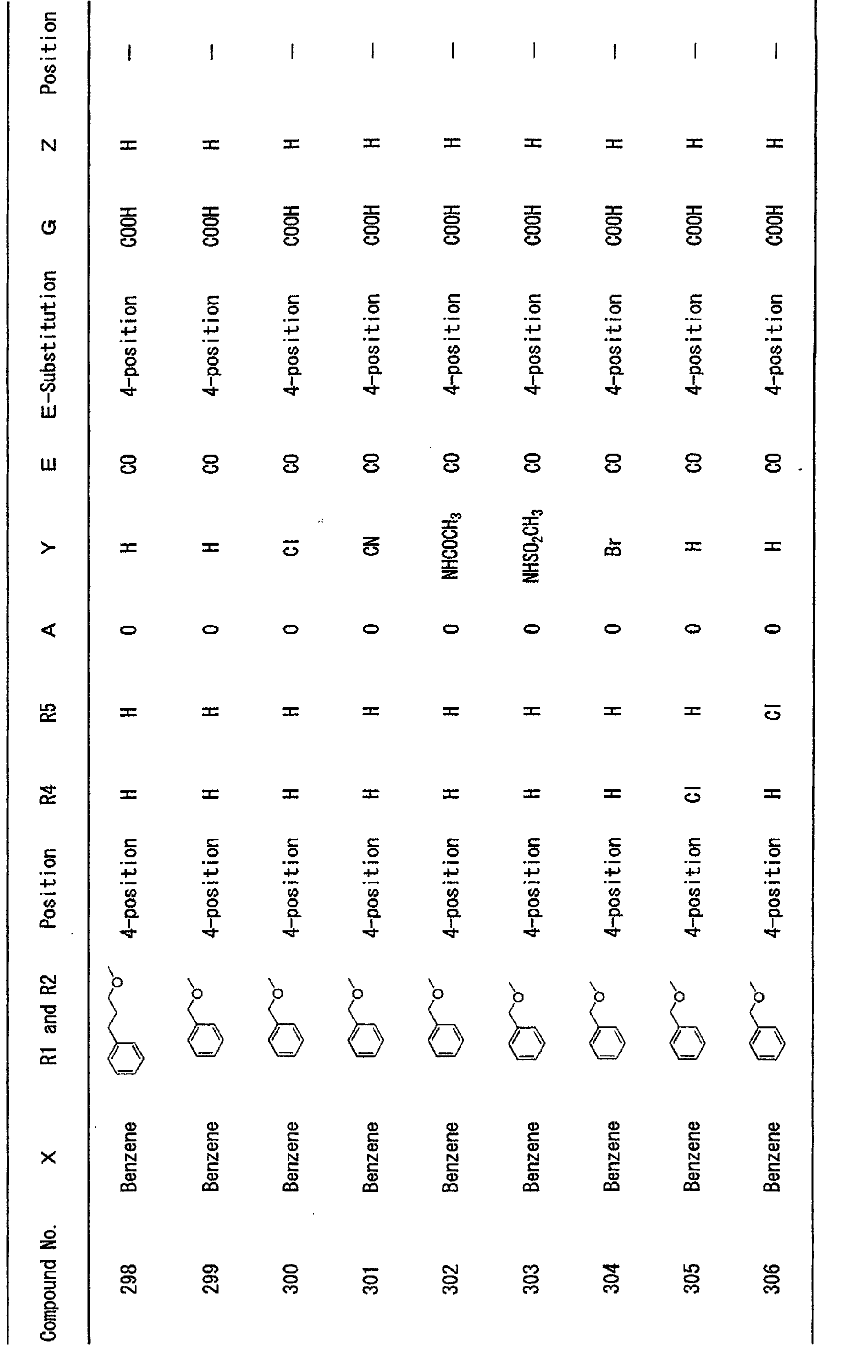

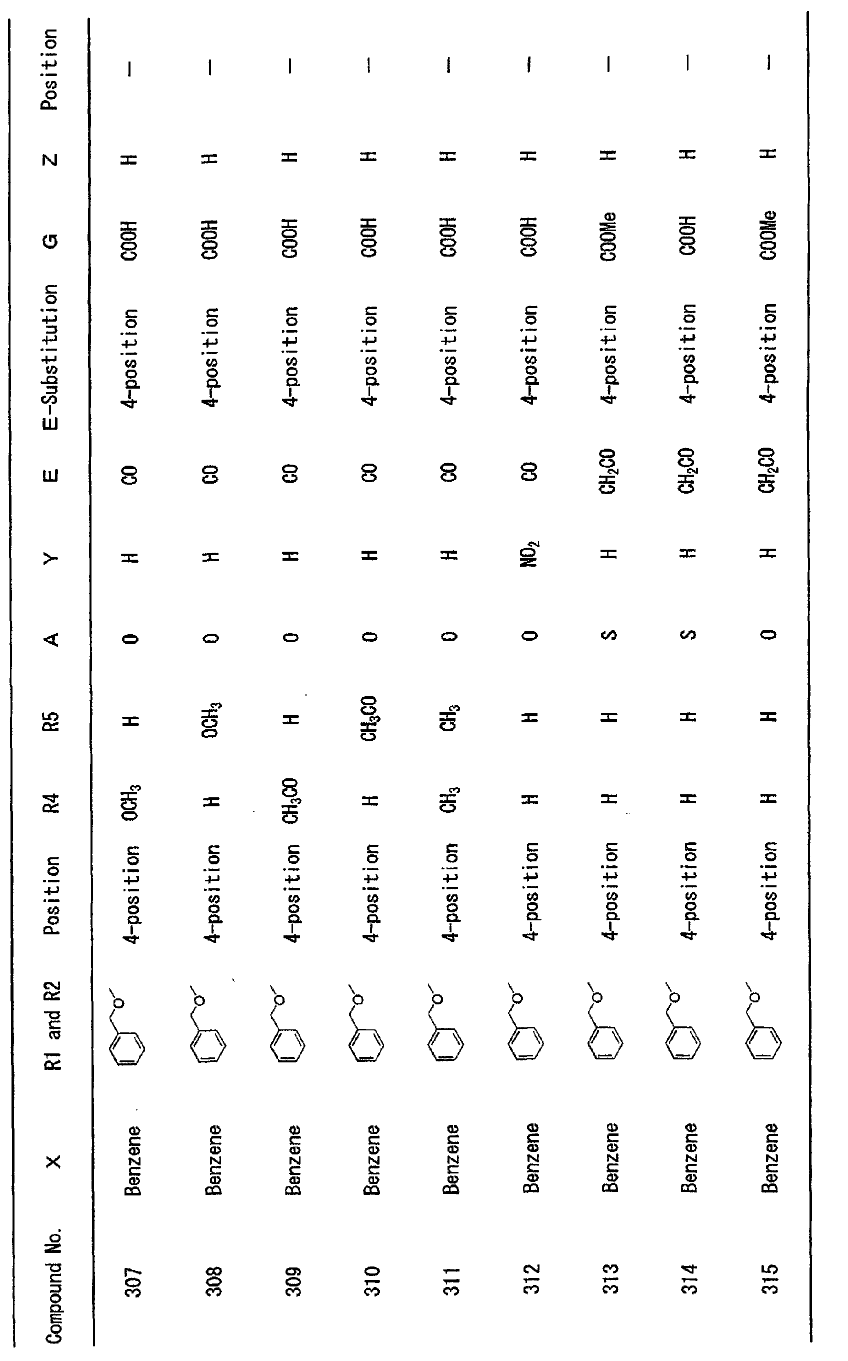

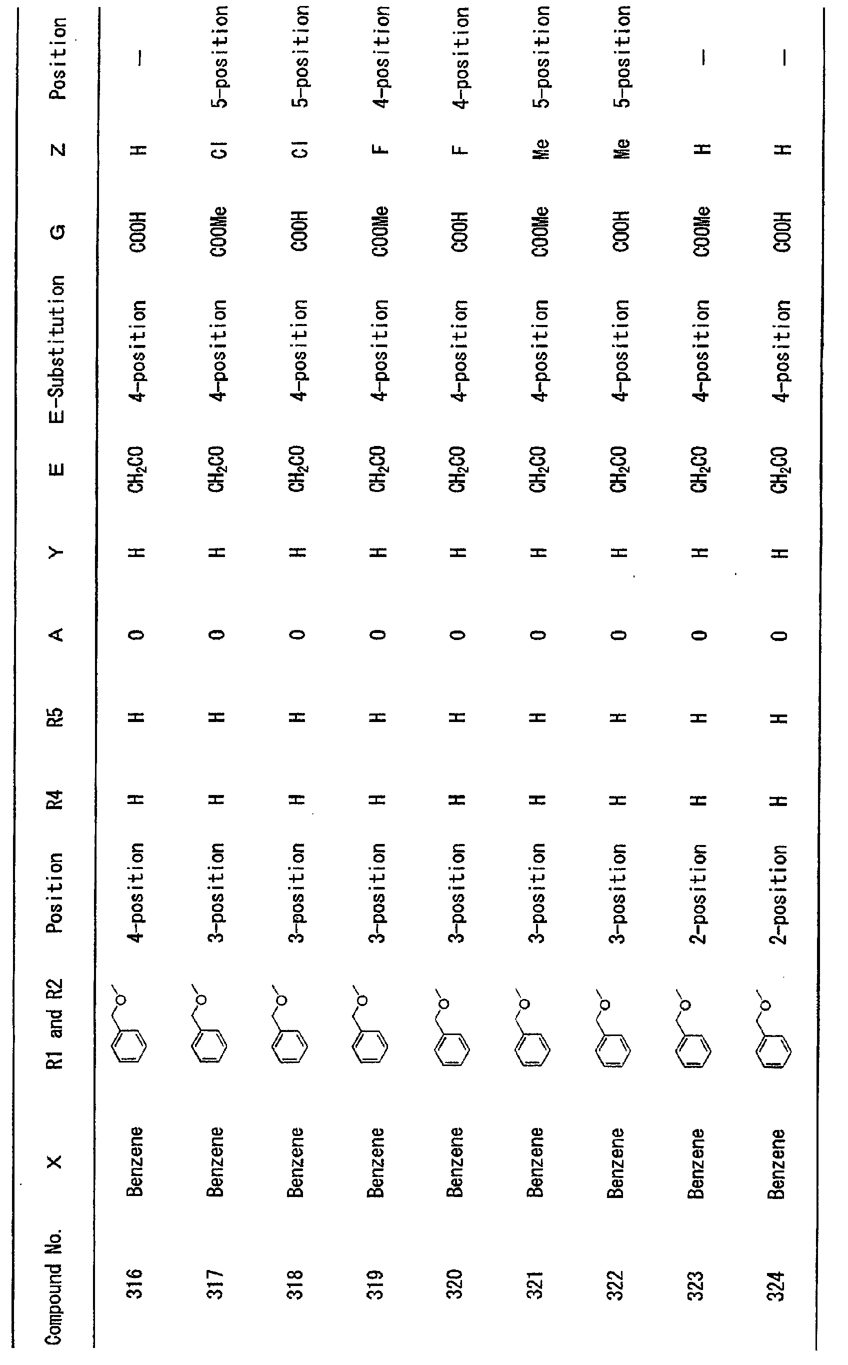

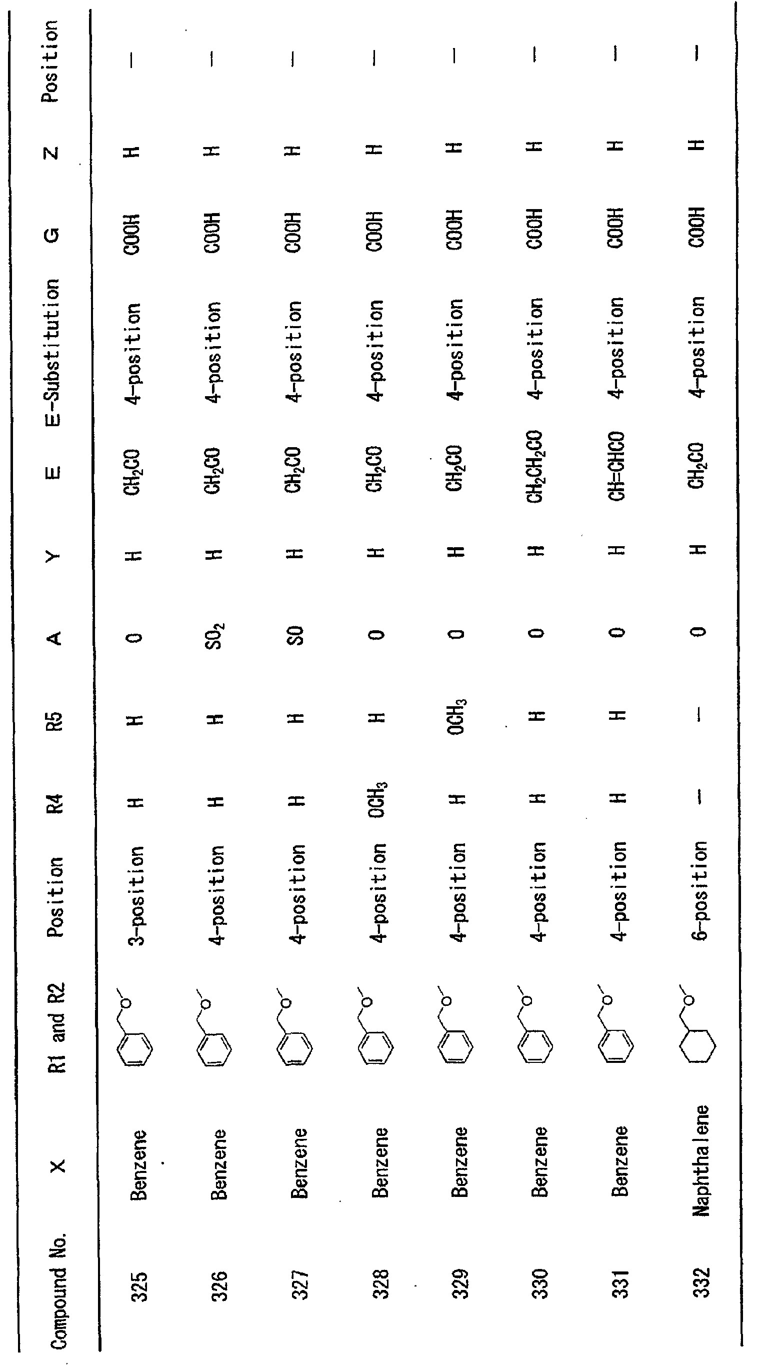

The anthranilic acid derivative that is an active ingredient of the cancer remedy of the present invention has a strong cytotoxic activity as described in the Examples hereinafter. Specifically, the anthranilic acid derivative has an LC₅₀ or a GI₅₀ of 5µM or below, preferably 0.05 nM or above and 1µM or below, more preferably 0.05 nM or above and 500 nM or below.

The anthranilic acid derivative having the excellent cytotoxic activity can be used as an active ingredient of the remedy clinically applicable to cancer.

Furthermore, the anthranilic acid derivative or a pharmaceutically acceptable salt thereof represented by the formula (1) can be produced if the persons are those skilled in the art by referring to WO95/32943 and WO97/19910. Namely, as shown in the following scheme, the objective compound represented by the following formula [I] can be obtained by condensing a carboxylic acid [II] having a naphthalene skeleton or a carboxylic acid [III] having a benzene skeleton with an aniline derivative [IV].

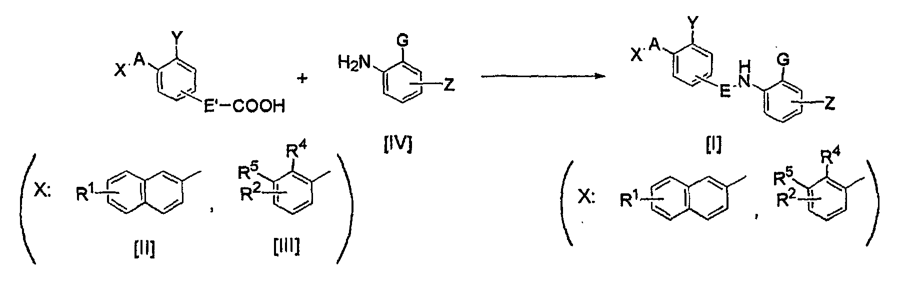

R¹, R², X, A, Y, E, G and Z in each formula mentioned above are the same as defined above. E' represents a single bond or a bond; CR¹⁰R¹¹⁻, -CH₂CH₂- or CH=CH-, wherein R¹⁰ and R¹¹ are the same as defined above. The compounds, which are starting materials, represented by the formula [II] and formula [III] can be obtained according to a known method. The method for condensation represents roughly classified into a method for passing through an acid halide and a method without passing through the acid halide. Both the methods are basically known.

When the acid halide is passed, the compound [I] can be obtained by reacting the compound [II] or [III] with oxalyl chloride or thionyl chloride in the presence or absence of an additive such as DMF, producing the acid halide of the compound [II] or [III] and reacting the resultant acid halide with the compound [IV] in the presence or absence of a base.

On the other hand, in the method without passing through the acid halide, the compound [I] can be obtained by activating the compound [II] or [III] using various activators such as mixed acid anhydrides, carbodiimides, imidazole-forming agent, halophosphoric esters or cyanophosphoric esters and reacting the activated compound [II] or [III] with the compound [IV].

In the compound [I] thus obtained, when Y represents -COOR⁷ and R⁷ represents a lower alkyl group or G represents -COOR³ and R³ represents a lower alkyl group, the compound [I], if necessary, can be hydrolyzed under acidic or basic conditions and converted into a compound wherein R⁷ or R³ represents a hydrogen atom.

In the compound [I] thus obtained, when G represents -CN, the compound [I], if necessary, can be subjected to a treatment such as reaction with an azide compound and converted into a compound wherein G represents a tetrazol-5-yl group.

Furthermore, the compound [I] thus obtained (when Y represents -COOR⁷ and R⁷ represents a hydrogen atom or when G represents -COOR³ and R³ represents a hydrogen atom or G represents the tetrazol-5-yl group), if necessary, can be converted into the pharmaceutically acceptable salt described above.

Therefore, the anthtranilic acid derivative represented by the formula (1) or a pharmaceutically acceptable salt thereof which is an active ingredient of the cancer remedy of the present invention can be obtained.

The cancer remedy of the present invention can be administered orally or parenterally such as intravenously, subcutaneously, intramuscularly, percutaneously, intrarectally or by instillation or by inhalation.

Examples of the dosage form for oral administration include a tablet, a pill, a granule, a powder, a solution, a suspension, a syrup, a capsule and the like.

The tablet form can be produced according to a conventional method using, for example a vehicle such as lactose, starch or crystalline cellulose; a binder such as carboxymethyl cellulose, methyl cellulose or polyvinylpyrrolidone; a disintegrating agent such as sodium alginate, sodium hydrogencarbonate or sodium lauryl sulfate.

The pill, granule and powder can similarly be formed according to a conventional method using the vehicle and the like.

The solution, suspension and syrup can be formed according to a conventional method using glycerol esters, for example tricaprylin or triacetin; alcohols, for example ethanol; water; vegetable oils, for example corn oil, cottonseed oil, coconut oil, almond oil, peanut oil and olive oil.

The capsule is formed by filling a granule, a powder, a solution or the like in a capsule such as gelatin.

The dosage form for intravenous, subcutaneous or intramuscular administration includes a parenteral injection in the form of an aseptic aqueous or nonaqueous solution or the like. For example, an isotonic sodium chloride solution is used as the aqueous solution. For example, propylene glycol, polyethylene glycol, a vegetable oil such as olive oil and an injectable organic ester such as ethyl oleate are used as the nonaqueous solution. An isotonic agent, a preservative, a wetting agent, an emulsifying agent, a dispersing agent, a stabilizer and the like, if necessary, are added to the pharmaceutical preparation and the resulting pharmaceutical preparation can be sterilized by suitably carrying out treatment such as filtration through a bacterial filter, formulation of a disinfectant, heating, irradiation or the like. An aseptic solid pharmaceutical preparation is produced and can be used by dissolving the resulting pharmaceutical preparation in aseptic water or an aseptic solvent for injection just before use.

Examples of the dosage form for percutaneous administration include an ointment and a cream. The ointment is formed by using oils and fats such as castor oil and olive oil; vaseline and the like. The cream is formed according to a conventional method using a fatty oil; diethylene glycol; an emulsifying agent such as a sorbitan monofatty acid ester, and the like.

A usual suppository such as a gelatin soft capsule is used for rectal administration.

The dosage form of the eye drop includes an aqueous or a nonaqueous eye drop. Sterilized purified water, an isotonic sodium chloride solution or a suitable aqueous solvent is used as a solvent in the aqueous eye drop, and examples of the eye drop include an aqueous eye drop using only sterilized purified water as the solvent; a viscous eye drop prepared by adding a mucilage such as carboxymethyl cellulose, methyl cellulose, hydroxypropyl cellulose or polyvinylpyrrolidone; an aqueous suspension eye drop obtained by adding a suspending agent such as a surfactant or a polymeric thickener; a solubilized eye drop and the like prepared by adding a solubilizing agent such as a nonionic surfactant. The nonaqueous eye drop uses a nonaqueous solvent for injection as the solvent, and examples of the nonaqueous eye drop include a nonaqueous eye drop using a vegetable oil, a liquid paraffin, a mineral oil, proplylene glycol or the like; a nonaqueous suspension eye drop obtained by carrying out suspension using a thixotropic colloid such as aluminum monostearate and the like. An isotonic agent, a preservative, a buffer, an emulsifying agent, a stabilizer and the like, if necessary, can be added to the pharmaceutical preparation. The resulting pharmaceutical preparation can be sterilized by suitably carrying out treatment such as filtration through a bacterial filter, formulation of a disinfectant heating, irradiation or the like. An aseptic solid pharmaceutical preparation is produced and can be used by dissolving or suspending the pharmaceutical preparation in a suitable aseptic solution just before use.

Examples of the dosage form administered to eyes other than the eye drop include an ophthalmic ointment formed by using vaseline or the like; a liniment solution using a dilute iodine tincture, a zinc sulfate solution, a methylrosaniline chloride solution or the like; a dusting powder for directly administering a fine powder of an active ingredient; or an insert agent used by formulating or impregnating a suitable substrate or a material with an active ingredient and inserting the resultant substrate or material into palpebrae or the like.

A solution or a suspension of the active ingredient and a commonly used pharmaceutical vehicle is employed for inhalation and used as, for example an aerosol spray for inhalation. The active ingredient in the form of a dry powder can be administered even with an inhalator or other apparatuses so that the active ingredient can directly be brought into contact with the lungs.

The dose of the active ingredient of the cancer remedy of the present invention depends on the kinds of diseases, administration routes, conditions, ages, sexuality, body weight and the like of patients; however, the dose is usually about 1 to 1000 mg/day and is preferably formulated so as to satisfy the conditions.

As specifically described in Examples, the active ingredient of the cancer remedy of the present invention inhibits the growth of L929 cells having a strong growth property at a low concentration. Since the active ingredient is capable of similarly inhibiting even the growth of various human cultured cancer cells at a low concentration, the active ingredient is a very useful compound as a carcinostatic agent.

### Examples

The present invention will be explained specifically hereafter with Reference Examples and Examples. The groups of compounds used are described below; however, the present invention is not limited only to the Examples. In some cases, the ¹H-NMR peaks derived from carboxylic acids, hydroxy groups, amines and amides are not observed. There are some cases where an amine substance is a hydrochloride though not specifically mentioned.

When there is a description "the following compounds were synthesized according to the same method using the respective corresponding substrates", the reagents were synthesized using the substrates corresponding to the products. However, when it was difficult to understand, part of the substrates were also specified. In these reactions, though there is a somewhat difference in the reaction temperature, reaction time and method for purification, it is needless to say that appropriate conditions can easily be found by trials if persons are those skilled in the art. The number (compound No.) after the generic name of the compound in each Example indicates the "compound No." listed in the above table.

### [Example 1]

### Preparation of methyl 2-(4-(2-naphthyloxy)benzamido)benzoate (compound No. 1)

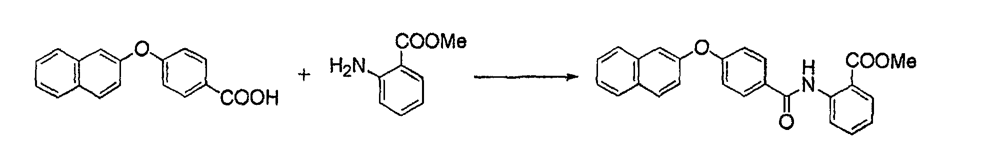

In 500 mL of dry methylene chloride, was suspended 29.1 g (0.11 mol) of 4-(2-naphthyloxy)benzoic acid under a nitrogen atmosphere. To the resulting suspension, was then added 15.4 g (0.121 mol) of oxalyl chloride. Ten drops of DMF were subsequently added to the suspension with a pipet. The mixture liquid was stirred at 35°C for 2 hours. The reaction liquid was then concentrated with an evaporator, and the residue was dissolved in 300 mL of dry methylene chloride. The resulting solution was dropped into a solution (250 mL) of 16.6 g (0.11 mol) of methyl anthranilate and 12.3 g (0.121 mol) of triethylamine in dry methylene chloride under cooling with ice under a nitrogen atmosphere. The mixture liquid was stirred under cooling with ice for 4 hours and then stirred at room temperature overnight. Water was added to the reaction liquid, and the resulting reaction liquid was extracted with methylene chloride twice. The organic layer was washed with a saturated brine and then dried over anhydrous sodium sulfate to distill off the solvent. The resulting residue was recrystallized from isopropyl alcohol (1.6 L) to provide 40.26 g (yield 92%) of methyl 2-(4-(2-naphthyloxy)benzamido)benzoate. Colorless needlelike crystals.
¹H-NMR(CDCl₃) δ (ppm):
3.96 (S, 3H), 7.09-7.17 (m, 3H), 7.27-7.31 (m, 1H), 7.42-7.53 (m, 3H), 7.58-7.64 (m, 1H), 7.76 (d, J = 8.5Hz, 1H), 7.84-7.90 (m, 2H), 8.03-8.10 (m, 3H), 8.93 (d, J = 8.3Hz, 1H), 12.02 (br. s, 1H).

### [Example 2]

### Preparation of 2-(4-(2-naphthyloxy)benzamido)benzoic acid (compound No. 4)

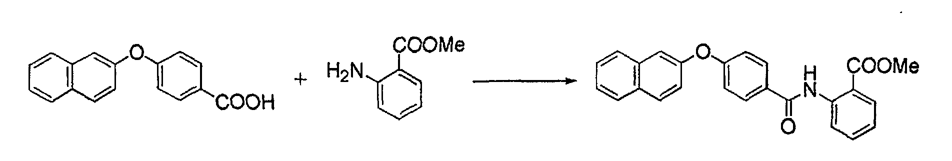

In a mixed solvent of methanol/THF (200 mL/400 mL), was dissolved 40.26 g (0.101 mol) of the methyl 2-(4-(2-naphthyloxy)benzamido)benzoate obtained in Example 1. To the resulting solution, was added 127 mL (0.51 mol) of a 4 M aqueous solution of lithium hydroxide. The mixture liquid was stirred at room temperature overnight. A 5 M hydrochloric acid was added to the reaction liquid to adjust the pH to about 1, and the reaction liquid was then stirred at room temperature for 0.5 hour. Water was added to the reaction liquid, and the obtained reaction liquid was extracted with ethyl acetate twice. The organic layer was washed with a saturated brine and then dried over anhydrous sodium sulfate, and the solvent was distilled off. The residue was recrystallized from isopropyl alcohol (1.3 L) to afford 31.23 g (yield 80%) of 2-(4-(2-naphthyloxy)benzamido)benzoic acid.
Colorless needlelike crystals.
¹H-NMR(CDCl₃) δ (ppm):
7.12-7.18 (m, 3H), 7.27-7.30 (m, 1H), 7.43-7.53 (m, 3H), 7.65 (dt, J = 1.7 and 8.6Hz, 1H), 7.76 (d, J = 7.6Hz, 1H), 7.85-7.91 (m, 2H), 8.03 (dd, J = 2.0 and 6.9Hz, 2H), 8.14 (dd, J = 1.7 and 7.9Hz, 1H), 8.96 (d, J = 7.6Hz, 1H), 11.84 (br. s, 1H).

### [Examples 3 to 69]

In the following Examples, the compounds used in the present invention were prepared according to the method in Example 1 or 2 using the respective corresponding starting materials. The following tables show 1H-NMR spectral data and reaction yields of the prepared compounds. The compound No. in the tables corresponds to the compound No. listed in the tables mentioned above. The spectral data marked with " 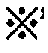 " are measured data in DMSO-d₆. All the others are data measured in CDCl₃.

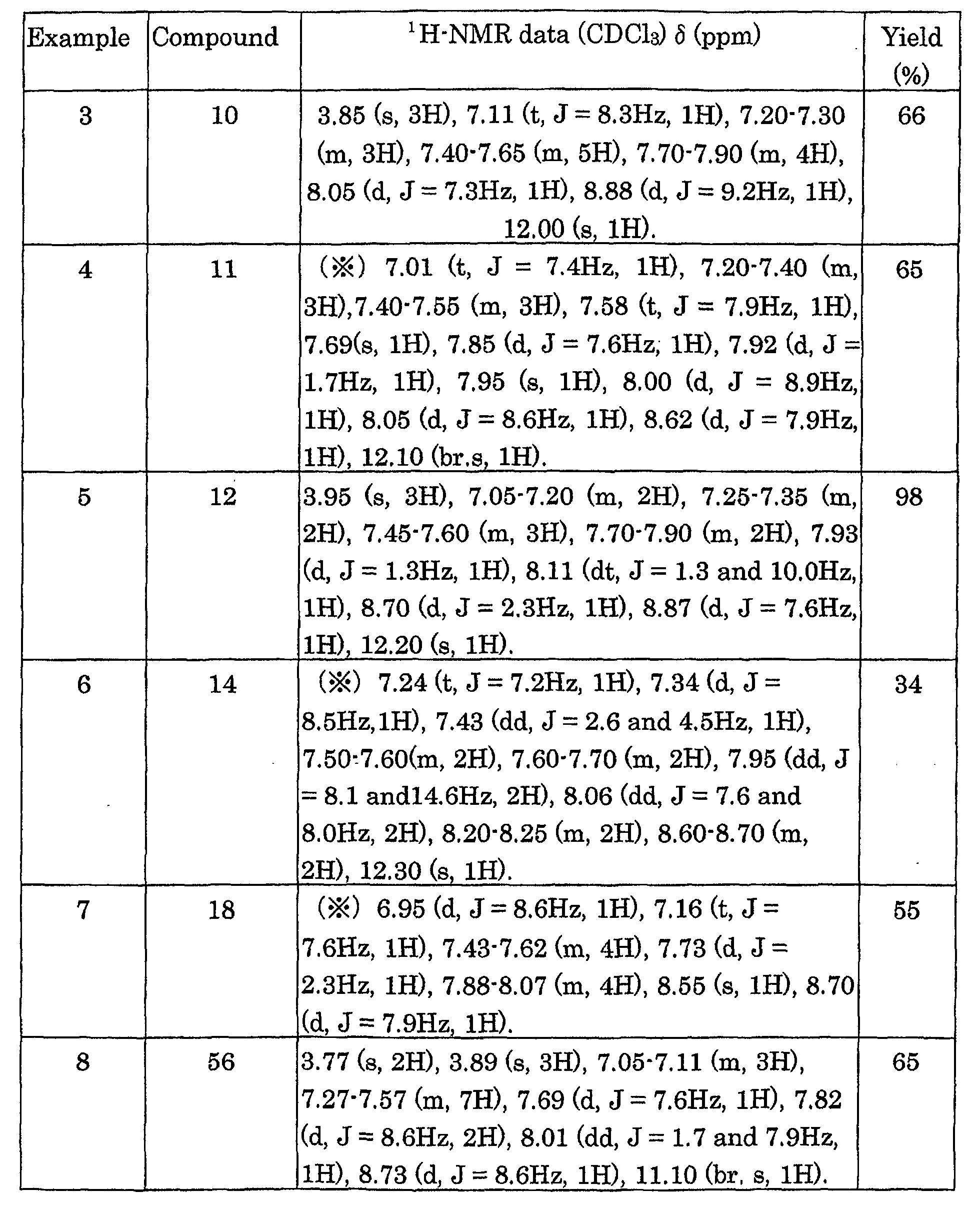

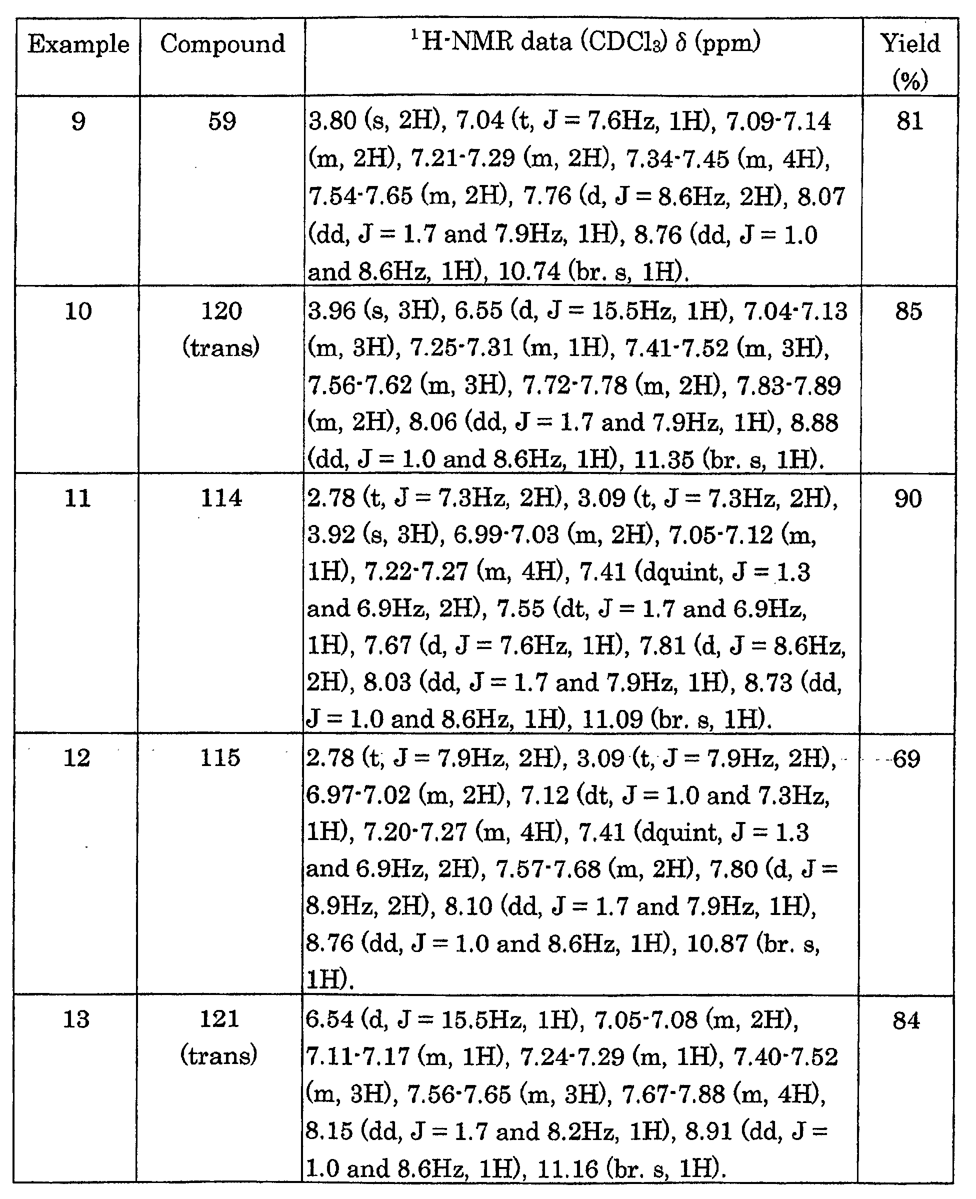

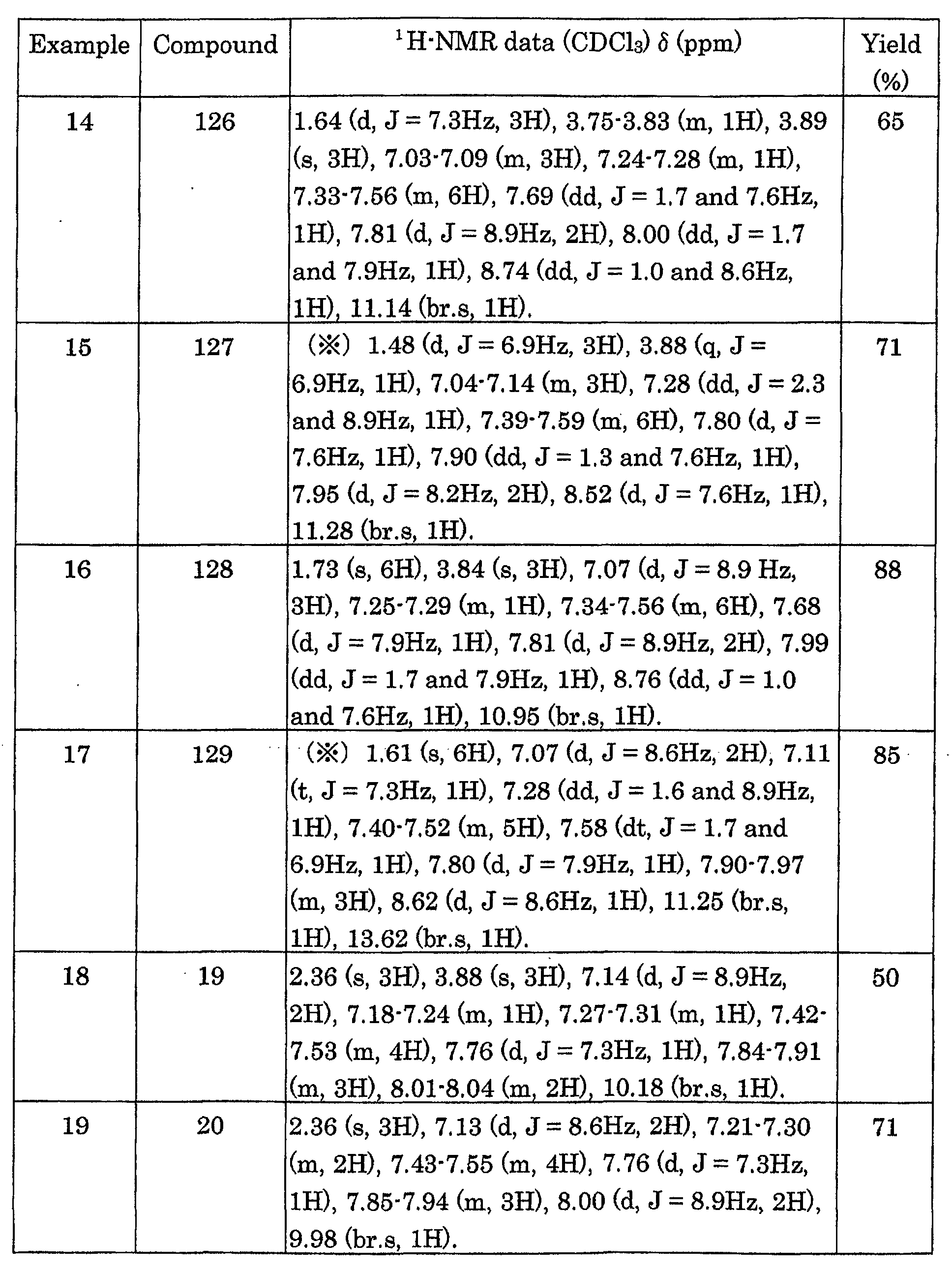

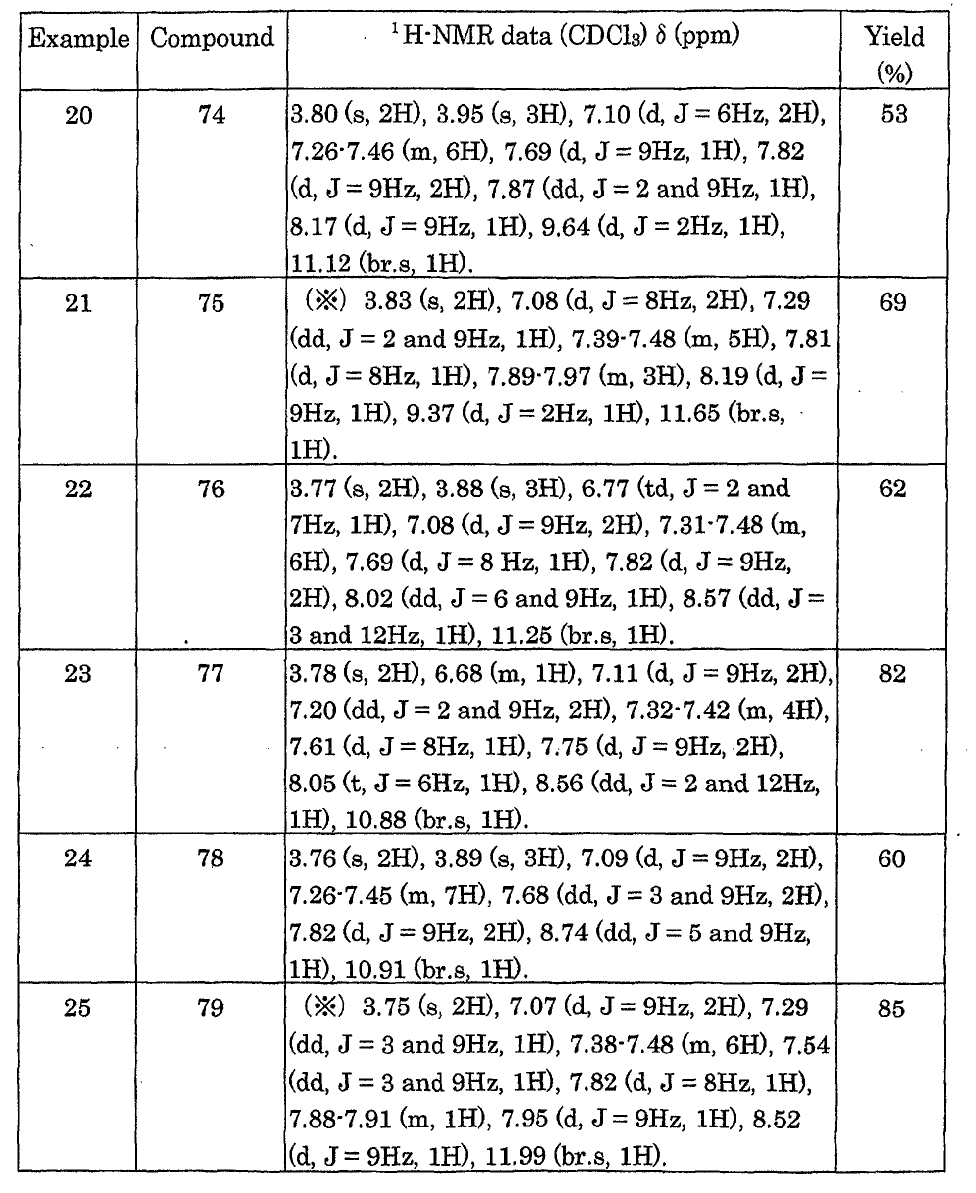

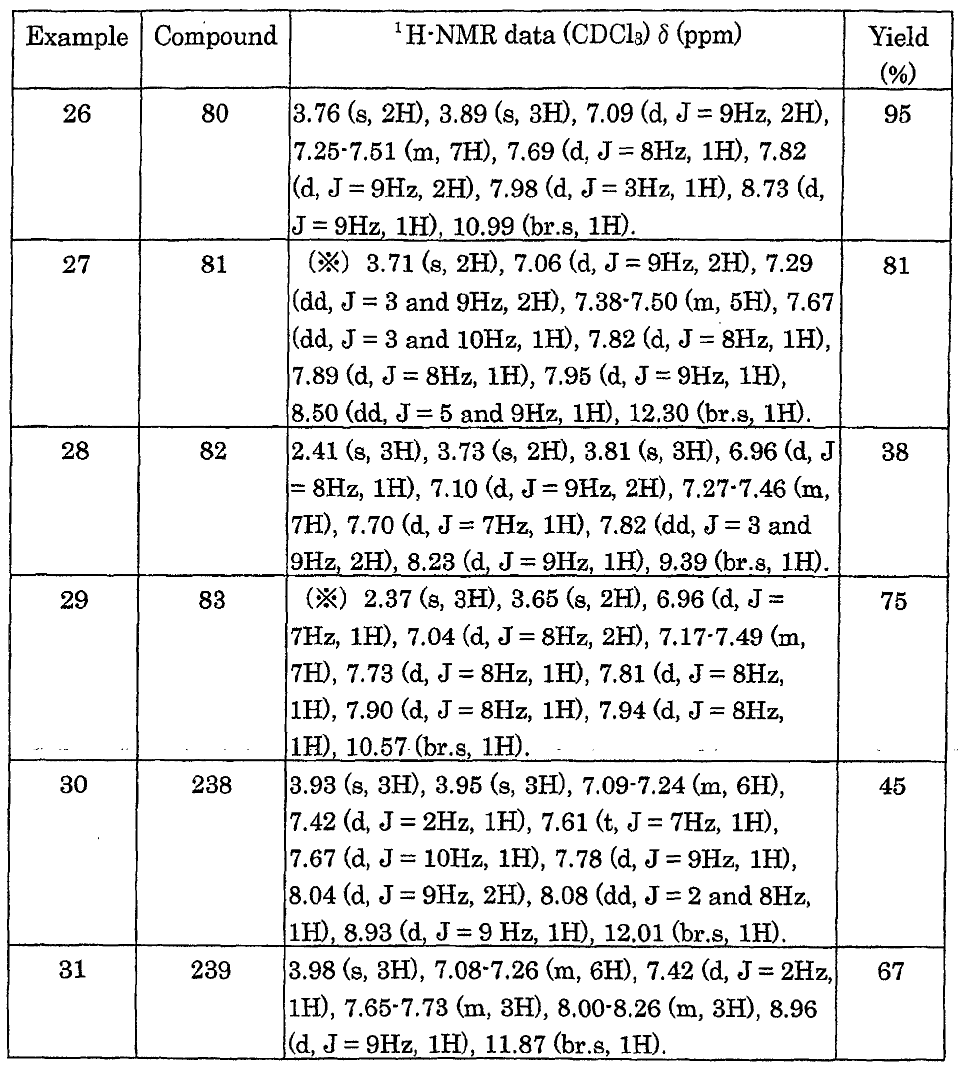

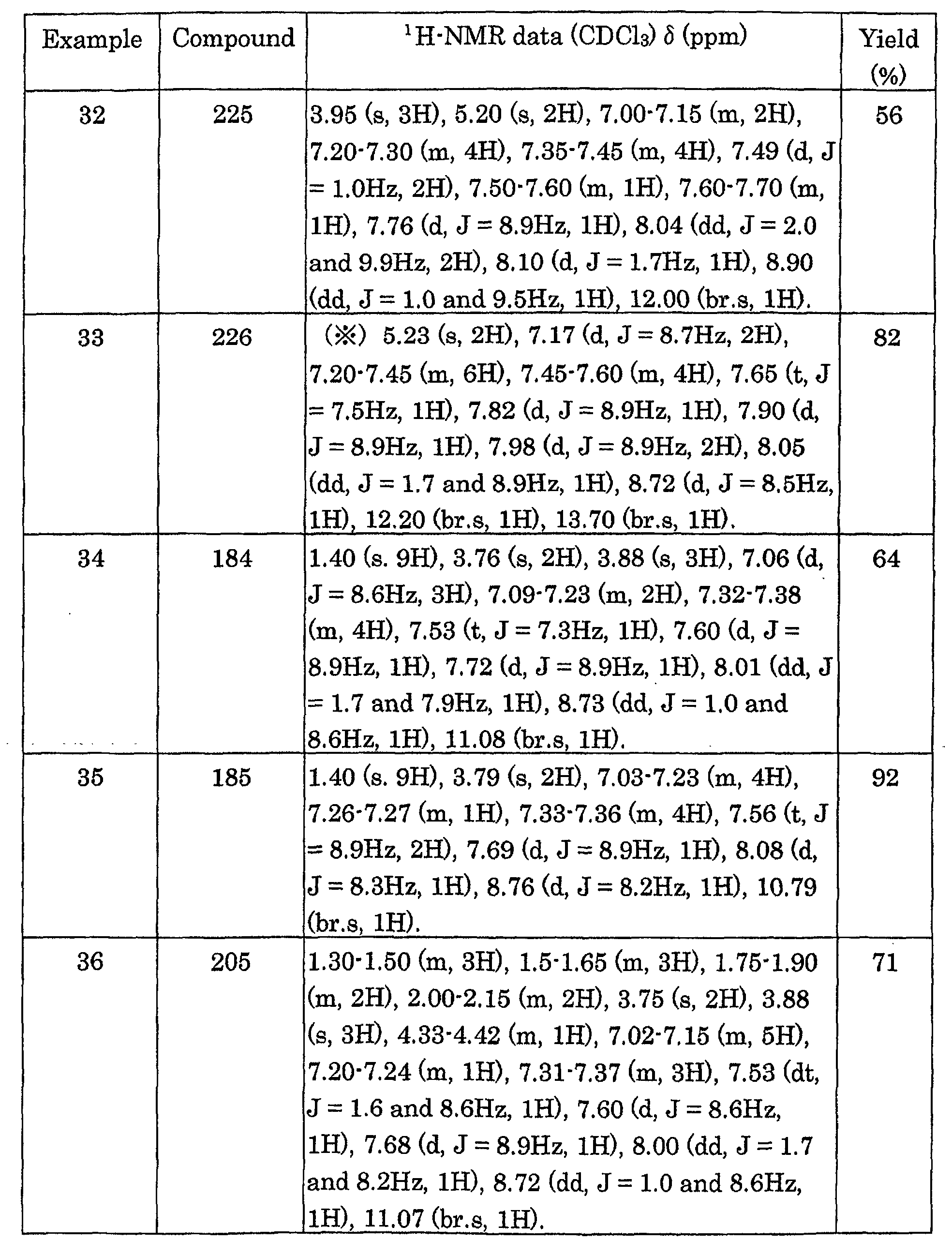

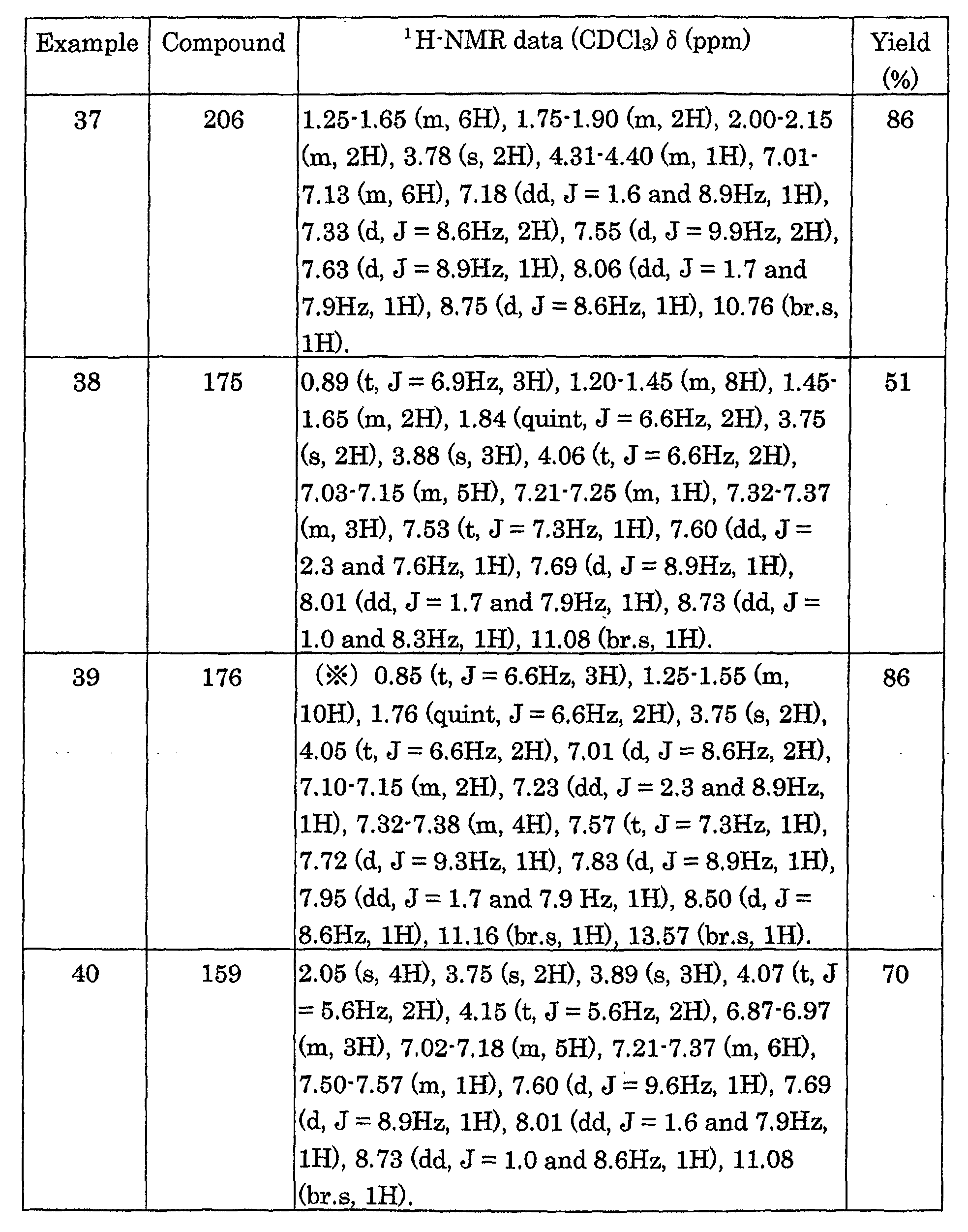

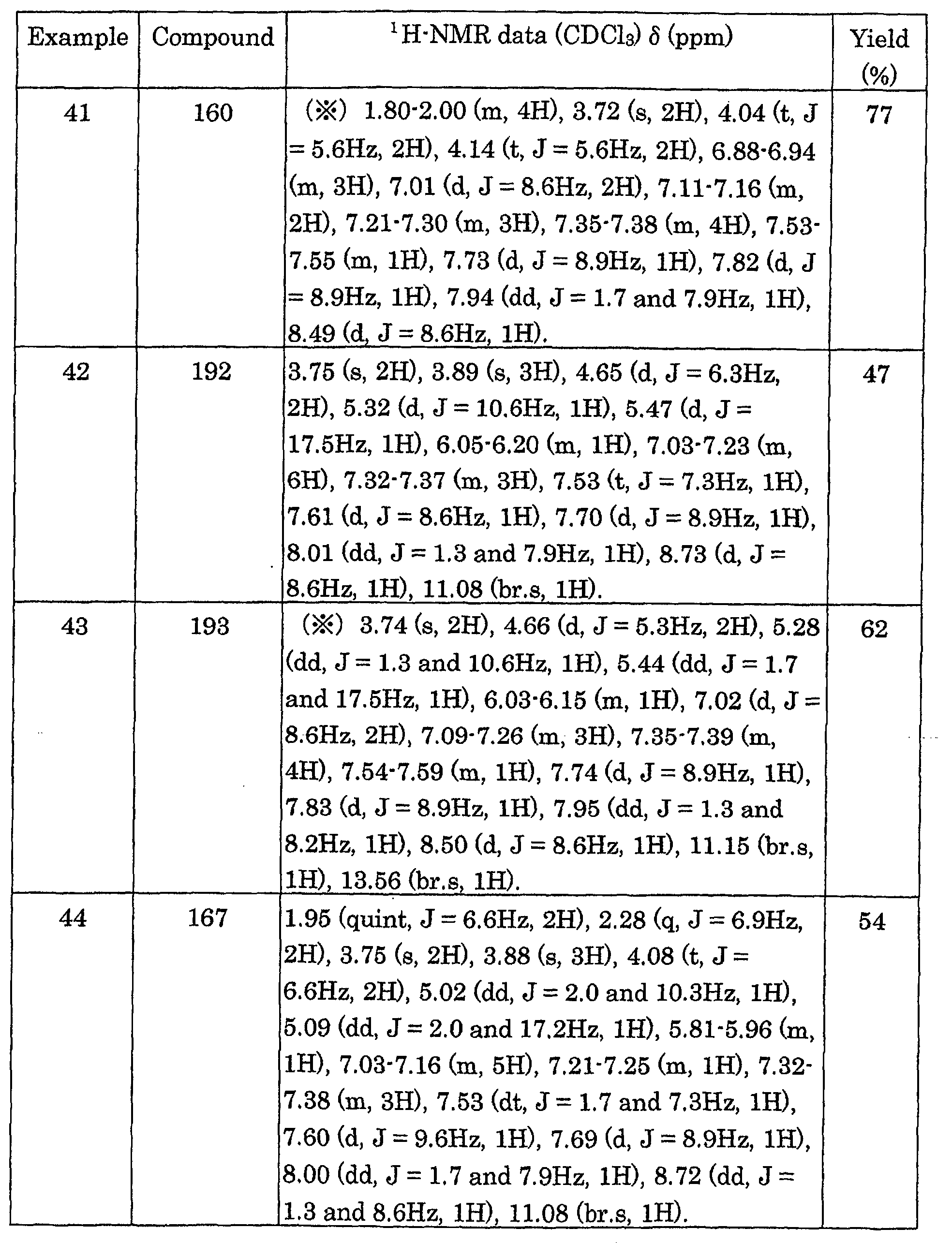

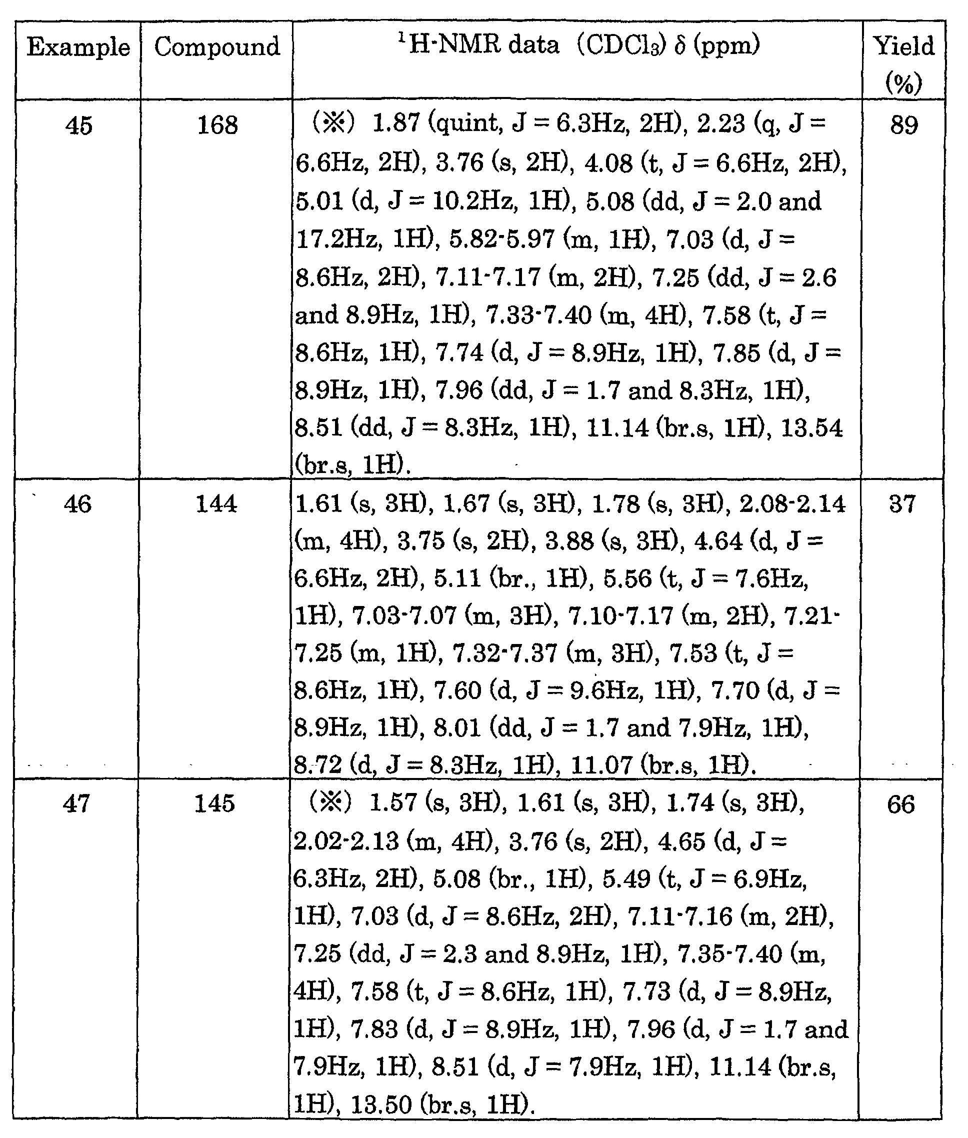

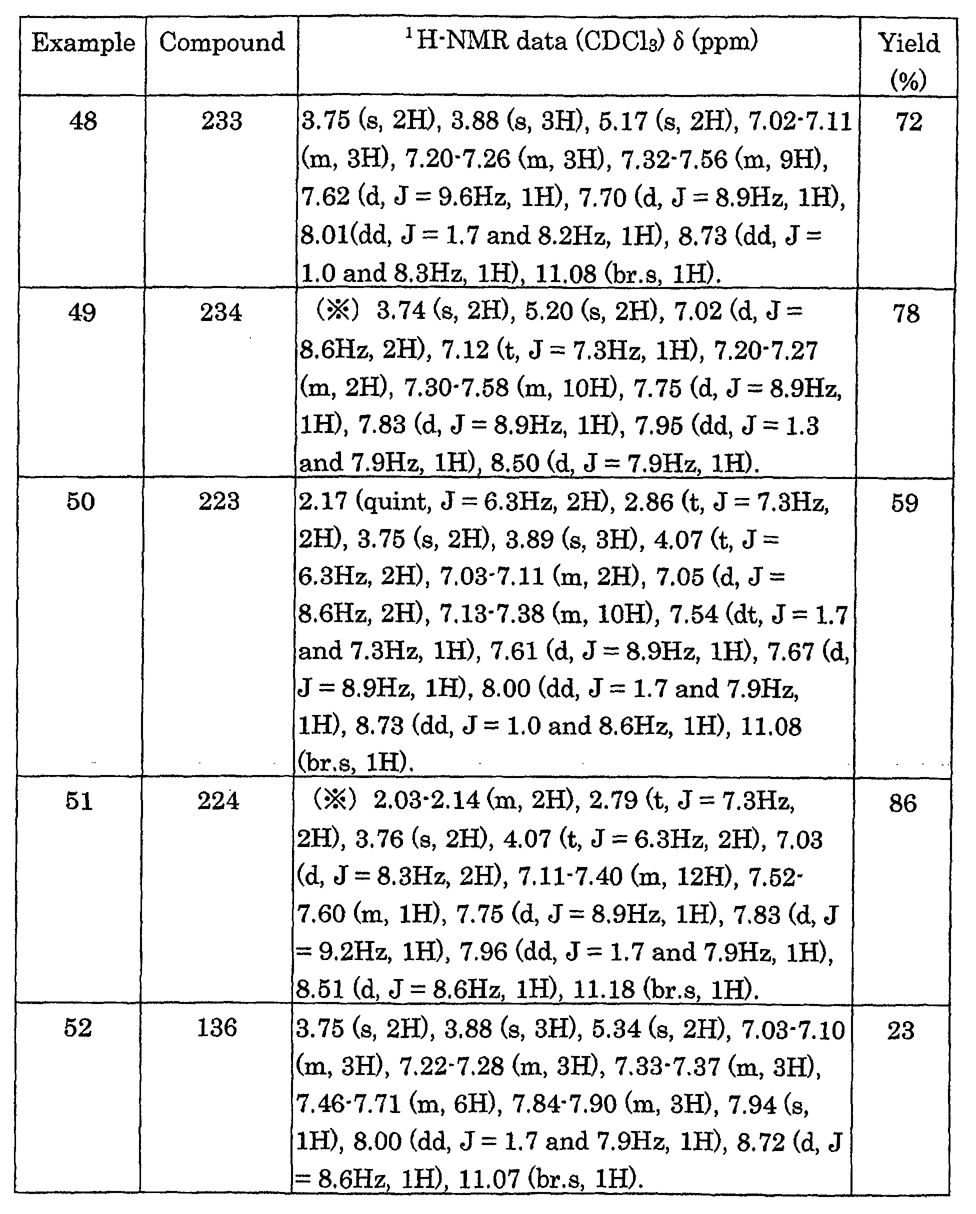

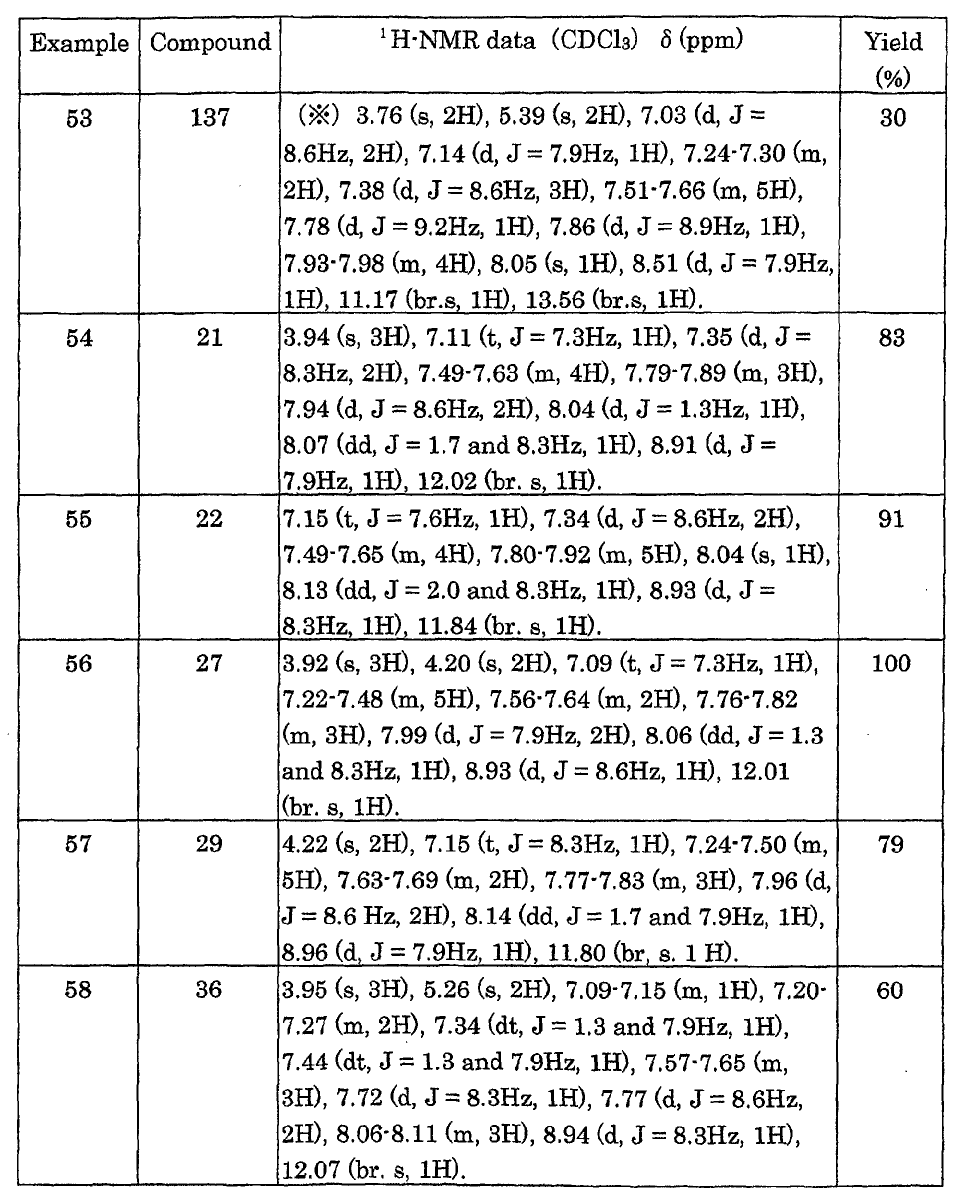

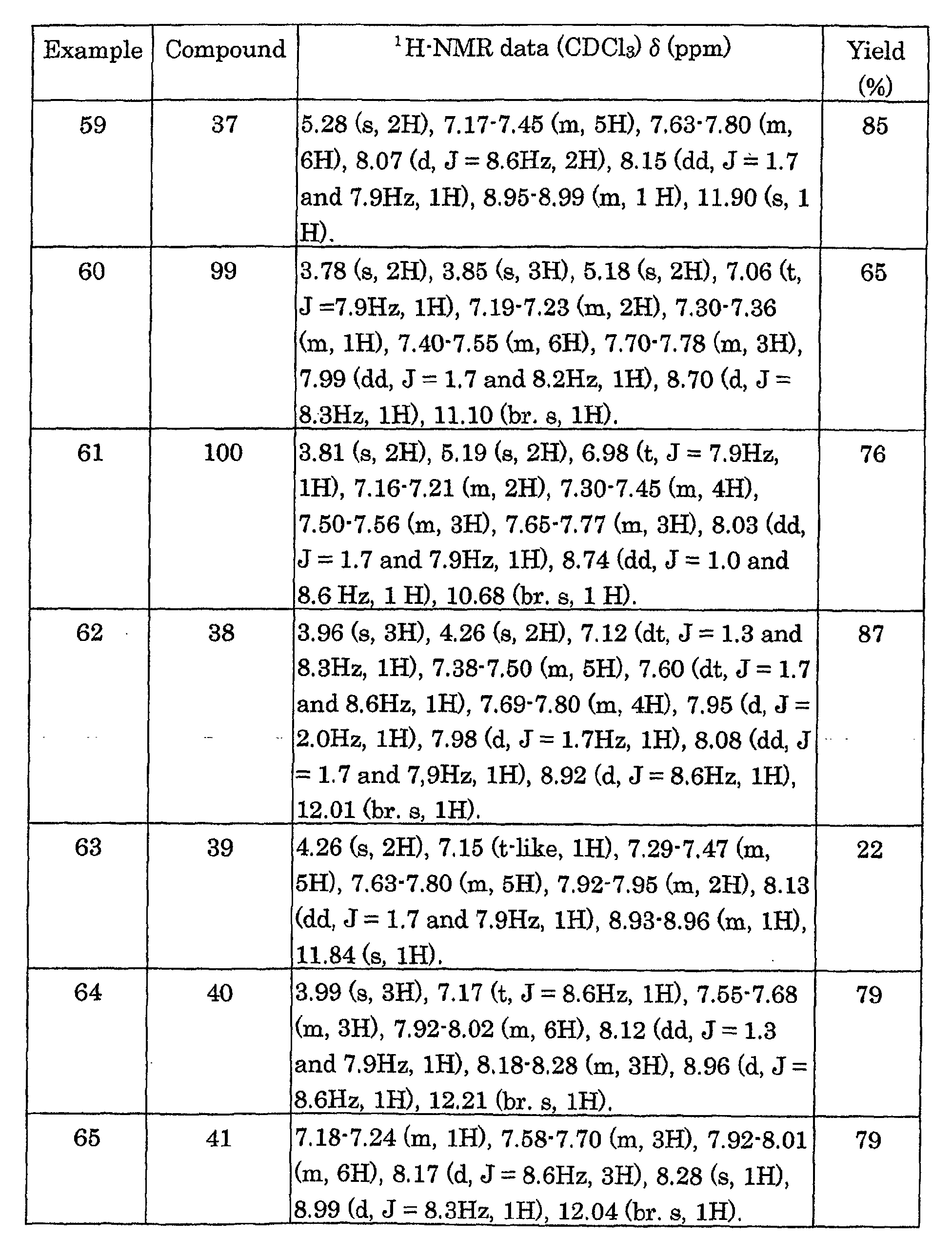

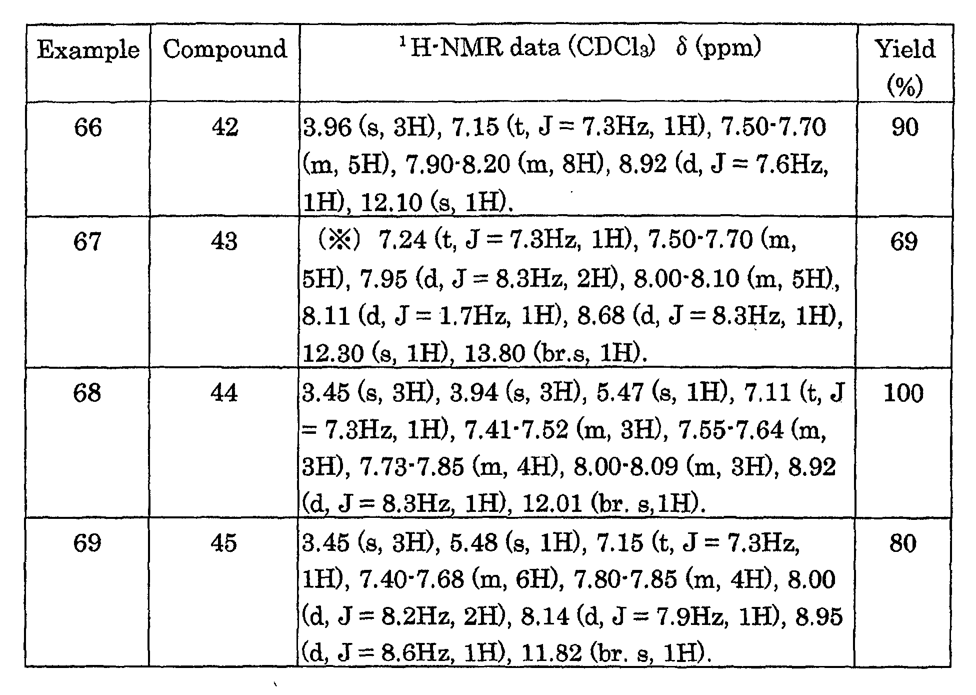

### [Example 70]

### Preparation of N-phenyl-(4-(2-naphthyloxy))benzamide (compound No. 5)

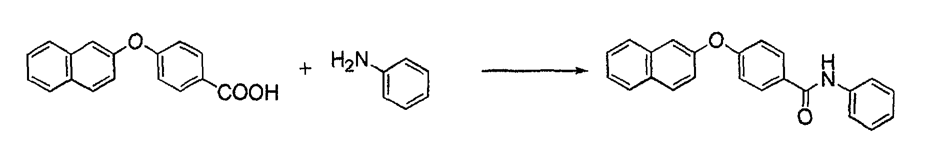

In 5 mL of dry methylene chloride, was suspended 53 mg (0.20 mmol) of 4-(2-naphthyloxy)benzoic acid under a nitrogen atmosphere. To the resulting suspension, were then added 56 mg (0.44 mmol) of oxalyl chloride. One drop of DMF was subsequently added to the suspension with a pipet. The mixture liquid was stirred at 35°C for 1.5 hours. The reaction liquid was concentrated with an evaporator, and the residue was dissolved in 5 mL of dry methylene chloride. The resulting solution was then dropped into a dry methylene chloride solution (5 mL) of 19 mg (0.20 mmol) of aniline and 22 mg (0.22 mmol) of triethylamine under cooling with ice under a nitrogen atmosphere. The mixture liquid was stirred under cooling with ice for 4 hours and then at room temperature overnight. Water was added to the reaction liquid, and the resulting reaction liquid was extracted with methylene chloride twice. The organic layer was washed with a saturated brine and then dried over anhydrous sodium sulfate, and the solvent was distilled off. The residue was purified by silica gel column chromatography (hexane : ethyl acetate = 20 : 1) to provide 27 mg (yield 40%) of N-phenyl-(4-(2-naphthyloxy))benzamide. Colorless solid.
¹H-NMR(CDCl₃) δ (ppm):
7.10-7.18 (m, 3H), 7.24-7.29 (m, 2H), 7.34-7.53 (m, 4H), 7.62-7.65 (m, 2H), 7.74-7.77 (m, 2H), 7.86-7.90 (m, 3H).

### [Example 71]

### Preparation of 2-(4-(2-naphthyloxy)benzamido)phenol (compound No. 6)

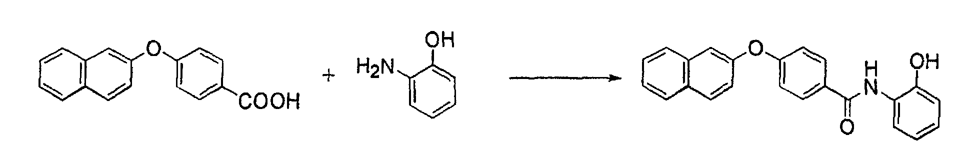

In 5 mL of dry methylene chloride, was suspended 144 mg (0.54 mmol) of 4-(2-naphthyloxy)benzoic acid under a nitrogen atmosphere. To the resulting suspension, was then added 76 mg (0.60 mmol) of oxalyl chloride. One drop of DMF was subsequently added to the suspension with a pipet. The mixture liquid was stirred at 35°C for 1.5 hours. The reaction liquid was concentrated with an evaporator, and the residue was dissolved in 9 mL of dry methylene chloride. The obtained solution was dropped into a dry methylene chloride solution (6 mL) of 59 mg (0.54 mmol) of o-aminophenol and 3 mL of dry pyridine under cooling with ice under a nitrogen atmosphere. The resulting solution was stirred under cooling with ice for 1.5 hours and then at room temperature for 3 days. Water was added to the reaction liquid, and the resulting reaction liquid was extracted with methylene chloride twice. The organic layer was washed with a saturated brine and then dried over anhydrous sodium sulfate, and the solvent was distilled off. The residue was purified by silica gel column chromatography (hexane : ethyl acetate = 20 : 1 to 10 : 1) to afford 147 mg (yield 76%) of 2-(4-(2-naphthyloxy)benzamido)phenol. Colorless solid.
¹H-NMR(CDCl₃) δ (ppm):
6.89-6.96 (m, 1H), 7.03-7.23 (m, 5H), 7.28-7.29 (m, 1H), 7.44-7.76 (m, 3H), 7.78-7.79 (d, J = 1.7Hz, 1H), 7.85-7.94 (m, 4H), 8.67 (s, 1H).

### [Example 72]

### Preparation of 2-(4-(2-naphthyloxy)benzamido)benzenesulfonamide (compound No. 7)

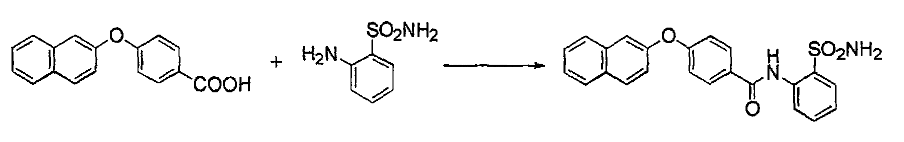

In 5 mL of dry methylene chloride, was suspended 132 mg (0.5 mmol) of 4-(2-naphthyloxy)benzoic acid under a nitrogen atmosphere. To the resulting suspension, was then added 70 mg (0.55 mmol) of oxalyl chloride. One drop of DMF was subsequently added to the suspension with a pipet. The obtained mixture liquid was stirred at 35°C for 2 hours. The reaction liquid was concentrated with an evaporator, and the residue was dissolved in 5 mL of dry methylene chloride. The resulting solution was dropped into a dry methylene chloride solution (4 mL) of 86 mg (0.5 mmol) of o-aminobenzenesulfonamide and 2 mL of dry pyridine under cooling with ice under a nitrogen atmosphere. The solution was stirred under cooling with ice for 4 hours and then at room temperature overnight. Water was added to the reaction liquid, and the resulting reaction liquid was extracted with methylene chloride twice. The organic layer was washed with a saturated brine and dried over anhydrous sodium sulfate, and the solvent was distilled off. The residue was recrystallized from a mixed solvent of benzene/ethyl acetate (8 mL/3 mL) to provide 112 mg (yield 54%) of 2-(4-(2-naphthyloxy)benzamido)benzenesulfonamide. Colorless granular crystals.
¹H-NMR(DMSO-d₆) δ (ppm):
7.23 (d, J = 8.9Hz, 2H), 7.26-7.38 (m, 2H), 7.46-7.68 (m, 4H), 7.90 (d, J = 7.9Hz, 2H), 7.97 (d, J = 8.6Hz, 3H), 8.04 (d, J = 9.2Hz, 1H), 8.46 (dd, J = 1.0 and 8.6Hz, 1H).

### [Example 73]

### Preparation of 2-(4-(2-naphthyloxy)benzamido)benzonitrile (compound No. 8)

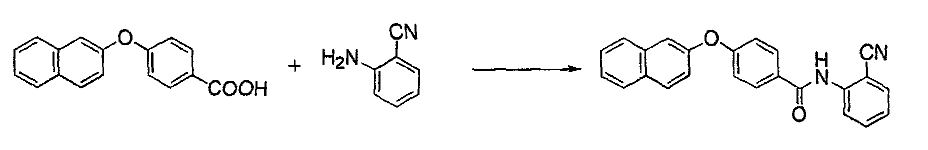

In 5 mL of dry methylene chloride, was suspended 264 mg (1.0 mmol) of 4-(2-naphthyloxy)benzoic acid under a nitrogen atmosphere. To the resulting suspension, was then added 140 mg (1.1 mmol) of oxalyl chloride. One drop of DMF was subsequently added to the suspension with a pipet. The mixture liquid was stirred at 36°C for 2 hours. The reaction liquid was concentrated with an evaporator, and the residue was dissolved in 7 mL of dry methylene chloride. The resulting solution was dropped into a dry methylene chloride solution (5 mL) of 118 mg (1.0 mmol) of anthranilonitrile and 111 mg (1.1 mmol) of triethylamine under cooling with ice under a nitrogen atmosphere. The mixture liquid was stirred under cooling with ice for 4 hours and then at room temperature overnight. Water was added to the reaction liquid, and the resulting reaction liquid was extracted with methylene chloride twice. The organic layer was washed with a saturated brine and then dried over anhydrous sodium sulfate, and the solvent was distilled off. The residue was purified by silica gel column chromatography (hexane : ethyl acetate = 20 1 to 5 : 1) to afford 263 mg (yield 72%) of 2-(4-(2-naphthyloxy)benzamido)benzonitrile. Colorless solid.
¹H-NMR(CDCl₃) δ (ppm):
7.15 (d, J = 8.9Hz, 2H), 7.18-7.30 (m, 2H), 7.46-7.54 (m, 3H), 7.61-7.69 (m, 2H), 7.76-7.79 (m, 1H), 7.85-7.96 (m, 4H), 8.34 (br.s, 1H), 8.61 (d, J = 8.6Hz, 1H).

### [Example 74]

### Preparation of 2-(4-(2-naphthylthio)benzamido)benzonitrile (compound No. 23)

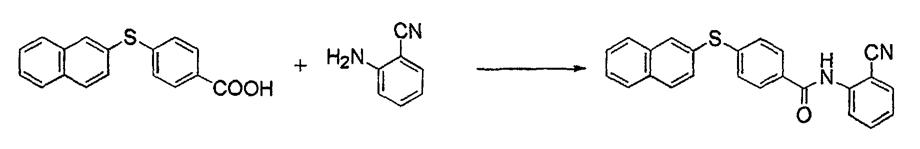

Procedures were carried out in the same manner as in Example 73 by using 280 mg (1.0 mmol) of 4-(2-naphthylthio)benzoic acid to afford 104 mg (yield 27%) of the title compound.
¹H-NMR(CDCl₃) δ (ppm):
7.21 (t, J = 8.6Hz, 1H), 7.33 (d, J = 8.6Hz, 2H), 7.49-7.68 (m, 4H), 7.78-7.89 (m, 4H), 8.06 (d, J = 1.3Hz, 1H), 8.31 (br.s, 1H), 8.59 (d, J = 8.6Hz, 1H).

### [Example 75]

### Preparation of 1-(4-(2-naphthyloxy)benzamido)-2-(tetrazol-5-yl)benzene (compound No. 9)

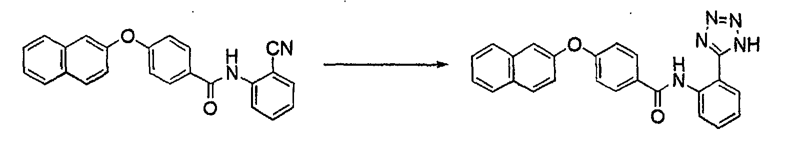

In 3 mL of dry DMF, were suspended 109 mg (0.30 mmol) of the 2-(4-(2-naphthyloxy)benzamido)benzonitrile obtained in Example 73, 48 mg (0.9 mmol) of ammonium chloride and 59 mg (0.9 mmol) of sodium azide. The resulting suspension was stirred at 80°C for 24 hours. To the reaction liquid, were added 5 mL of water and 5 mL of a 5 M hydrochloric acid, and the obtained reaction liquid was extracted with ethyl acetate twice. The organic layer was washed with a saturated brine and dried over anhydrous sodium sulfate, and the solvent was distilled off. The residue was recrystallized from 15 mL of acetonitrile to provide 92 mg (yield 75%) of 1-(4-(2-naphthyloxy)benzamido)-2-(tetrazol-5-yl)benzene. Colorless needlelike crystals.
¹H-NMR(CD₃OD) δ (ppm):
7.15-7.21 (m, 2H), 7.27-7.35 (m, 2H), 7.43-7.53 (m, 3H), 7.57-7.63 (m, 1H), 7.78-8.01 (m, 4H), 8.14-8.19 (m, 2H), 8.76-8.81 (m, 1H).

### [Example 76]

### Preparation of 1-(4-(2-naphthylthio)benzamido)-2-(tetrazol-5-yl)benzene (compound No. 24)

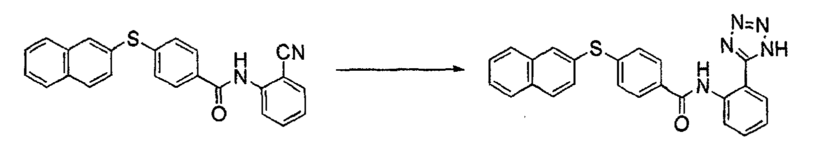

Procedures were carried out in the same manner as in Example 75 by using 50 mg (0.13 mmol) of the 2-(4-(2-naphthylthio)benzamido)benzonitrile obtained in Example 74 as a raw material to afford 43 mg (yield 77%) of the title compound.
¹H-NMR(DMSO-d₆) δ (ppm):
7.42 (t, J = 8.6Hz, 1H), 7.48 (d, J = 8.3Hz, 2H), 7.67-7.70 (m, 4H), 8.00-8.09 (m, 6H), 8.24 (d, J = 1.7Hz, 1H), 8.57 (d, J = 7.6Hz, 1H), 11.56 (br.s, 1H).

### [Example 77]

### Preparation of methyl 2-(3-amino-4-(2-naphthyloxy)benzamido)benzoate (compound No. 15)

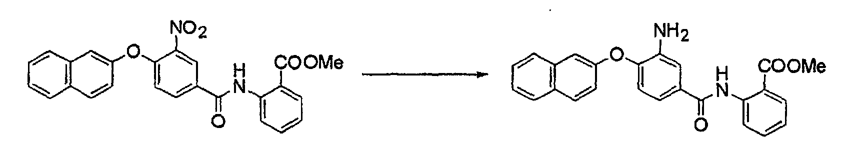

In 20 mL of ethyl acetate, was dissolved 350 mg (0.79 mmol) of the methyl 2-(4-(2-naphthyloxy)-3-nitrobenzamido)benzoate obtained in Example 5 (compound No. 12). To the resulting solution, was added 97 mg of a 10% Pd/C. The system was kept under a hydrogen atmosphere, and stirred at room temperature for 4 hours. The reaction liquid was filtered through Celite, and the resulting filtrate was concentrated. The residue was purified by silica gel column chromatography (hexane : ethyl acetate = 4 : 1 to 2 : 1) to provide 200 mg (yield 61%) of methyl 2-(3-amino-4-(2-naphthyloxy)benzamido)benzoate.
¹H-NMR(CDCl₃) δ (ppm):
3.95 (s, 3H), 6.95 (d , J = 8.5Hz, 1H), 7.11 (t, J = 7.0Hz, 1H), 7.30 (dd, J = 2.3 and 8.9Hz, 2H), 7.36 (dd, J = 2.3 and 8.2Hz, 2H), 7.40-7.50 (m, 3H), 7.57 (d, J = 2.0Hz, 1H), 7.61 (dd, J = 1.4 and 8.6Hz, 1H), 7.71 (d, J = 7.9Hz, 1H), 7.84 (t, J = 8.0Hz, 2H), 8.07 (dd, J = 1.5 and 8.7Hz, 1H), 8.92 (d, J = 1.3 and 8.3Hz, 1H), 11.90 (s, 1H).

### [Example 78]

### Preparation of 2-(3-amino-4-(2-naphthyloxy)benzamido)benzoic acid (compound No. 16)

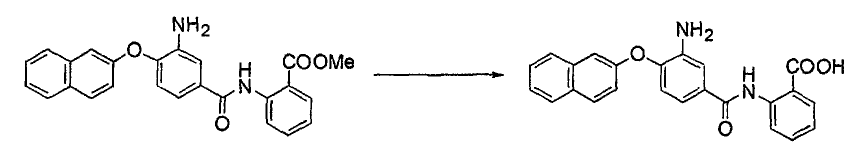

Procedures were carried out in the same manner as in Example 2 by using 200 mg (0.48 mmol) of the methyl 2-(3-amino-4-(2-naphthyloxy)benzamido)benzoate obtained in Example 77 as a raw material to afford 51 mg (yield 26%) of the title compound.
¹H-NMR(DMSO-d₆) δ (ppm):
5.40 (br.s, 2H), 6.96 (d, J = 7.8Hz, 1H), 7.10-7.30 (m, 2H), 7.30-7.35 (m, 2H), 7.40-7.50 (m, 3H), 7.63 (dt, J = 1.5 and 7.6Hz, 1H), 7.82 (d, J = 7.8Hz, 1H), 7.90 (d, J = 7.8Hz, 1H), 7.96 (d, J = 9.8Hz, 1H), 8.05 (dd, J = 1.5 and 7.8Hz, 1H), 8.73 (d, J = 7.8Hz, 1H), 12.20 (s, 1H).

### [Example 79]

### Preparation of 2-(4-(2-naphthyloxy)benzamido)benzoic acid (compound No. 4) sodium salt monoethanolate

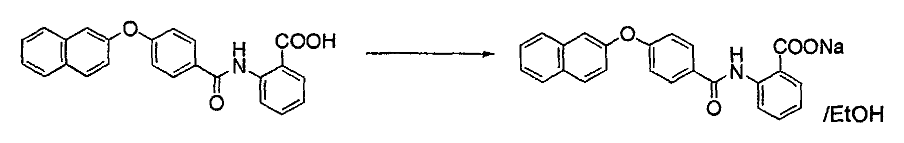

In 250 mL of ethanol, was dissolved 10.35 mg (27.0 mmol) of the 2-(4-(2-naphthyloxy)benzamido)benzoic acid obtained in Example 2 with heating. To the resulting solution, was added 13.77 mL (27.54 mmol) of a 2 M aqueous solution of sodium hydroxide. The resulting solution was stirred at room temperature for 10 minutes and then allowed to stand overnight. The separated colorless solid was collected by filtration to provide 10.15 g (yield 83%) of the title compound.
¹H-NMR(DMSO-d₆) δ (ppm):
1.07 (t, J = 6.9Hz, 3H), 3.44-3.47 (m, 2H), 4.30-4.32 (m, 1H), 6.97 (t, J = 7.5Hz, 1H), 7.17 (d, J = 7.5Hz, 2H), 7.30 (t, J = 6.9Hz, 1H), 7.35 (d, J = 8.5Hz, 1H), 7.47-7.55 (m, 3H), 7.87 (d, J = 8.0Hz, 1H), 7.94 (d, J = 8.0Hz, 1H), 8.02 (t, J = 8.0Hz, 2H), 8.09 (d, J = 8.5Hz, 2H), 8.69 (d, J = 8.0Hz, 1H), 15.66 (br.s, 1H).

### [Example 80]

### Preparation of 2-(4-(2-naphthyloxy)benzamido)benzoic acid (compound No 4) lysine salt

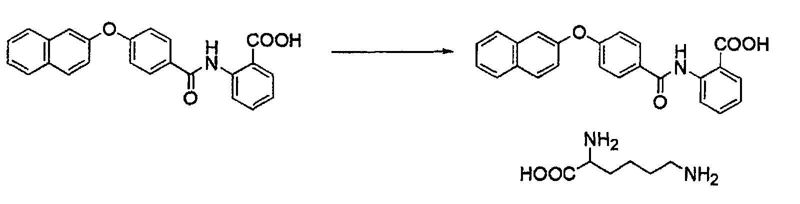

In ethanol (6 mL), was dissolved 192 mg (0.5 mmol) of the 2-(4-(2-naphthyloxy)benzamido)benzoic acid obtained in Example 2. To the resulting solution, was added a methanol solution (3 mL) of 73 mg (0.5 mmol) of lysine (1-lysine, free base). The mixture liquid was stirred at room temperature for 5 minutes and then allowed to stand for 6 hours. The separated colorless solid was collected by filtration to afford 247 mg (yield 93%) of the title compound.
¹H-NMR(CDCl₃-CD₃OD) δ (ppm):
1.40-1.58 (m, 2H), 1.58-1.73 (m, 2H), 1.78-1.90 (m, 2H), 2.86-2.97 (m, 2H), 3.50-3.60 (m, 1H), 7.03-7.19 (m, 3H), 7.23-7.32 (m, 1H), 7.39-7.53 (m, 4H), 7.75-7,83 (m, 1H), 7.83-7.98 (m, 2H), 8.05-8.17 (m, 3H), 8.65-8.73 (m, 1H).

### [Example 81]

### Preparation of 2-(4-(2-naphthyloxy)benzamido)benzoic acid (compound No 4) N-methyl-D-glucamine salt

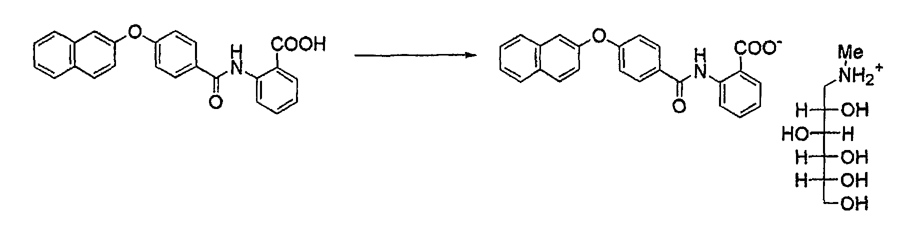

In ethanol (12 mL), was dissolved 383 mg (1.0 mmol) of the 2-(4-(2-naphthyloxy)benzamido)benzoic acid obtained in Example 2. To the resulting solution, was added an aqueous solution (1 mL) of 195 mg (1.0 mmol) of N-methyl-D-glucamine. The mixture liquid was stirred at room temperature for 1 hour. The reaction liquid was filtered through a glass filter to remove fine insolubles. The filtrate was then concentrated. The residue thick malt syrupy substance was dissolved in a mixed solvent of 20 mL of water and 1 mL of methanol, and the obtained solution was freeze-dried to provide 542 mg (yield 94%) of the title colorless powdery compound.
¹H-NMR(DMSO-d₆) δ (ppm):
2.49-2.51 (m, 5H), 2.89-3.07 (m, 2H), 3.38-3.47 (m, 3H), 3.57-3.61 (m, 1H), 3.66-3.67 (m, 1H), 3.86 (br.s, 1H), 4.40-4.44 (br.s, 1H), 4.58 (br.s, 1H), 5.43 (br.s, 1H), 6.98 (t, J = 8.6Hz, 1H), 7.20 (d, J = 8.9Hz, 2H), 7.22-7.39 (m, 2H), 7.45-7.57 (m, 3H), 7.87-8.09 (m, 6H), 8.64 (d, J = 8.3Hz, 1H).

### [Example 82]

### Preparation of 1-(4-(2-naphthyloxy)benzamido)-2-(tetrazol-5-yl)benzene (compound No. 9) sodium salt

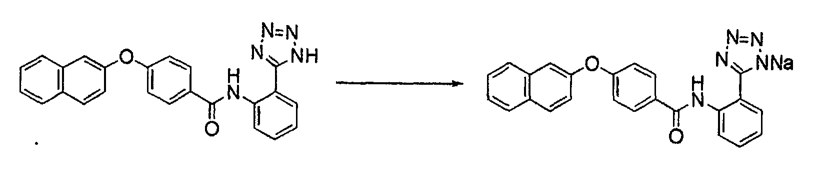

In 80 mL of ethanol, was dissolved 732 mg (1.80 mmol) of the 1-(4-(2-naphthyloxy)benzamido)-2-(tetrazol-5-yl)benzene obtained in Example 75 with heating. To the resulting solution, was added 0.897 mL (1.80 mmol) of a 2 M aqueous solution of sodium hydroxide. The resulting mixture liquid was stirred at room temperature for 2.5 hours. The reaction liquid was concentrated, and the residue transparent film was dissolved in 30 mL of distilled water. The obtained solution was filtered through a filter (0.45 µ m), and the filtrate was freeze-dried to afford 767 mg (yield 99%) of the title colorless powdery compound.
¹H-NMR(DMSO-d₆) δ (ppm):
7.15 (td, J = 1.5 and 7.8Hz, 1H), 7.25 (dt, J = 2.9 and 8.8Hz, 2H), 7.31 (td, J = 1.5 and 8.8Hz, 1H), 7.39 (dd, J = 2.5 and 8.8Hz, 1H), 7.47-7.54 (m, 2H), 7.60 (d, J = 2.4Hz, 1H), 7.90 (d, J = 7.8Hz, 1H), 7.90 (d, J = 7.8Hz, 1H), 7.96 (d, J = 7.8Hz, 1H), 8.03 (d, J = 9.3Hz, 1H), 8.25-8.30 (m, 3H), 8.79 (dd, J = 1.0 and 8.3Hz, 1H), 13.39 (br.s, 1H).

### [Example 83]

### Preparation of 2-(4-(2-naphthyloxy)phenylacetamido)benzoic acid (compound No. 59) sodium salt

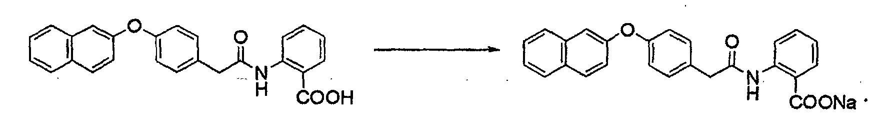

In 100 mL of ethanol, was dissolved 9.538 g (24.00 mmol) of the 2-(4-(2-naphthyloxy)phenylacetamido))benzoic acid (compound No. 59) obtained in Example 9 with heating. To the resulting solution, was added 11.976 mL (24.00 mmol) of a 2 M aqueous solution of sodium hydroxide. The resulting mixture liquid was stirred at room temperature for 1.5 hours. The reaction liquid was concentrated, and the residue transparent film was dissolved in 200 mL of distilled water. The obtained solution was filtered through a filter (0.45 µm), and the filtrate was freeze-dried to provide 9.97 g (yield 99%) of the title colorless powdery compound.
¹H-NMR(DMSO-d₆) δ (ppm):
3.65 (s, 2H), 6.95 (t, J = 8.2Hz, 1H), 7.10 (d, J = 8.6Hz, 2H), 7.25 (t, J = 7.3Hz, 1H), 7.33-7.36 (m, 1H), 7.37-7.53 (m, 5H), 7.93 (t, J = 7.3Hz, 2H), 7.99 (d, J = 8.9Hz, 2H), 8.46 (d, J = 8.3Hz, 1H), 14.80-14.91 (m, 1H).

### [Example 84]

### Preparation of methyl 2-(4-(6-hydroxy-2-naphthyloxy)benzamido)benzoate (compound No. 213)

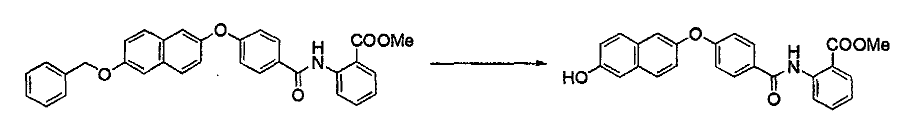

In 50 mL of THF, was dissolved 1.35 g (2.68 mmol) of the methyl 2-(4-(6-benzyloxy-2-naphthyloxy)benzamido))benzoate (compound No. 225) obtained in Example 32. To the resulting solution, was added 630 mg of a 10% Pd/C. The system was kept under a hydrogen atmosphere and stirred at room temperature for 32 hours. The reaction liquid was filtered through Celite, and the filtrate was concentrated to afford 1.04 g (yield 94%) of methyl 2-(4-(6-hydroxy-2-naphthyloxy)benzamido)benzoate.
¹H-NMR(CDCl₃) δ (ppm):
3.88 (s, 3H), 5.26 (br.s, 1H), 6.90-7.20 (m, 6H), 7.35 (br.s, 1H), 7.50-7.70 (m, 3H), 7.90-8.05 (m, 3H), 8.84 (d, J = 7.6Hz, 1H), 11.95 (br.s, 1H).

### [Example 85]

### Preparation of 2-(4-(6-hydroxy-2-naphthyloxy)benzamido)benzoic acid (compound No. 214)

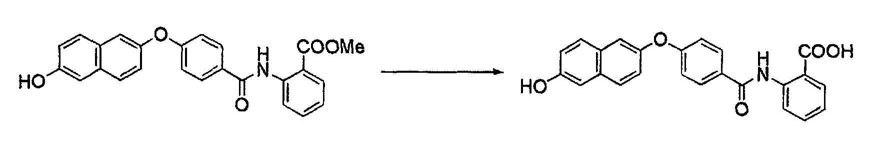

Procedures were carried out in the same manner as in Example 2 by using 1.04 g (2.52 mmol) of the methyl 2-(4-(6-hydroxy-2-naphthyloxy)benzamido)benzoate (compound No. 213) obtained in Example 84 as a raw material to provide 0.78 g (yield 78%) of the title compound.
¹H-NMR(DMSO-d₆) δ (ppm):
7.05-7.20 (m, 6H), 7.24 (s, 1H), 7.60 (dt, J = 2.0 and 9.0Hz, 1H), 7.74 (dd, J = 9.0 and 13.0Hz, 2H), 7.95 (d, J = 8.9Hz, 2H), 8.03 (dd, J = 1.7 and 8.0Hz, 1H), 8.28 (d, J = 9.0Hz, 1H), 9.70 (s, 1H), 12.20 (br.s, 1H), 13.70 (br.s, 1H).

### [Example 86]

### Preparation of methyl 2-(4-(6-hydroxy-2-naphthyloxy)phenylacetamido)benzoate (compound No. 217)

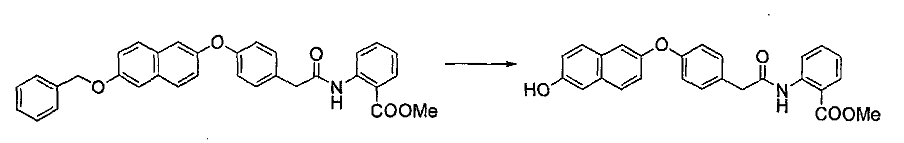

Procedures were carried out in the same manner as in Example 84 by using 50 mg (0.097 mmol) of the methyl 2-(4-(6-benzyloxy-2-naphthyloxy)phenylacetamido)benzoate (compound No. 233) obtained in Example 48 as a raw material to afford 22 mg (yield 53%) of the title compound.
¹H-NMR(CDCl₃) δ (ppm):
3.76 (s, 2H), 3.89 (s, 3H), 5.26 (br.s, 1H), 7.02-7.15 (m, 5H), 7.22 (dd, J = 2.3 and 8.9Hz, 1H), 7.31-7.37 (m, 3H), 7.53 (dt, J = 1.7 and 8.9Hz, 1H), 7.60 (d, J = 9.2Hz, 1H), 7.64 (d, J = 8.9Hz, 1H), 8.01 (dd, J = 1.7 and 8.3Hz, 1H), 8.72 (d, J = 8.3Hz, 1H), 11.10 (br.s, 1H).

### [Example 87]

### Preparation of 2-(4-(6-hydroxy-2-naphthyloxy)phenylacetamido)benzoic acid (compound No. 218)

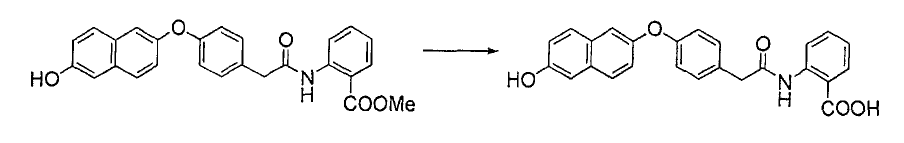

Procedures were carried out in the same manner as in Example 2 by using 22 mg (0.05 mmol) of the methyl 2-(4-(6-hydroxy-2-naphthyloxy)phenylacetamido)benzoate (compound No. 217) obtained in Example 86 as a raw material to provide 9 mg (yield 42%) of the title compound.
¹H-NMR (DMSO-d₆) δ (ppm):
3.83 (s, 2H), 7.07-7.28 (m, 6H), 7.41-7.46 (m, 3H), 7.65 (d, J = 7.6Hz, 1H), 7.75 (d, J = 8.9Hz, 1H), 7.81 (d, J = 8.9Hz, 1H), 8.04 (dd, J = 1.3 and 7.9Hz, 1H), 8.59 (d, J = 8.3Hz, 1H), 9.72 (s, 1H), 11.24 (br.s, 1H), 13.65 (br.s, 1H).

### [Reference Example 1]

### Synthesis of 4-(4-benzyloxyphenoxy)phenylacetic acid

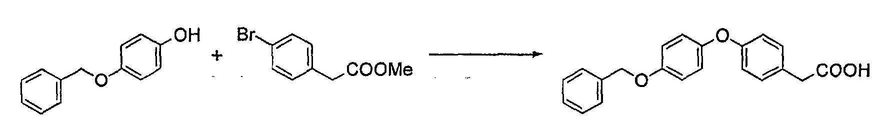

To hydroquinone monobenzyl ether (8.01 g, 40 mmol), were added benzene (100 mL) and methanol (25 mL). Into the resulting mixture, was slowly dropped 7.3 mL (38 mmol) of a 28% sodium methylate. The obtained mixture liquid was stirred at room temperature for 1 hour. The reaction liquid was concentrated, and pyridine (100 mL), 9.16 g (40 mmol) of methyl 4-bromophenylacetate and 1.25 g (12 mmol) of cupper(I) chloride were then added. The resulting mixture was stirred at 120°C for 30 hours with heating. The obtained reaction mixture was neutralized with hydrochloric acid, and the resultant product was extracted with ethyl acetate. The extract was dried and concentrated. The resulting concentrate was purified by silica gel chromatography to afford 4.76 g (13.7 mmol) of methyl ester of the objective compound.

In THF (10 mL), was dissolved 4.76 g (13.7 mmol) of the methyl ester compound. To the resulting solution, were added methanol (5 mL) and a 4 M aqueous solution (5 mL) of lithium hydroxide. The obtained reaction mixture liquid was stirred at room temperature for 4 hours. After completing the reaction, the resulting reaction liquid was neutralized with hydrochloric acid and concentrated until the quantity of the liquid reached a half. The produced crystals were collected by filtration and dried to provide 4.31 g (12.9 mmol) of the objective compound. Yield 94%.

### [Example 88]

### Preparation of methyl 2-(4-(4-benzyloxyphenoxy)phenylacetamido)benzoate (compound No. 315)

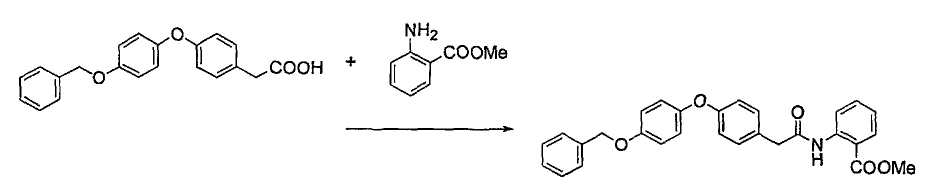

To 4.30 g (12.9 mmol) of the 4-(4-benzyloxyphenoxy)phenylacetic acid obtained in Reference Example 1, were added methylene chloride (70 mL) and further 2.13 g (16.8 mmol) of oxalyl chloride under a nitrogen gas atmosphere. The resulting reaction mixture liquid was stirred at 50°C for 3 hours with heating. The obtained reaction liquid was concentrated, and the concentrate was dissolved in dry methylene chloride (60 mL). The resulting solution was cooled with ice, and 1.80 g (12.3 mmol) of methyl benzoate was then added to the cooled solution. To the obtained mixture, was further added 1.80 g (18.1 mmol) of triethylamine. The resulting mixture liquid was stirred at 50°C for 1 hour and further at room temperature overnight. The obtained reaction liquid was washed with water, and the reaction product was extracted with ethyl acetate. The extract was dried and concentrated. The obtained concentrate was purified by silica gel chromatography to afford 4.29 g (9.2 mmol) of the objective compound. Yield 75%.
¹H-NMR(CDCl₃) δ (ppm):
3.72(s, 2H), 3.87(s, 3H), 5.04(s, 2H), 6.91-7.02(m, 6H), 7.06(td, J = 8.6Hz, 1.6Hz, 1H), 7.24-7.46(m, 7H), 7.52(td, J = 8.0Hz, 1.6Hz, 1H), 7.99(dd, J = 8.2Hz, 1.6Hz, 1H), 8.71(dd, J = 8.6Hz, 1.3Hz, 1H), 11.03(brs, 1H)

### [Examples 89 to 93]

Compounds listed in the following table (compound Nos. 313, 317, 319, 321 and 324) were synthesized according to the same method as in Example 88. The table shows yields and results of NMR measurement of the compounds.

| Example | Compound | ¹H-NMR data (CDCl₃) δ (ppm) | Yield (%) |
|---|---|---|---|
| 89 | 313 | 3.74 (2H, s), 3.85 (3H,s), 4.03 (2H, s), 6.91 (2H, d, J = 8.57Hz),7.00 (2H, d, J = 8.58Hz), 7.06 (1H, ddd, J = 0.99, 7.25, 7.92Hz), 7.19-7.26 (7H, m), 7.34 (2H, d, J=8.25Hz), 7.52 (1H, ddd, J = 1.32, 7.26, 8.57Hz), 7.99 (1H, dd, J = 1.32, 7.91Hz), 8.72 (1H, d, J = 8.58Hz), 11.05 (1H, br). | 63 |
| 90 | 317 | 3.74 (2H, s), 3.84 (3H, s), 5.02 (2H, s), 6.62-6.74 (3H, m), 7.03 (2H, d, J = 8.6Hz), 7.21 (1H, t, J = 8.2Hz), 7.32 (2H, d, J = 8.6Hz), 7.37-7.40 (5H, m), 7.47 (1H, dd, J = 8.9 and 2.6Hz), 7.95 (1H, d, J = 2.6Hz), 8.72 (1H, d, J = 8.9Hz), 10.95 (1H, sbr). | 46 |
| 91 | 319 | 3.75 (2H, s), 3.84 (3H, s), 5.02 (2H, s), 6.61-6.78 (4H, m), 7.03 (2H, d, J = 8.6Hz), 7.20 (1H, t, J = 8.2Hz), 7.33 (2H, d, J = 8.3Hz), 7.33-7.38 (5H, m), 7.97-8.03 (1H, m), 8.56 (1H, dd, J = 11.9 and 2.6Hz). | 55 |
| 92 | 321 | 2.31 (3H, s), 3.73 (2H, s), 3.83 (3H, s), 5.01 (2H, s), 6.61-6.73 (3H, m), 7.02 (2H, d, J = 8.6Hz), 7.20 (1H, t, J = 8.2Hz), 7.31-7.41 (8H, m), 7.78 (1H, d, J = 2.0Hz), 8.60 (1H, d, J = 8.6Hz), 10.93 (1H, sbr). | 47 |
| 93 | 324 | 3.72 (2H, s), 3.81 (3H, s), 5.07 (2H, s), 6.88-7.10 (7H, m), 7.14-7.28 (5H, m), 7.31 (2H, d, J = 8.25Hz), 7.50 (1H, ddd, J = 1.65, 7.26, 8.58Hz), 7.97 (1H, dd, J = 1.65, 7.92Hz), 8.72 (1H, d, J = 8.25Hz), 11.04 (1H, s). | 89 |

### [Example 94]

### Preparation of 2-(4-(4-benzyloxyphenoxy)phenylacetamido)benzoic acid (compound No. 316)

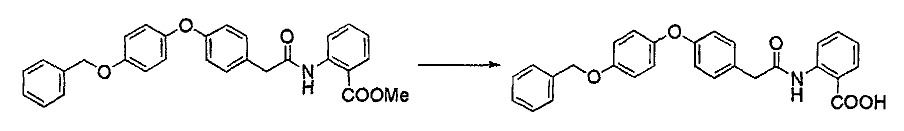

In THF (5 ml), was dissolved 278 mg (0.59 mmol) of the methyl 2-(4-(4-benzyloxyphenoxy)phenylacetamido)benzoate obtained in Example 88. To the resulting solution, were added methanol (5 mL) and a 4 M aqueous solution (2 mL) of lithium hydroxide. The resulting mixture liquid was stirred at room temperature for 2 hours. After completing the reaction, the obtained reaction liquid was neutralized with hydrochloric acid and concentrated until the quantity of the liquid reached a half. Crystals formed in the concentrate were collected by filtration and dried. The resulting crystals were further recrystallized from acetonitrile to provide 130 mg (0.29 mmol) of the objective compound. Yield 49%.
¹H-NMR(CDCl₃) δ (ppm):
3.74 (2H, s), 5.00 (2H, s), 6.87-6.99 (6H, m), 7.08 (1H, t, J = 7.5H z), 7.24-7.43 (7H, m), 7.57 (1H, t, J = 7.5Hz), 8.07 (1H, d, J = 8.0 Hz), 8.75 (1H, d, J = 8.0Hz), 10.77 (1H, brs).

### [Example 95]

### Preparation of methyl 2-(4-(4-hydroxyphenoxy)phenylacetamido)benzoate (compound No. 296)

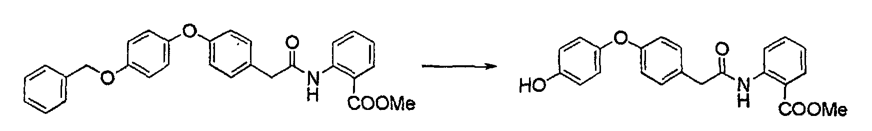

In ethyl acetate (17 mL), was dissolved 4.20 g (9.0 mmol) of the methyl 2-(4-(4-benzyloxyphenoxy)phenylacetamido)benzoate obtained in Example 88 under a nitrogen gas atmosphere. A 10% palladium carbon (800 mg) was added to the resulting solution to prepare a reaction mixture, The nitrogen gas was replaced with hydrogen gas, and the reaction mixture was stirred at room temperature for 32 hours. The obtained reaction liquid was filtered on Celite and concentrated. The resulting concentrate was recrystallized from ethyl acetate to afford 2.26 g (6.0 mmol) of the objective compound. Yield 66%.
¹H-NMR(DMSO-d₆) δ (ppm):
3.70 (2H, s), 3.78 (3H, s), 6.76 (2H, d, J = 8.9Hz), 6.88 (4H, d-like, J = 8.6Hz), 7.18 (1H, t, J = 7.5Hz), 7.30 (2H, d, J = 8.6Hz), 7.59 (1H, J = 7. 8Hz), 7.89 (1H, dd, J = 7.9Hz, 1.7Hz), 8.29 (1H, d, J = 7.6Hz), 9.31 (1H, s), 10.61 (1H, brs).

### [Example 96]

Procedures were carried out in the same manner as in Example 95 to synthesize compound No. 294. Yield 93%.
¹H-NMR(CDCl₃) δ (ppm):
3.98 (s, 3H), 6.92 (d, 2H, J = 8.91Hz), 7.01-7.15 (m, 4H), 7.32 (t,1 H, J = 8.24Hz), 7.76 (t, 1H, J = 8.56Hz), 8.02 (d, 2H, J = 8.59Hz), 8.10 (dd, 1H, J = 1.32, 7.91Hz), 8.65 (d, 1H, J = 8.26Hz), 9.57 (s, 1H), 11.63 (s, 1H).

### [Example 97]

### Preparation of methyl 2-(4-(4-cyclohexyloxyphenoxy)phenylacetamido)benzoate (compound No. 287)

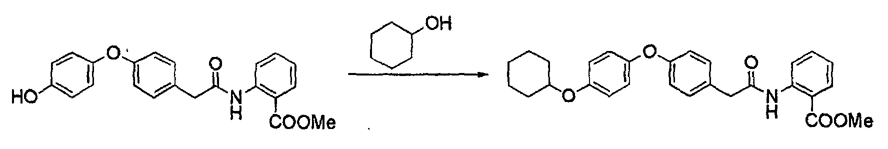

To an N-methylmorpholine (4 mL) solution of 250 mg (0.66 mmol) of the methyl 2-(4-(4-hydroxyphenoxy)phenylacetamido)benzoate obtained in Example 95, were added 350 mg (1.3 mmol) of triphenylphosphine, 0.13 mL (1.3 mmol) of cyclohexanol and 230 mg (1.3 mmol) of diethyl azodicarboxylate under a nitrogen gas atmosphere. The resulting mixture liquid was stirred at room temperature for 2 hours. To the obtained mixture liquid, were further added 350 mg (1.3 mmol) of triphenylphosphine, 0.13 mL (1.3 mmol) of cyclohexanol and 230 mg (1.3 mmol) of diethyl azodicarboxylate. The resulting mixture liquid was stirred at room temperature for 2 hours. After completing the etherifying reaction, white precipitates formed in the reaction mixture were removed by filtration, and the filtrate was purified by silica gel column chromatography to provide 241 mg (0.53 mmol) of the objective compound. Yield 81%.
¹H-NMR(CDCl₃) δ (ppm):
1.10-1.60 (6H, m), 1.79-1.84 (2H, brm), 1.96-2.04 (2H, brm), 3.72 (2 H, s), 3.87 (3H, s), 4.10-4.19 (1H, m), 6.86 (2H, d, J = 9.2Hz), 6.96 (4H, d, J = 8.3Hz), 7.06 (1H, t, J = 8.3Hz), 7.30 (2H, d, J = 8.6H z), 7.52 (1H, td, J = 8.6Hz, 1.7Hz), 7.99 (1H, dd, J = 8.3Hz, 1.7H z), 8.71 (1H, dd, J = 8.6Hz, 1.0Hz), 11.03 (1H, brs).

### [Example 98]

### Synthesis of 2-(4-(4-cyclohexyloxyphenoxy)phenylacetamido)benzoic acid (compound No. 288)

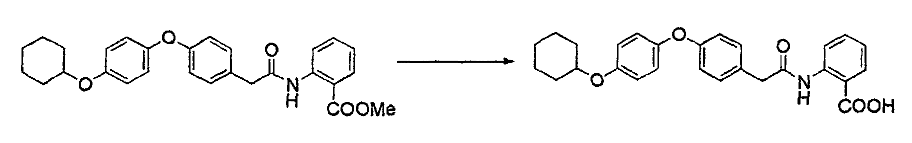

In THF (8 mL), was dissolved 240 mg (0.53 mmol) of the methyl 2-(4-(4-cyclohexyloxyphenoxy)phenylacetamido)benzoate obtained in Example 97. To the resulting solution, were added methanol (5 mL) and a 4 M aqueous solution (2 mL) of lithium hydroxide. The obtained reaction mixture liquid was stirred at room temperature for 3 hours. After completing the hydrolysis reaction, the resulting reaction liquid was neutralized with hydrochloric acid and concentrated until the quantity of the liquid reached a half. The reaction product was extracted from the concentrate with ethyl acetate, and the extract was dried and concentrated. The obtained oily concentrate was recrystallized from acetonitrile to afford the objective compound (121 mg). Yield 51%.
¹H-NMR(DMSO-d₆) δ (ppm):
1.24-1.55 (6H, m), 1.65-1.75(2H, brm), 1.85-1.95 (2H, brm), 3.72 (2H, s), 4.20-4.28 (1H, m), 6.89-6.96 (6H, m), 7.13 (1H, t, J = 8.5Hz), 7.32 (2H, d, J = 8.6Hz), 7.56 (1H, t, J = 8.0Hz), 7.95 (1H, dd, J = 7.9Hz,1.7Hz), 8.61 (1H, d, J = 8.3Hz), 11.16 (1H, brs).

### [Examples 99 to 111]

The compounds listed in the following tables were synthesized according to the same method as in Example 97. The yields of the respective compounds were calculated on the basis of the molar amounts of the raw material hydroxy substances corresponding to the compounds.

| Example | Compound | ¹H-NMR(CDCl₃) δ (ppm) | Yield (%) |
|---|---|---|---|
| 99 | 291 | 1.56-1.68(m, 2H), 1.76-1.88 (m,6H), 3.72 (s, 2H), 3.87 (s, 3H), 4.70 (brm, 1H), 6.83 (d, 2H, J = 8.9Hz), 6.96 (d, 2H, J = 8.6Hz), 6.97 (d, 2H, J = 8.9Hz), 7.05 (ddd, 1H, J = 7.9Hz, 6.9Hz, 1.0Hz), 7.30 (d, 2H, J = 8.6Hz), 7.51 (ddd, 1H , J = 8.6Hz, 6.9Hz, 1.3Hz), 7.98 (dd, 1H, J = 7.9Hz, 1.3Hz), 8.72 (dd, 1H, J = 8.6Hz, 1.0Hz), 11.03 (brs, 1H). | 94 |
| 100 | 285 | 1.28-1.48 (m, 16H), 1.58-1.81 (m, 4H), 2.02-2.14 (m, 2H), 3.72 (s,2H), 3.86 (s, 3H), 4.34 (m, 1H), 6.84 (d, 2H, J = 9.2Hz), 6.96 (d, 2H, J = 9.2Hz), 6.97 (d, 2H, J = 8.6Hz), 7.05 (ddd, 1H, J = 7.9Hz, 6.9Hz, 1.0Hz), 7.30 (d, 2H, J = 8.6Hz), 7.51 (ddd, 1H, J = 8.6Hz, 6.9Hz, 1.7Hz), 7.98 (dd, 1H, J = 7.9Hz, 1.7Hz), 8.72(d, 1H, J=8.6Hz), 11.03(s, 1H). | 47 |
| 101 | 272 | 1.32 (d, 6H, J = 6.3Hz), 3.72 (s, 2H), 3.87 (s, 3H), 4.47 (sep, 1H, J = 6.3Hz), 6.84 (d, 2H, J = 8.9Hz), 6.96 (d, 2H, J = 8.6Hz), 6.97 (d, 2H, J = 8.9Hz), 7.06 (ddd, 1H, J = 8.2Hz, 6.9Hz, 1.0Hz), 7.30 (d, 2H, J = 8.6Hz), 7.51 (ddd, 1H, J = 8.6Hz, 6.9Hz, 1.7Hz), 7.98 (dd, 1H, J = 8.2Hz, 1.7Hz), 8.72 (dd, 1H, J = 8.6Hz, 1.0Hz), 11.03 (s, 1H). | 72 |
| 102 | 265 | 0.96 (t, 6H, J = 7.6Hz), 1.67 (dq, 4H, J = 7.6Hz, 5.9Hz), 3.72 (s,2H), 3.86 (s, 3H), 4.03 (quint, 1H, J = 5.9Hz), 6.85 (d, 2H, J = 9.2Hz), 6.94-6.99 (m, 4H), 7.05 (ddd, 1H, J = 7.9Hz, 6.9Hz, 1.0Hz), 7.30 (d, 2H, J = 8.6Hz), 7.51 (ddd, 1H, J = 8.6Hz, 6.9Hz, 1.7Hz), 7.98 (dd, 1H, J = 7.9Hz, 1.7Hz), 8.72 (dd, 1H, J = 8.6Hz, 1.0Hz), 11.03 (s, 1H). | 50 |
| 103 | 274 | 1.5-1.7 (8H, m), 1.77-1.95 (6H, brm), 3.95 (3H, s), 4.38 (1H, m), 6.88 (2H, d, J = 8.9Hz), 6.98-7.04 (4H, m), 7.11 (1H, t, J= 7.7Hz), 7.60 (1H, td, J = 7.6Hz, 1.6Hz), 8.00 (2H, d, J = 8.9Hz), 8.07 (1H, dd, J = 7.9Hz, 1.7Hz), 8.92 (1H, dd, J = 8.6Hz, 0.7Hz), 11.98 (1H, br). | 82 |
| 104 | 276 | 1.51-1.74 (8H, brm), 1.76-2.00 (6H, brm), 3.96 (3H, s), 4.40 (1H, quint, J = 4.0Hz), 6.91 (2H, d, J = 8.9Hz), 7.00-7.06 (3H, m), 7.15 (1H, td, J = 7.6 and 1.3Hz), 7.62 (1H, td, J = 7.6 and 1.3Hz), 8.07-8.12 (2H, m), 8.63 (1H, d, J = 2.3Hz), 8.86 (1H, d, J = 8.0Hz), 12.15 (1H, brs). | 85 |
| 105 | 281 | 1.40-1.60 (8H, m), 1.72-1.95 (6H, m), 2.31 (3H, s), 3.71 (2H, s), 3.86 (3H, s), 4.34 (1H, quint, J = 4.0Hz), 6.81 (2H, d, J = 8.9Hz), 6.94-6.98 (4H, m), 7.30 (2H, d, J = 8.9Hz), 7.34 (1H, m), 7.79 (1H, d, J = 1.7Hz), 8.59 (1H, d, J = 8.6Hz), 10.90 (1H, brs). | 44 |
| 106 | 259 | 1.02 (6H, d, J = 6.6Hz), 2.07 (1H, quint, J = 6.6Hz), 3.69 (2H, d, J = 6.6Hz), 3.72 (2H, s), 3.88 (3H, s), 6.86 (2H, d, J = 9.2Hz), 6.95 (2H, d, J = 7.9Hz), 6.98 (2H,d, J = 8.9Hz), 7.06 (1H, td, J = 7.7 and 1.0Hz), 7.29 (2H, t, J = 8.0Hz), 7.52 (1H, td, J = 7.9 and 1.6Hz), 7.99 (1H, dd, J = 7.9 and 1.6Hz), 8.71 (1H, d, J = 7.9Hz). | 46 |
| 107 | 284 | 1.23-1.92 (14H, m), 3.71 (2H, s), 3.87 (3H, s), 4.35 (1H, tt, J = 3.96, 7.92Hz), 6.86-6.97 (4H, m),7.02-7.11 (3H, m), 7.28 (2H, m), 7.51 (1H. ddd, J = 1.65, 7.26, 8.57Hz), 7.98 (1H, dd, J = 1.65, 7.91Hz), 8.70 (1H, dd, J = 0.99, 8.58Hz), 11.30 (1H, s). | 69 |
| 108 | 257 | 3.72 (2H, s), 3.87 (3H, s), 4.31 (4H, s), 6.90-7.02 (10H, m), 7.06 (1H, ddd, J = 8.2Hz, 7.3Hz, and 1.0Hz), 7.25-7.33 (4H, m), 7.52 (1H, ddd, J = 8.6Hz, 7.3Hz and 1.7Hz), 7.99 (1H, dd, J = 8.2Hz and 1.7Hz), 8.71 (1H, dd, J = 8.6Hz and 1.0Hz), 11.04 (1H, brs). | 68 |
| 109 | 261 | 2.52(1H, ddd, J = 6.9Hz, 6.6Hz, and 1.3Hz), 2.55 (1H, ddd, J = 6.9Hz, 6.9Hz, and 1.3Hz), 3.72(2H, s), 3.87 (3H, s), 3.99 (2H, t, J = 6.9Hz), 5.11 (1H, dd, J= 10.2Hz, and 1.7Hz), 5.17 (1H, dd, J = 17.1Hz, and 1.7Hz), 5.90 (1H, dddd, J = 17.1Hz, 10.2Hz, 6.9Hz, and 6.6Hz), 6.86 (2H, d, J = 8.9Hz), 6.95 (2H, d, J = 8.6Hz), 6.98 (2H, d, J = 8.9Hz), 7.06 (1H, ddd, J = 8.2Hz, 7.3Hz, and 1.0Hz), 7.30 (2H, d, J = 8.6Hz), 7.51 (1H, ddd, J = 8.6Hz, 7.3Hz, and 1.3Hz), 7.98 (1H, dd, J= 8.2Hz, and 1.3Hz), 8.71 (1H, dd, J = 8.6Hz, and 1.0Hz), 11.04 (1H, brs). | 52 |
| 110 | 255 | 0.93 (6H, t, J = 7.3Hz), 1.34-1.55 (4H, m), 1.62-1.71 (1H, m), 3.72 (2H, s), 3.82 (2H, d, J = 5.6Hz), 3.87 (3H, s), 6.86 (2H, d, J = 8.9Hz), 6.95 (2H, d, J = 8.6Hz), 6.98 (2H, d, J = 8.9Hz), 7.06 (1H, ddd, J = 8.2Hz, 6.9Hz, and 1.0Hz), 7.30 (2H, d, J = 8.6Hz), 7.52 (1H, ddd, J = 8.6Hz, 6.9Hz, and 1.3Hz), 7.99 (1H, dd, J = 8.2Hz, and 1.3Hz), 8.71 (1H, dd, J = 8.6Hz, and 1.0Hz), 11.03(1H, brs). | 41 |
| 111 | 269 | 0.98 (3H, t, J = 7.3Hz), 1.45-1.64 (2H, m), 1.71-1.82 (2H, m), 3.72 (2H, s), 3.87 (3H, s), 3.93 (2H,t, J = 6.6Hz), 6.85 (2H, d, J = 8.9Hz), 6.96 (2H, d, J = 8.6Hz), 6.98 (2H, d, J = 8.9Hz), 7.06 (1H,ddd, J = 8.2Hz, 6.9Hz, and 1.0Hz), 7.30 (2H, d, J = 8.6Hz), 7.52 (1H, ddd, J = 8.6Hz, 6.9Hz, and 1.7Hz), 7.99 (1H, dd, J= 8.2Hz, and 1.3Hz), 8.71 (1H, dd, J = 8.6Hz, and 1.0Hz), 11.03 (1H, brs). | 95 |

### [Examples 112 to 133]

The following compounds listed in the following tables were synthesized according to the same method as in Example 98. The yields of the respective compounds were calculated on the basis of the molar amounts of the raw material methyl esters corresponding to the compounds.

| Example | Compound | ¹H-NMR (DMSO-d₆) δ (ppm): | Yield (%) |
|---|---|---|---|
| 112 | 314 | 3.74 (2H, s), 4.16 (2H, s), 6.93 (2H, d, J = 8.91Hz), 6.97 (2H, d, J = 8.58Hz), 7.12 (1H, ddd, J = 1.32, 7.26, 7.92Hz), 7.19-7.38 (9H, m), 7.56 (1H, ddd, J = 1.65, 7.26, 8.58Hz), 7.94 (1H, dd, J = 1.65, 7.92Hz), 8.50 (1H, dd, J = 0.66, 8.25Hz), 11.14 (1H, s), 13.55 (1H, br). | 78 |
| 113 | 292 | 1.53-1.85 (m, 6H), 1,86-1.92 (m,2H), 3.72 (s, 2H), 4.76 (br, 1H), 6.88-6.98 (m, 6H), 7.13 (dd, 1H, J = 7.9Hz, 7.6Hz), 7.32 (d, 2H, J = 8.6Hz), 7.57 (ddd, 1H, J = 8.9Hz, 7.6Hz, 1.3Hz), 7.95 (dd, 1H, J = 7.9Hz, 1.3Hz), 8.50 (d, 1H, J = 8.9Hz), 11.16 (s, 1H). | 66 |
| 114 | 279 | 1.50-1.70 (8H, br), 1.70-1.90 (6H, brm), 3.72 (2H, s), 4.41 (1H, m), 6.87-6.98 (6H, m), 7.13 (1H, t, J = 6.9Hz), 7.32 (2H, d, J = 8.6Hz), 7.58 (1H, td, J = 7.9Hz, 1.7Hz), 7.95 (1H, dd, J = 7.9Hz, 1.7Hz),8.50 (1H, d, J= 8.6Hz), 11.10 (1H, brs). | 80 |
| 115 | 286 | 1.34-1.72 (m, 22H), 3.72 (s, 2H), 4.37 (br, 1H), 6.91 (d, 2H, J = 9.2Hz), 6.92 (d, 2H, J = 8.6Hz), 6.96 (d, 2H, J = 9.2Hz), 7.13 (dd,1H, J = 7.9Hz, 7.3Hz), 7.33 (d, 2H, J = 8.6Hz), 7.57 (ddd, 1H, J = 8.6Hz, 7.3Hz, 1.7Hz), 7.95 (dd, 1H, J = 7.9Hz, 1.7Hz), 8.51 (d, 1H, J = 8.6Hz), 11.17 (s, 1H). | 68 |
| 116 | 273 | 1.32 (d, 6H, J = 6.3Hz), 3.72 (s, 2H), 3.87 (s, 3H), 4.47 (sep, 1H, J = 6.3Hz), 6.84 (d, 2H, J = 8.9Hz), 6.96 (d, 2H, J = 8.6Hz), 6.97 (d, 2H, J = 8.9Hz), 7.06 (ddd, 1H, J = 8.2Hz, 6.9Hz, 1.0Hz), 7.51 (ddd, 1H, J = 8.6Hz, 6.9Hz, 1.7Hz), 7.98 (dd, 1H, J = 8.2Hz, 1.7Hz), 8.72 (dd, 1H, J = 8.6Hz, 1.0Hz), 11.22 (s, 1H), 13.56 (brs, 1H). | 45 |
| 117 | 266 | 0.90 (d, J = 7.3Hz), 1.60 (dq, 4H, J = 7.3Hz, 5.9Hz), 3.72 (s, 2H), 4.14 (quint, 1H, J = 5.9Hz), 6.91 (d, 2H, J = 8.6Hz), 6.94 (s, 4H), 7.13 (dd, 1H, J = 7.9Hz, 7.6Hz), 7.32 (d, 2H, J = 8.6Hz), 7.57 (dd, 1H, J= 8.6Hz, 7.9Hz), 7.95 (d, 1H, J = 7.6Hz), 8.50 (d, 1H, J = 8.6Hz), 11.14 (s, 1H). | 40 |
| 118 | 275 | 1.50-1.70(8H, br), 1.71-2.00 (6H, brm), 4.46 (1H, m), 6.95 (2H, d, J = 9.2Hz), 7.05-7.09 (4H, m), 7.20 (1H, t, J = 7.7Hz), 7.66 (1H, t, J = 8.0Hz), 7.94 (2H, d, J = 8.9Hz), 8.05 (1H, dd, J = 7.9Hz, 0.7Hz), 8.70 (1H, d, J = 8.6Hz), 12.13 (1H, brs). | 78 |
| 119 | 297 | 3.92 (s, 2H), 6.98 (d, 2H, J = 9.23Hz), 7.11-7.00 (m, 4H), 7.34 (t,1H, J = 7.59Hz), 7.51 (d, 2H, J= 8.24Hz), 7.77 (t, 1H, J= 8.24Hz), 8.16 (dd, 1H, J = 1.32, 7.91Hz), 8.71 (d, 1H, J = 8.24Hz), 9.53 (s, 1H), 11.34(s, 1H), 13.77 (br, 1H). | 76 |
| 120 | 295 | 6.92 (d, 2H, J = 8.89Hz), 7.14-7.06 (m, 4H), 7.28 (t, 1H, J = 7.59Hz), 7.74 (t, 1H, J = 8.26Hz), 8.02 (d, 2H, J = 8.59Hz), 8.14 (dd, 1H, J = 1.32, 7.91Hz), 8.78 (d, 1H, J = 8.26Hz), 9.56 (s, 1H), 12.20 (s, 1H), 13.86 (br, 1H). | 78 |
| 121 | 277 | (∗) 1.4-1.70 (8H, brm), 1.80-2.00 (6H, brm), 4.48 (1H, m), 6.99 (2H, d, J = 9.2Hz), 7.11-7.17 (3H, m), 7.23 (1H, t, J = 7.9Hz), 7.67 (1H, t, J = 8.3Hz), 8.05 (1H, d, J = 7.5Hz), 8.16 (1H, dd, J = 8.9 and 2.3Hz), 8.58-8,63 (2H, m), 12.23 (1H, brs). | 80 |
| 122 | 318 | 3.76 (2H, s), 5.07 (2H, s), 6.55 (1H, dd, J= 7.7 and 1.8Hz), 6.64 (1H, t, J = 2.3Hz), 6.79 (1H, dd, J = 8.3 and 1.8Hz), 6.98 (2H, d, J = 8.6Hz), 7.27 (1H, t, J = 8.3Hz), 7.31-7.42 (7H, m), 7.63 (1H, dd, J = 8.9 and 2.6Hz), 7.88 (1H, d, J = 2.6Hz), 8.53 (1H, d, J = (8.9Hz), 11.05 (1H, brs), 13.91 (1H, brs). | 66 |
| 123 | 280 | 1.40-1.60 (8H, brm), 1.72-1.96 (6H, m), 3.75 (2H, s), 4.31 (1H, quint, J= 4.0Hz), 6.78 (2H, d, J = 9.0Hz), 6.92 (2H, d, J = 9.0Hz), 6.98 (2H, d, J = 8.6Hz), 7.27 (2H, d, J =8.6Hz), 7.52 (1H, dd, J = 9.2 and 2.6Hz), 8.07 (1H, d, J = 2.6Hz), 8.75 (1H, d, J = 9.2Hz), 10.70 (1H, brs). | 80 |
| 124 | 320 | 3.81 (2H, s), 5.05 (2H, s), 6.64 (1H, dd, J = 8.0 and 2.3Hz), 6.69-6.78 (2H, m), 6.96 (1H, t, J = 2.3Hz), 7.11 (2H, d, J = 8.3Hz), 7.23 (1H, t, J = 8.3Hz), 7.32 (2H, d, J= 8.3Hz), 7.37 (5H, s), 7.83 (1H, dd, J = 8.9 and 7.7Hz), 8.59 (1H, dd, J = 11.9 and 2.6Hz), 11.01 (1H, brs). | 76 |
| 125 | 322 | 2.27 (3H, s), 3.79 (2H, s), 5.05 (2H, s), 6.63 (1H, dd, J = 8.3 and 2.3Hz), 6.74 (1H, dd, J = 8.3 and 2.3Hz), 6.94 (1H, t, J = 2.3Hz), 7.09 (2H, d, J = 8.6Hz), 7.20 (1H, t, J = 8.3Hz), 7.24-7.36 (8H, m) 7.65 (1H, s), 8.65 (1H, d, J= 8.6Hz), 10.78 (1H, brs). | 63 |
| 126 | 282 | 1.40-1.60 (8H, m), 1.74-1.92 (6H, m), 2.27 (3H, s), 3.74 (2H, s), 4.29 (1H, m), 6.77 (2H, d, J = 9.2Hz), 6.92 (2H, d, J = 8.9Hz), 6.97 (2H, d, J = 8.6Hz), 7.28 (2H, d, J = 8.9Hz), 7.38 (1H, dd, J= 8.6 and 1.7Hz), 7.92 (1H, d, J = 1.7Hz), 8.63 (1H, d, J= 8.6Hz), 10.67 (1H, brs). | 93 |
| 127 | 260 | (*) 1.01 (6H, d, J = 6.6Hz), 2.05 (1H, quint, J = 6.6Hz), 3.64 (2H, d,, J = 6.6Hz), 3.76 (2H, s), 6.81 (2H, d, J = 9.2Hz), 6.93 (2H, d, J = 9.2Hz), 6.98 (2H, d, J = 8.6Hz), 7.09 (1H, t, J = 7.5Hz), 7.28 (2H, t, J = 8.3Hz), 7.59 (1H, td, J = 7.9Hz and 1.6Hz), 8.11 (1H, dd,J = 8.3 and 1.6Hz), 8.76 (1H, d, J = 8.3Hz), 11.74 (1H, brs). | 46 |
| 128 | 324 | 3.71 (2H, s), 5.06 (2H, s), 6.84 (2H, d, J = 8.58Hz), 6.91-7.24 (10H, m), 7.31 (2H, d, J = 8.58Hz), 7.56 (1H, dd, J = 7.26, 8.25Hz), 7.94 (1H, dd, J = 1.32, 7.92Hz), 8.50 (1H, d, J = 8.25Hz), 11.21 (1H, s), 13.55 (1H, br). | 68 |
| 129 | 284 | 1.37-1.80 (14H, m), 3.77 (2H, s), 4.48 (1H, tt, J = 3.96, 7.92Hz), 6.88 (2H, d, J = 8.57Hz), 7.02 (1H, ddd, J = 1.65, 6.27, 8.57Hz), 7.12-7.26 (4H, m), 7.36 (2H, d, J = 8.25Hz), 7.64 (1H, ddd, J = 1.65, 7.26, 8.57Hz), 8.03 (1H, dd, J = 1.65, 7.92Hz), 8,57 (1H, d, J = 8.57Hz), 11.27 (1H, s), 13.66 (1H, br). | 63 |
| 130 | 258 | 3.72 (2H, s), 4.30 (4H, s), 6.91 (2H, d, J = 8.6Hz), 6.92-7.01 (8H, m), 7.12 (1H, dd, J = 7.9Hz and 7.3Hz), 7.30 (1H, t, J = 7.3Hz), 7.32 (2H, d, J = 8.6Hz), 7.56 (1H, ddd, J = 8.6Hz, 7.3Hz, and 1.7Hz), 7.95 (1H, dd, J = 7.9Hz and 1.7Hz), 8.50 (1H, d, J = 8.6Hz), 11.24 (1H, brs), 13.50-13.60 (1H, br). | 37 |
| 131 | 262 | 2.52 (1H, ddd, J = 6.9Hz, 6.6Hz, and 1.3Hz), 2.55 (1H, ddd, J = 6. 9Hz, 6.9Hz, and 1.3Hz), 3.72 (2H,s), 3.87 (3H, s), 3.99 (2H, t, J = 6.9Hz), 5.11 (1H, dd, J = 10.2Hz, and 1.7Hz), 5.17 (1H, dd, J = 17.1Hz, and 1.7Hz), 5.86-5.90 (1H, m), 6.90 (2H, d, J = 8.6Hz), 6.96 (4H, s), 7.13 (1H, dd, J = 7.6Hz, and 7.3Hz), 7.32 (2H, d, J = 8.6Hz), 7.57 (1H, dd, J = 8.6Hz, and 7.6Hz), 7.95 (1H, d, J = 7.3Hz), 8.50 (1H, d, J = 8.6Hz), 11.13 (1H, brs), 13.50-13.60 (1H, br). | 100 |
| 132 | 256 | 0.90 (6H, t, J = 7.3Hz), 1.33-1.50 (4H, m), 1.57-1.66 (1H, m), 3.72 (2H, s), 3.83 (2H, d, J = 5.9Hz), 6.90 (2H, d, J = 8.6Hz), 6.96 (4H,s), 7.13 (1H, dd, J = 7.6Hz, and 7.3Hz), 7.32 (2H, d, J = 8.6Hz), 7.57 (1H, dd, J = 8.6Hz, and 7.6Hz), 7.95 (1H, d, J = 7.3Hz), 8.50 (1 H, d, J = 8.6Hz), 11.13 (1H, brs), 13.50-13.60 (1H, br). | 80 |
| 133 | 270 | 0.98 (3H, t, J = 7.3Hz), 1.45-1.64 (2H, m), 1.71-1.82 (2H, m), 3.72 (2H, s), 3.93 (2H, t, J = 6.6Hz), 6.90 (2H, d, J = 8.6Hz), 6.96 (4H, s),7.13 (1H, dd, J = 7.6Hz, and 7.3Hz), 7.32 (2H, d, J = 8.6Hz), 7.57 (1H, dd, J = 8.6Hz, and 7.6Hz), 7.95 (1H, d, J= 7.3Hz), 8.50 (1H, d, J = 8.6Hz), 11.13 (1H, brs), 13.50-13.60 (1H, br). | 100 |

### [Example 134]

### Measurement of cytotoxicity using mouse tumorous cells L929

### [Method]

The cytotoxic activity against tumorous cells was measured according to a Neutral Red assay method [the method described in Journal of Tissue Culture Methodology, vol. 9, p. 7 (1984), Toxicology Letters, vol. 24, p. 119 (1985)]. Namely, 100 µL each of L929 cells (5 × 10⁴ cells/mL, 10% FCS/RPM1) was added to a 96-well ELISA plate, and the cells were cultured overnight. The test compound at each measuring concentration was dissolved in a DMSO solution and added, and culturing was further continued for 3 days. To the cultured cells, was then added 2.0 µ L of Neutral Red so as to provide the final concentration of 0.01%. Incubation was conducted at 37°C for 1 hour, and the cell culture supernatant was removed. The resultant cultured cells were washed with 200 µL of PBS twice to remove the excessive Neutral Red. To the washed cells, was then added 100 µL of a 50% ethanol-1% aqueous acetic acid. The dye incorporated in the cells was extracted, and the amount of the dye was determined by measuring the absorbance at 490 nm. The case where a drug was not added was taken as 100%, and the cytotoxicity was determined at the concentrations of the respective test compounds. The compound concentration and cytotoxicity at each concentration were plotted for each test compound to determine the concentration (LD₅₀ value) of the test compound manifesting 50% cytotoxicity. The measurements under the same conditions in two sets each were made, and the data were obtained from the average values. Results are shown in the following tables.

| Compound No. | LD₅₀ (µM) |
|---|---|
| 4 | >5 |
| 22 | >5 |
| 29 | >5 |
| 37 | >5 |
| 59 | 1.6 |
| 66 | >5 |
| 115 | >5 |
| 129 | >5 |
| 130 | 0.16 |
| 137 | 0.29 |
| 145 | 0.30 |
| 153 | 0.042 |
| 154 | >5 |
| 160 | 0.31 |
| 168 | 0.22 |
| 176 | 0.40 |
| 179 | 0.170 |
| 185 | 0.65 |
| 197 | 3.0 |
| 198 | 0.052 |
| 200 | 0.46 |
| 206 | 0.039 |
| 211 | 0.060 |
| 212 | 0.078 |
| 224 | 0.34 |
| 234 | 0.29 |
| 237 | 3.0 |
| 243 | 1.2 |
| 250 | 2.5 |
| 251 | 0.340 |
| 254 | 0.44 |
| 256 | 0.048 |
| 258 | 0.75 |
| 260 | 0.210 |
| 262 | 0.64 |
| 266 | 0.25 |
| 270 | 0.30 |
| 279 | 0.080 |
| 286 | 0.15 |
| 288 | 0.20 |
| 292 | 0.17 |
| 293 | 1.6 |
| 296 | >5 |
| 314 | 0.25 |
| 316 | 0.60 |
| 330 | 7.5 |
| 331 | 1.0 |
| 332 | 0.19 |

### [Example 135]

### Carcinostatic activity against human cultured cancer cells

### [Method]

Human cultured cancer cells (39 strains) were seeded in a 96-well plate, and a test substance solution (at concentrations of 5 grades diluted 10-fold ranging from 10⁻⁴ M to 10⁻⁸ M) was added on the next day to carry out culturing for two days. The number of grown cells in each plate was measured by colorimetric determination with Sulforhodamine B. The concentration at which the cell growth was inhibited by 50% (GI₅₀ value) as compared with the control (without addition of the test substance) was calculated, and the following values (concentrations) were calculated on the basis of the number of cells just before adding the test substance.

TGI : the concentration at which the growth is inhibited to the reference number of cells (no apparent increase or decrease in number of cells)

LC₅₀: the concentration at which the number of cells is decreased to 50% of the reference number of cells (cellulicidal activity)
The following table collectively shows the results of growth inhibition of the compound 206 of the test substance against representative 9 strains of cancer cells in 39 strains.

| Compound No. | Cancer cell strain | GI₅₀ (µM) | TGI (µM) | LC₅₀ (µM) |
|---|---|---|---|---|
| 206 | HBC-4 | 0.51 | 27 | >100 |
| | SF-539 | 0.36 | 20 | 51 |
| | HCT-15 | 0.066 | 17 | 58 |
| | NCI-H460 | 0.092 | 12 | 53 |
| | LOX-IMVI | 0.27 | 6.3 | 44 |
| | OVCAR-8 | 0.92 | 29 | 89 |
| | RXF-631L | 0.27 | 16 | 51 |
| | MKN-74 | 0.38 | 22 | >100 |
| | PC-3 | 14 | 30 | 62 |

### Industrial Applicability

The cancer remedy of the present invention has a cytotoxic activity on cell strains having a strong growth property, and further has a strong growth inhibitory activity or cytotoxic activity even against human cancer cells. Therefore, the remedy of the present invention can be used as a remedy for cancer.

## Claims

1. A cancer remedy comprising an anthranilic acid derivative represented by the following formula (1) or a pharmaceutically acceptable salt thereof as an active ingredient: 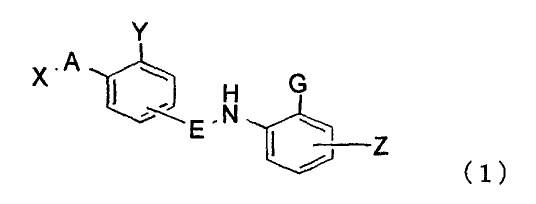 wherein, X represents a group selected from the following formula (2)-1 and formula (2)-2 in the formula (1): 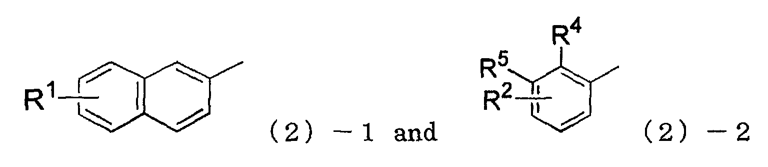 wherein, R¹ and R² represent each independently a hydrogen atom, a hydroxy group, a trihalomethyl group, an alkoxy group or an alkylthio group comprising a C₁-C₁₂ chain or cyclic hydrocarbon group and an oxy group or a thio group, a C₇-C₁₁ aralkyloxy group wherein an aryl group moiety may be substituted with one or more halogen atoms, methyl groups or methyloxy groups or a C₃-C₁₀ alkenyloxy group which may be substituted with one or more phenyl groups; R⁴ and R⁵ represent each independently a hydrogen atom, a halogen atom, a C₁-C₄ alkyl group or a C₁-C₄ alkoxy group, in the formula (2)-1 or the formula (2)-2;
A represents a bond; -O-, -S-, -S(=O)-, -S(=O)₂-, -CH₂-, -OCH₂-, -SCH₂-, -C(=O)- or -CH(OR⁶)-,
wherein, R⁶ represents a hydrogen atom or a C₁-C₄ alkyl group;
Y represents a hydrogen atom, a halogen atom, a nitro group, a nitrile group, an amino group, -COOR⁷, -NHCOR⁸ or -NHSO₂R⁹,
wherein, R⁷ represents a hydrogen atom or a C₁-C₄ alkyl group; R⁸ and R⁹ represent each independently a C₁-C₄ alkyl group;
E represents a bond; -C(=O) -CR¹⁰R¹¹C(=O)- (wherein, R¹⁰ and R¹¹ represent each independently a hydrogen atom or a fluorine atom), -CH₂CH₂C(=O)- or -CH=CHC(=O)-;
G represents a hydrogen atom, a hydroxy group, -SO₂NH₂, -COOR³, (wherein, R³ represents a hydrogen atom or a C₁-C₄ alkyl group), -CN or a tetrazol-5-yl group; and
Z represents a hydrogen atom, a halogen atom, a nitro group or a methyl group.

2. The cancer remedy according to claim 1, wherein G represents -COOR³ (wherein R³ represents a hydrogen atom or a C₁-C₄ alkyl group) or a tetrazol-5-yl group, in the formula (1).

3. The cancer remedy according to claim 1 or 2, wherein R¹ is located in the 6-position with respect to the group A (2-position) on the naphthalene ring in the formula (2)-1.

4. The cancer remedy according to claim 1 or 2, wherein, R² is located in the 4-position with respect to the group A on the benzene ring in the formula (2)-2.

5. The cancer remedy according to any of claims 1 to 4, wherein R⁴ and R⁵ represent each a hydrogen atom in the formula (2)-2.

6. The cancer remedy according to any of claims 1 to 5, wherein R¹ or R² represents a hydrogen atom; a hydroxy group; an alkoxy group comprising a C₁-C₁₂ chain or cyclic hydrocarbon group and an oxy group; a C₃-C₁₀ alkenyloxy group which may be substituted with one or more phenyl groups; a benzyloxy group, a phenylpropyloxy group or a naphthylmethyloxy group, in the formula (2)-1 or (2)-2.

7. The cancer remedy according to any of claims 1 to 6, wherein A represents -O- or -S- in the formula (1).

8. The cancer remedy according to any of claims 1 to 7, wherein E represents -C(=O)- or -CH₂C(=O)- in the formula (1).

9. The cancer remedy according to any of claims 1 to 8, wherein the bond A and the bond E are located in the para-positions in the benzene ring substituted with the group Y, in the formula (1).

10. The cancer remedy according to any of claims 1 to 9, wherein Y represents a hydrogen atom, a halogen atom, a nitro group or a nitrile group, in the formula (1).
